(19) Europäisches Patentamt
European Patent Office
Office européen des brevets

(11) EP 0 416 304 B1

(12) **EUROPEAN PATENT SPECIFICATION**

(45) Date of publication and mention of the grant of the patent:
**12.03.1997 Bulletin 1997/11**

(51) Int Cl.[6]: **C07F 11/00**, B01J 31/18, B01J 31/22, C08F 4/78, C07C 2/32

(21) Application number: **90115225.6**

(22) Date of filing: **08.08.1990**

(54) **Chromium compounds and uses thereof**

Chromverbindungen und ihre Verwendung

Composés du chrome et leur utilisation

(84) Designated Contracting States:
**AT BE CH DE DK ES FR GB GR IT LI LU NL SE**

(30) Priority: **10.08.1989 US 392688**
**21.12.1989 US 454554**

(43) Date of publication of application:
**13.03.1991 Bulletin 1991/11**

(73) Proprietor: **PHILLIPS PETROLEUM COMPANY**
**Bartlesville Oklahoma 74004 (US)**

(72) Inventors:
- **Reagen, William Kevin**
  **Dewey, OK 74029 (US)**
- **Conroy, Brian Keith**
  **Naperville, IL 60540 (US)**

(74) Representative:
**Dost, Wolfgang, Dr.rer.nat., Dipl.-Chem. et al**
**Patent- und Rechtsanwälte**
**Bardehle . Pagenberg . Dost . Altenburg .**
**Frohwitter . Geissler & Partner**
**Postfach 86 06 20**
**81633 München (DE)**

(56) References cited:
- **PREPRINTS AMERICAN CHEMICAL SOCIETY, vol. 34, no. 3, August 1989; W.K. REAGEN, pp. 583-588**
- **ZEITSCHRIFT FÜR ANORGANISCHE UND ALLGEMEINE CHEMIE, vol. 384, 1971, Leipzig (DD); D. TILLE, pp. 136-146**
- **ZEITSCHRIFT FÜR ANORGANISCHE UND ALLGEMEINE CHEMIE, vol. 404, no. 3, 1974, Leipzig (DD); W. SEIDEL, pp. 225-229**
- **PYRROLES, part 1, R.A. Jones (ed.); John Wiley & Sons; p. 428**

EP 0 416 304 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

**Description**

BACKGROUND OF THE INVENTION

This invention relates to chromium compounds, to their use as catalysts to trimerize and/or polymerise olefins. This invention also relates to a process to prepare these novel chromium compounds.

Supported chromium oxide catalysts have been a dominant factor in the production of olefin polymers, such as polyethylene or copolymers of ethylene and hexene. These catalysts can be used in a variety of polymerization processes. However, most known chromium compounds must be supported to be catalytically active. Furthermore, most supported chromium compounds are useful only for olefin polymerization. If an olefin copolymer is desired, the polymerization process becomes more complex in that two different monomers must be fed to the polymerization reactor.

Olefin trimerization catalysts are also known in the art, but usually lack selectivity to a desired product and also have a low product yield. However, olefin trimerization, if done efficiently, is a process to provide useful olefins, which in turn, can be further trimerized or, optionally, incorporated into a polymerization process.

Chromium compounds carrying a pyrrolyl residue are known from Z. anorg. allg. Chemie 384, 136-146 (1971), where D. Tille summarized general features of preparing these compounds. A process for preparing the Cr compounds by refluxing a chromium salt in the presence of a metal amide and an electron pair donor solvent is not reported. Nor is the use of these Cr compounds for tri- or polymerization of olefins.

SUMMARY OF THE INVENTION

Therefore, it is an object of this invention to provide novel chromium compounds.

It is yet another object of this invention to provide a process to prepare at least one novel chromium compound.

It is a further object of this invention to use these compounds for trimerizing olefins.

It is a further object of this invention to provide a composition comprising such chromium compound and use the compounds or composition as an improved olefin trimerization catalyst or as an olefin polymerization catalyst.

Therefore, in accordance with this invention, novel chromium-containing compounds are prepared from a reaction mixture comprising a chromium salt, a metal amide, and any electron pair donor solvent, such as, for example, an ether, wherein these reactants are refluxed.

In accordance with another embodiment of this invention this chromium-containing compound can be used, either supported or unsupported, to trimerize or polymerize olefins.

BRIEF DESCRIPTION OF THE DRAWINGS

FIGURE 1 is a computer generated ball and stick projection, with the exception of the chromium atoms which are represented as thermal ellipsoids, or a simplified structural representation or formula, of a molecule of Product I, $Cr_5(NC_4H_4)_{10}(OC_4H_8)_4$, as determined by single crystal x-ray crystallography, and

FIGURE 2 is a further simpiified ball and stick projection, with the exception of the chromium atoms which are represented as thermal ellipsoids, or a structural representation, of the same molecule shown in FIGURE 1.

FIGURE 3 is a computer generated Ortep drawing of the structure, or a simplified structural representation or formula, of a molecule of Product III, $Cr(NC_4H_4)_4$, as determined by single crystal x-ray crystallography.

FIGURE 4 is a further simplified ball and stick projection, with the exception of the chromium atoms which are represented as thermal ellipsoids, or a structural representation, of the same molecule shown in Figure 3, however, the entire crystal structure or lattice, with the formula $Cr(NC_4H_4)_4Na_2{\cdot}2OC_4H_8$ is shown.

FIGURE 5 is a computer generated Ortep drawing of the structure, or a simplified structural representation or formula, of a molecule of Product IV, $Cr(NC_4H_4)_5(OC_4H_8)$, as determined by single crystal x-ray crystallography,

FIGURE 6 is a further simplified ball and stick projection, with the exception of the chromium atoms which are represented as thermal ellipsoids, or a structural representation, of the same molecule shown in Figure 5, however the entire crystal structure or lattice, with the formula $[Cr(NC_4H_4)_5(OC_4H_8)][Na]_2{\cdot}4(OC_4H_8)$, is shown.

FIGURE 7 is a computer generated Ortep drawing of a molecule of Product V, $Cr(NC_4H_4)_3Cl(O_2C_2H_4(CH_3)_2)_3Na$, which includes the entire crystal structure or lattice, as determined by single crystal x-ray crystallography.

FIGURE 8 is a further simplified ball and stick projection of the same molecule shown in Figure 7.

FIGURE 9 is a further simplified ball and stick projection of $Cr(NC_4H_4)_3Cl(O_2C_2H_4(CH_3)_2)$, with the exception of the chromium atom which is represented as a thermal ellipsoid. This is the same molecule as that shown in Figures 7 and 8, however, the entire crystal structure or lattice is not shown.

## DETAILED DESCRIPTION OF THE INVENTION

### The Chromium Compounds

The inventive chromium compounds, which can be used preferably for olefin trimerization and, optionally, olefin polymerization, can be produced by forming a reaction mixture comprising a chromium salt, a metal amide, and any electron pair donor solvent, such as, for example, an ether. As used in this disclosure, the inventive chromium compounds are referred to by a variety of interchangeable names, such as inventive or novel chromium compound(s), chromium complex(es), chromium pyrrole complex(es) and/or chromium pyrrolide.

The chromium salt can be one or more organic or inorganic chromium salts, wherein the chromium oxidation state is from 0 to 6. As used in this disclosure, chromium metal is included in this definition of a chromium salt. Generally, the chromium salt will have a formula of $CrX_n$, wherein X can be the same or different and can be any organic or inorganic radical, and n is an integer from 1 to 6. Exemplary organic radicals can have from about 1 to about 20 carbon atoms, and are selected from alkoxy, ester, ketone, and/or amido radicals. The organic radicals can be straight-chained or branched, cyclic or acyclic, aromatic or aliphatic, and can be made of mixed aliphatic, aromatic, and/or cycloaliphatic groups. Exemplary inorganic radicals include, but are not limited to halides, sulfates, and/or oxides.

Preferably, the chromium salt is a halide, such as, for example chromous bromide, chromic bromide, chromous iodide, chromic iodide, chromous fluoride, chromic fluoride, chromous chloride, chromic chloride, and mixtures thereof. Most preferably, the chromium salt is a chloride, such as, for example chromous chloride and/or chromic chloride, due to simple separation of the reaction by-products such as, for example, sodium chloride, as well as relatively low cost.

The metal amide can be any metal amide that will react a chromium salt to form a chromium-amido complex. Broadly, the metal amide can be any heteroleptic or homoleptic metal complex or salt, wherein the amide radical can be any nitrogen-containing organic radical. The metal amide can be either affirmatively added to the reaction, or generated in-situ. Generally, the metal amide will have from about 1 to about 20 carbon atoms. Exemplary metal amides include, but are not limited to, primary and/or secondary amines, any alkali metal (Group IA, and including hydrogen, of the Periodic Table) amide and/or any alkaline earth metal (Group IIA of the Periodic Table) amide. The hydrocarbyl portion of the salt of the metal amide is selected from the group consisting of straight chain or branched, cyclic or acyclic, aromatic or aliphatic, and mixtures of two or more thereof. Preferably, the metal amide is selected from a Group IA, and including hydrogen, or Group IIA metal amide, due to ease of reaction with chromium halides.

Exemplary preferred metal amides include, but are not limited to, lithium dimethylamide, lithium diethylamide, lithium diisopropylamide, lithium dicyclohexylamide, sodium bis(trimethylsilyl)amide, sodium indolide, sodium pyrrolide, and mixtures of two or more thereof. Most preferably, the metal amide is a pyrrolide. As used in this disclosure, a pyrrolide is defined as a compound comprising a 5-membered, nitrogen-containing heterocycle, such as, for example, pyrrole, derivatives of pyrrole, and mixtures thereof. Exemplary pyrrolides are selected from the group consisting of hydrogen pyrrolide (pyrrole), lithium pyrrolide, sodium pyrrolide, potassium pyrrolide, cesium pyrrolide, and/or the salts of substituted pyrrolides, because of high reactivity and activity with the other reactants. Examples of salts of substituted pyrrolides include, but are not limited to sodium 2,5-dimethyl pyrrolide and/or 3,4-dimethyl pyrrolide. When the metal amide is a pyrrolide ligand, the resultant chromium compound is a chromium pyrrolide.

The electron pair donor solvent can be any electron pair donor solvent to affect a reaction between the chromium salt and the metal amide. While not wishing to be bound by theory, it is believed that the electron pair donor solvent can be a reaction solvent, as well as a possible reactant. Exemplary electron pair donor solvents include, but are not limited to, nitrogen-, oxygen-, phosphorous-, and/or sulfur-containing compounds and/or ethers.

Exemplary nitrogen-containing compounds include, but are not limited to nitriles, such as, for example, acetonitrile; amines, such as, for example, pyridine, and/or derivatives of pyridine; and/or amides. Additional exemplary nitrogen-containing compounds include, but are not limited to, nitromethane, dimethylpyridine, dimethylformamide, N-methylformamide, aniline, nitrobenzene, tetramethyldiaminomethane, hexamethyldisilazane, and/or pyrrolidone.

Exemplary oxygen-containing compounds include, but are not limited to, acetone, ethyl acetate, methyl acetate, methanol, ethanol, ethyl methyl ketone, acetaldehyde, furan, and/or hexamethyldisiloxane.

Exemplary phosphorous-containing compounds include, but are not limited to, hexamethylphosphoramide, hexamethylphosphorous triamide, triethylphosphite, tributylphosphine oxide, and/or triethylphosphine.

Exemplary sulfur-containing compounds include, but are not limited to, carbon disulfide, dimethylsulfoxide, tetramethylene sulfone, thiophene, and/or dimethylsulfide or mixtures thereof.

The ether as one particular electron pair donor solvent in the reaction mixture can be one or more ether compounds to effect a reaction between the chromium salt and the metal amide. While not wishing to be bound by theory, it is believed that the ether can be a reaction solvent, as well as a possible reactant. The ether can be any aliphatic and/or aromatic compound containing an R-O-R functionality, wherein the R groups can be the same or different, but preferably is not hydrogen. Preferred ethers are aliphatic ethers, for safety reasons in that aromatic ethers are human toxins. Furthermore, the preferred ethers are those which facilitate a reaction between a chromium halide and a Group

IA or Group IIA metal pyrrolide, and also can be easily removed from the reaction mixture. Exemplary compounds include, but are not limited to, tetrahydrofuran, dioxane, diethylether, dimethoxyethane (glyme), diglyme, triglyme, and mixtures of two or more thereof. Most preferably, the ether is selected from tetrahydrofuran, derivatives of tetrahydrofuran, dimethoxyethane, derivatives of dimethoxyethane, and mixtures thereof, for the reasons given above, as well as the reason that the preferred salt of an amine is soluble in these ethers.

The amount of each reactant used to prepare one or more of the novel chromium compounds can vary, based on the desired chromium compound product. Any amount of each reactant can be used to produce the novel chromium compounds, depending on the desired product. Different reaction stoichiometries can produce different chromium compounds. For example, the reaction of about one mole of chromium (II) with about two moles of sodium pyrrolide can produce different products than reacting about one mole of chromium (II) with an excess of sodium pyrrolide. Furthermore, as stated earlier, selection of different, although similar reactants, can produce different products. For example, using either tetrahydrofuran or dimethoxyethane can result in a different reaction product.

The three reactants can be combined in any manner under conditions suitable to form a solution comprising one or more of the inventive chromium compounds. The reaction preferably occurs in the absence of oxygen and therefore under an inert, such as, for example, nitrogen and/or argon. The reaction pressure can be any pressure sufficient to maintain the reactants in a liquid state. Generally, pressure within the range of from about atmospheric pressure to about three atmospheres are acceptable. For ease of operation atmospheric pressure is generally employed.

The reaction temperature solvent is at the boiling point of the solvent and the reaction mixture is refluxed for a period of time.

The reaction time can be any amount of time necessary for the reaction to occur. Depending on the reactants, as well as the reaction temperature and pressure, reaction time can vary from about 1 minute to about 1 week. Usually, reaction time ranges from about 3 hours to about 5 days. Under optimum conditions, the reaction time can be within the range of from about 3 to about 48 hours.

After the reaction is complete, a solid reaction product can be recovered by any method known in the art. Preferably, though not required, upon completion of the reaction, the reaction mixture first is filtered to remove any reaction by-products such as, for example, salts, like sodium chloride, prior to any other treatment. Although removal of any reaction by-products is not necessary, such removal preferably is done in order to expedite later purification of the chromium product. After filtering, one exemplary method to recover a solid reaction product is to remove the excess ether as an electron pair donor solvent, from the reaction mixture. The excess electron pair donor solvent, such as, for example an ether, can be removed according to any method known in the art. Exemplary electron pair donor solvent, such as, for example an ether, removal methods include, but are not limited to, slow evaporation, under vacuum and/or a nitrogen purge.

Other electron pair donor solvent, such as, for example an ether, removal procedures can be used either alone or in combination. For example, the reaction mixture can be filtered and then vacuum dried. Preferably, the reaction mixture is heated slowly and maintained at temperature within the range of about 10° to about 300°C, preferably about 25° to about 200°C, under a vacuum, for safety, to remove the excess electron pair donor solvent, such as, for example an ether. The resultant solid reaction product is one or more of the inventive chromium compounds.

Alternatively, the reaction mixture can be filtered to remove any solid reaction by-product solids and the filtered reaction mixture can be contacted with a non-polar organic solvent. Addition of a non-polar organic solvent causes one or more of the inventive chromium compounds to form a solid precipitate. Exemplary non-polar organic solvents include, but are not limited to, pentane, hexane, cyclohexane, heptene, and mixtures thereof. Most preferably pentane is added to the filtered reaction mixture because of the availability and ease of use. The precipitated inventive chromium compounds can be recovered by any method known in the art. The simplest procedure to recover the inventive precipitated chromium compounds is by filtration.

The reaction mixture and the resultant solid reaction products, as stated earlier, are kept in an oxygen-free atmosphere at all times. Preferably, due to availability and ease of use, an inert atmosphere such as, for example, nitrogen, is the ambient.

Numerous chromium compounds can be prepared in accordance to the invention, by varying the reactants and/or the quantity of each reactant employed. The recovered, novel chromium compound or compounds can be used for olefin trimerization and/or polymerization without further purification.

Optionally, the chromium compound can be purified in accordance with any method known in the art. For example, one of the simplest purification procedures is to wash the recovered solid with a non-polar organic solvent such as, for example, toluene. Preferably, a non-polar aliphatic organic solvent is used for best results. Exemplary wash solvents include, but are not limited to, pentane, hexane, cyclohexane, heptane, and mixtures thereof. Most preferably, pentane is the wash solvent.

The inventive chromium compounds can be used as a supported and/or unsupported catalyst for olefin trimerization and/or polymerization. A supported chromium catalyst can be prepared according to any method known in the art. Any support useful to support chromium catalyst can be used. Exemplary catalyst supports include, but are not limited to,

inorganic oxides, either alone or in combination, phosphated inorganic oxides, and mixtures thereof. Particularly preferred are supports selected from the group consisting of silica, silica-alumina, alumina, fluorided alumina, silated alumina, thoria, aluminophosphate, aluminum phosphate, phosphated silica, phosphated alumina, silica-titania, coprecipitated silica/titania, and mixtures, thereof, fluorided/silated alumina, being presently preferred, as well as any one or more of these supports which can contain chromium. The presently most preferred catalyst support is because of the greatest trimerization activity, is aluminophosphate, as disclosed in U.S. Patent 4,364,855 (1982), herein incorporated by reference.

The supported chromium catalyst system can be prepared according to any method known in the art. For example, the reaction mixture, which preferably has been filtered to remove any particulate reaction by-products and contains one or more of the novel chromium pyrrolide compounds, is combined and thoroughly contacted with a catalyst support. Excess electron pair donor solvent, such as, for example an ether, does not have to be removed prior to contacting the catalyst support. However, a solid chromium pyrrolide compound can be re-dissolved in an electron pair donor solvent, such as, for example an ether, if desired. The chromium pyrrolide/ether solution is usually a blue or blue/green color, although other colors can be observed.

The catalyst support usually is insoluble in the ether/chromium pyrrolide complex solution, said ether being the electron pair donor solvent. Any excess of the chromium pyrrolide in relation to the catalyst support is sufficient. However, usually, at least about 5 grams of chromium pyrrolide compound per gram of catalyst support is sufficient. Preferably about 0.001 to about 1 grams of chromium pyrrolide compound per gram of support, and most preferably about 0.01 to about 0.5 grams of chromium pyrrolide compound per gram of support is used for best support loading and most efficient use of the reagents. This mixture can be contacted and mixed at any time, temperature, and pressure to thoroughly contact the chromium pyrrolide compound and support. For ease of use, ambient temperatures and pressures are preferred. Mixing times can be up to about 24 hours, preferably about 5 seconds to about 10 hours, and most preferably about 5 seconds to about 8 hours. Longer times usually provide no additional benefit and shorter times can be insufficient for thorough contacting.

After the support is added and thoroughly combined with the chromium pyrrolide it is collected by filtration, vacuum dried, then an activating compound, usually as a solution of one or more Lewis acids and/or metal alkyls, preferably in a hydrocarbon solvent, is added to the support/chromium pyrrolide mixture. As used in this disclosure, a Lewis acid is defined as any compound that is an electron acceptor. Preferably, the activating compound is a compound that can be considered both a Lewis acid and a metal alkyl. Preferred activating compounds which are both a metal alkyl and a Lewis acid include, but are not limited to, alkylaluminum compounds, derivatives of alkylaluminum compounds, halogenated alkylaluminum compounds, and mixtures thereof. Exemplary compounds include, but are not limited to, triethylaluminum, diethylaluminum, ethylaluminum sesquichloride, and mixtures thereof. The most preferred alkylaluminum compound is triethylaluminum, for best results in catalyst activity.

The hydrocarbon solvent can be any hydrocarbon that will dissolve the Lewis acid. Preferred hydrocarbons include, but are not limited to, aromatic compounds having from about 6 to about 50 carbon atoms per molecule. Most preferably, the hydrocarbon solvent is toluene, for ease of removal and minimal interference with the resultant catalyst.

Any amount of activating compound, such as a metal alkyl and/or a Lewis acid is sufficient to activate and/or react with the chromium pyrrolide catalyst. Usually about 200 grams of Lewis acid per gram of chromium can be used. Preferably, about 1 to about 100 grams of activating compound, such as a metal alkyl and/or a Lewis acid per gram of chromium pyrrolide, and most preferably about 5 to about 30 grams of activating compound, such as a metal alkyl and/or a Lewis acid per gram of chromium pyrrolide are used, for best catalyst activity. However, the amount of Lewis acid employed can vary with the catalyst support used. For example, if the support is silica and/or alumina, too much activating compound, such as a metal alkyl and/or a Lewis acid can decrease catalyst activity. However, a similar amount of activating compound, such as a metal alkyl and/or a Lewis acid used with an aluminophosphate support does not always significantly decrease catalyst activity.

As disclosed earlier, the mixture of chromium pyrrolide, catalyst support, and activating compound, such as a metal alkyl and/or a Lewis acid are mixed and/or contacted under a dry, inert atmosphere at all times. Any pressure can be used during the contacting; for ease of use, atmospheric pressure is preferred. Any temperature can be used during the contacting; for ease of use, room temperature, or ambient temperature, is preferred. Some care should be taken during the mixing, so as not to destroy the physical integrity of the chromium pyrrolide, catalyst support, and resultant supported catalyst. The three-component mixture can be contacted for any amount of time sufficient to prepare and activate a chromium catalyst. Usually times in the range of about one minute to about one week are sufficient. Preferably, times in the range of about 30 minutes to about 24 hours are used, and most preferably times in the range of about one hour to about 12 hours are used. Too short of mixing times can result in incomplete contacting and too long of mixing times will not provide any additional catalytic benefit.

An alternative, and presently preferred, method to produce a supported catalyst is to combine one or more solid, inventive chromium pyrrolide compounds with a hydrocarbon solvent, as disclosed earlier, such as, for example, toluene, and a activating compound, such as a metal alkyl and/or an activating compound, as disclosed earlier, such as

a solution of one or more Lewis acids and/or metal alkyls, preferably in a hydrocarbon solvent such as, for example, triethylaluminum. This mixture can be stirred for any time sufficient to dissolve the chromium pyrrolide compound, at any pressure or temperature. Usually, times of about one minute to about one week, preferably about one hour to about 24 hours, and most preferably within the range of about three hours to about 12 hours are used. For ease of operation, ambient temperatures and pressures are used. Usually, a brown solution will result.

After the solution is sufficiently mixed, a support is added to the solution and stirred to thoroughly contact the solution and support. The quantity of support is any amount sufficient to support the chromium pyrrolide compound. Generally, the amount of support necessary is the same as that disclosed in the previous exemplary process. Any suitable pressure and temperature can be used, although ambient temperature and pressure are preferred for ease of use. Usually, the mixing and/or contacting time is within the range of about 30 minutes to about one week, preferably from about 3 hours to about 48 hours. Most preferably, the mixing and/or contacting time is within the range of about 5 hours to about 24 hours, to maximize efficiency and result in a thoroughly contacted support.

The solution then can be filtered to recover a solid catalytic product. The catalytic product, as with the reactants and reactions, is preferably kept under an inert atmosphere to maintain chemical stability.

If the chromium compound, such as, for example, a chromium pyrrolide, is recovered and is to be used as an unsupported trimerization and/or polymerization catalyst, olefins can be trimerized or polymerized in the presence of one or more of the inventive homogeneous chromium compounds, a saturated hydrocarbon as a diluent, and an activating compound such as a solution of one or more Lewis acids and/or metal alkyls, preferably in a hydrocarbon solvent. Optionally, hydrogen can be added to the reactor to accelerate the reaction.

## Reactants

Reactants applicable for use in polymerization with the catalyst and processes of this invention are olefinic compounds which can polymerize, i.e., react the same or with other olefinic compounds. Catalyst of the invention can be used to polymerize at least one linear or branched mono-1-olefin having about 2 to about 8 carbon atoms. Exemplary compounds include, but are not limited to, ethylene, propylene, 1-butene, 1-pentene, 1-hexene, 1-octene, and mixtures thereof.

Reactants applicable for use in the trimerization process, i.e., the combination of any three olefins, of this invention are olefinic compounds which can a) self-react, i.e., trimerize, to give useful products such as, for example, the self reaction of ethylene can give one hexene; and/or b) olefinic compounds which can react with other olefinic compounds, i.e., co-trimerize, to give useful products such as, for example, co-trimerization of ethylene plus hexene can give one decene and/or 1-tetradecene, co-trimerization of ethylene and 1-butene gives one octene, or 1-decene and ethylene can give 1-tetradecene and/or 1-docosene. As used herein, the term "trimerization" is intended to include "co-trimerization" as defined above.

Suitable trimerizable olefinic compounds are those compounds having from about 2 to about 30 carbon atoms per molecule and having at least one olefinic double bond. Exemplary compounds include, but are not limited to acyclic and cyclic olefins such as, for example, ethylene, propylene, 1-butene, 2-butene, isobutylene, 1-pentene, 2-pentene, 1-hexene, 2-hexene, 3-hexene, 1-heptene, 2-heptene, 3-heptene, the four normal octenes, the four normal nonenes, and mixtures of any two or more thereof. If branched and/or cyclic olefins are used as reactants, while not wishing to be bound by theory, it is believed that steric hindrance could hinder the trimerization process. Therefore, the branched and/or cyclic portion(s) of the olefin should be distant from the carbon-carbon double bond.

## Reaction Conditions

The reaction products, i.e., trimers and/or polymers, can be prepared from the catalyst systems of this invention by solution reactions, slurry reactions, and/or gas phased reaction techniques using conventional equipment and contacting processes. Contacting of the monomer or monomers with the catalyst system can be effected by any manner known in the art of solid catalyst. One convenient method is to suspend the catalyst system in an organic medium and to agitate the mixture to maintain the catalyst system in suspension throughout the trimerization and/or polymerization process. Other known contacting methods such as fluidized bed, gravitating bed, and fixed bed can also be employed. Optionally, hydrogen can be added to the reactor to accelerate the reaction.

The catalyst systems of this invention are particularly suitable for use in slurry trimerization and/or polymerizations. The slurry process is generally carried out in an inert diluent (medium), such as a paraffin, cycloparaffin, or aromatic hydrocarbon. One exemplary reactor diluent is isobutane. When the reactant is predominately ethylene, a temperature in the range of about 60° to about 110°C is employed.

Products

The olefinic and/or polymeric products of this invention have established utility in a wide variety of application such as, for example, as monomers for use in the preparation of homopolymers, copolymers, and/or terpolymers. The polymeric products of this invention have established utility in a wide variety of application such as for example, polyethylene.

The further understanding of the present invention and its advantages will be provided by reference to the following examples.

Examples

Preparation of Chromium-Containing Compounds

Manipulations of all reactants were carried out either in a drybox employing nitrogen, or in airless glassware employing vacuum or nitrogen. Tetrahydrofuran (THF), toluene, benzene, diethylbenzene (Aldrich, 97% mixture of 1,2-, 1,3- 1,4- isomers) and pentane were purified by distillation over sodiumbenzophenenone ketyl under nitrogen, then degassed via a nitrogen purge. Dimethoxyethane (DME) (Aldrich, anhydrous) was degassed via nitrogen purge and used without further purification. Pyrrole (Aldrich, 98%) was vacuum distilled over sodium, then degassed via nitrogen purge. 2,5-Dimethylpyrrole was dried with calcium sulfate and vacuum distilled. Sodium 2,5-dimethylpyrrol ($NaC_6H_8N$) was prepared by reacting 2,3-dimethylpyrrole with an excess of sodium (40% by weight dispersion in mineral spirits) in refluxing tetrahydrofuran under nitrogen. Sodium pyrrolide was prepared by reacting pyrrole with an equivalent molar amount (1:1) of NaH (Aldrich, 60% by weight in mineral oil) or sodium (40% dispersion by weight in mineral spirits) in dimethoxyethane or tetrahydrofuran (THF) at ambient temperature under nitrogen. Triethylaluminum (TEA) (Aldrich 1.0M, hexanes and 1.9M Toluene) was used as received. Ketjen Grade B alumina ($Al_2O_3$) and Davison 952 silica ($SiO_2$) were the commercial materials used as supports for catalyst preparations. Fluorided-alumina ($F/Al_2O_3$, 15 wt% F) was prepared by the addition of a solution of $NH_4HF_2$ in methanol to Ketjen Grade B alumina. Phosphated silica ($P/SiO_2$, P/Si molar ratio = 0.1) was prepared by the addition of a 10% $H_3PO_4$/methanol solution to Davison 952 silica. The aluminophosphate ($AlPO_4$) used in the following experiments was made as described in McDaniel et al, U.S. Patent No. 4,364,855 (1982). The supports were activated by placing up to 25g into a fritted quartz tube, fluidizing with air and calcining at 700°C, except for $P/SiO_2$ at 350°C, for 3 hours. The air stream was switched to nitrogen until the support cooled to ambient temperature.

Chromium pyrrolide complexes were typically prepared from anhydrous chromium (II or III) chloride and sodium pyrrolide as follows:

A typical synthetic procedure useful to prepare chromium pyrrolide complexes was that of reacting the chromium chlorides with sodium pyrrolide ($NaC_4H_4N$, also referred to as NaPy) in refluxing tetrahydrofuran (THF). A molar reactant stoichiometry of $1CrCl_2$ and 2NaPy resulted in the isolation of a polymeric material, Product II, as the major product and a pentanuclear complex, Product I, ($Cr_5(NC_4H_4)_{10}(OC_4H_8)_4$), as the minor product, see Equation 1. Using molar excess of NaPy resulted in the isolation of the dianionic square planar complex $\{Cr(NC_4H_4)_4\}\{Na\}_2 \cdot 2OC_4H_8$, Product III, and the octahedral complex $\{Cr(C_4H_4N)_5(0C_4H_8)\}\{Na\}_2 \cdot 4OC_4H_8$, Product IV, see Equation 2. Each of the products was isolated through precipitation, Product II, or crystallization, Products I, III, IV, from THF solutions by the addition of pentane.

$$1CrCl_2 + 2NaPy \xrightarrow[\text{Nitrogen Atm.}]{\text{THF, Reflux, 20 hrs.}} \underset{\text{Pentanuclear Complex, (I) Product I}}{Cr_5(C_4H_4N)_{10}(C_4H_8O)_4} + \underset{\text{Product II (Major)}}{\text{Polymeric Complex, (II)}} \quad 1)$$

Equation 1

$$CrCl_2 + \underset{\text{(Excess)}}{NaPy} \xrightarrow[\text{Nitrogen Atm.}]{\text{THF, Reflux, 2 hrs.}} \underset{\text{Square Planar Cr(II), Product III (Major)}}{\{Cr(C_4H_4N)_4\}\{Na\}_2 \cdot 2OC_4H_8} + \underset{\text{Octahedral Cr(III), Product IV (Minor)}}{\{Cr(C_4H_4N)_5(OC_4H_8)\}\{Na\}_2 \cdot 4OC_4H_8} \quad 2)$$

Equation 2

Example I

To prepare the pentanuclear complex, Product I, ($Cr_5(NC_4H_4)_{10}$,$(OC_4H_8)_4$), and the polymeric material, Product II, chromous chloride (2.0g/16.27 mmole) was combined with sodium pyrrolide (33.68mmole) in tetrahydrofuran and refluxed 20 hours. The reaction mixture was filtered (medium porosity frit) and the filtrate was used for fractional crystallization of both ($Cr_5(NC_4H_4)_{10}(OC_4H_8)_4$), Product I, and the polymeric material, Product II, through the addition of pentane. The polymeric material crystallized as a blue solid followed by ($Cr_5(NC_4H_4)_{10}(OC_4H_8)_4$) as opaque dark blue/purple crystals.

Analysis calculated for $C_{56}H_{72}N_{10}Cr_5O_4$, Product I: C, 55.62; H, 6.00; N, 11.58%, by weight. Found: C, 55.46; H, 6.32; N, 11.15%, by weight. Analysis found for Product II: Cr, 11.5; C, 59.75; H, 7.61; N, 9.17%, by weight, but variable upon precipitation conditions. An x-ray crystal structure of Product I showed a pentanuclear complex incorporating bridging amido-pyrrolyl, terminal amido-pyrrolyl, and tetrahydrofuran ligands (Figures 1 and 2).

Example II

To prepare $\{Cr(NC_4H_4)_4\}\{Na\}_2 \cdot 2OC_4H_8$, Product III, and $\{Cr(C_4H_4N)_5(OC_4H_8)\}\{Na\}_2 \cdot 4OC_4H_8$, Product IV, chro-

mous chloride (3.0g/24.4mmole) was combined with sodium pyrrolide (100.9mmole) in tetrahydrofuran and refluxed 2 hours, see Equation 2. The reaction mixture was filtered (medium porosity frit) and the filtrate was used for fractional crystallization of both $\{Cr(NC_4H_4)_4\}\{Na\}_2 \cdot 2OC_4H_8$, Product III, and $\{Cr(C_4H_4N)_5(OC_4H_8)\}\{Na\}_2 \cdot 4OC_4H_8$, Product IV, through the addition of pentane. Product III crystallized as translucent orange/ red crystals followed by Product IV as translucent purple crystals. While not wishing to be bound by theory, the formation of Product IV is believed to result from the presence of chromic chloride in the chromous chloride reagent (Alfa, chromium (II) chloride, anhydrous, contains 5-10% by weight $CrCl_3$) used in the preparation.

Analysis calculated for $C_{24}H_{32}N_4O_2CrNa_2$, Product III: C, 56.94; H, 6.32; N, 11.07% by weight. Found: C, 57.04; H, 6.30; N, 10.92%, by weight. Analysis calculated for $C_{40}H_{60}N_5O_5CrNa_2$, Product IV: C, 60.90; H, 7.67; N, 8.88% by weight. Found: C, 60.81; H, 7.74; N, 9.44%, by weight. An x-ray crystal structure of Product III showed a square planar complex incorporating terminal amido-pyrrolyl ligands (Figure 4). An x-ray crystal structure of Product IV showed an octahedral complex incorporating terminal amido-pyrrolyl and a tetrahydrofuran ligand (Figures 5 and 6).

Example III

The reaction product obtained from sodium pyrrolide and $CrCl_3$ was the most preferred in the preparation of an active catalyst. Pyrrole (7.0ml/100.9mmole) was mixed with NaH (4.2g of 60%, about 105 mmole) in dimethoxyethane at ambient temperature until bubbling ceased. Chromic chloride (5.33g/33.7mmole) was added to the solution at ambient temperature. The reaction mixture was refluxed under nitrogen for five hours, see Equation 3. This resulted in a dark green solution. The solution was filtered (medium porosity frit) and stripped of solvent under vacuum and pumped dry under vacuum for 12 hours. The resultant chromium pyrrolide complex was a green solid, Product V. It was used in the preparation of an active catalyst without further purification.

$$1CrCl_3 + 3NaPy \xrightarrow[\text{Nitrogen Atm.}]{\text{DME, Reflux, 5 hrs.}} \text{Green Solid (V)} \qquad 3)$$

Equation 3

Example IV

All single crystal x-ray structure analyses were performed by Crystalytics Company, Lincoln, Nebraska. Examples IV, V, VI, and IX contain the resultant analytical and subsequently computer-generated data.

A single crystal x-ray structure was obtained for $[Cr_5(NC_4H_4)_{10}(OC_4H_8)_4]$, Product I, and shown in Figure 1 and Figure 2. The description of the single-crystal sample and mounting used for data collection are as follows:

Color: Dark blue
Shape: Rectangular parallelepiped
Dimensions: 0.20 x 0.48 x 0.80 mm
Crystal Mount: Crystal was sealed inside a thin-walled glass capillary with epoxy under $N_2$.
Crystal Orientation: Crystal was oriented with its longest edge nearly parallel to the phi axis of the diffractometer.
Width at Half-height from ω Scans: 0-38°

The space group and cell data are as follows:

Crystal System: Triclinic
Space Group and Number: P1-$C_i$ (No. 2)
Number of Computer-Centered Reflections Used in the Least-Squares Refinement of the Cell Dimensions:
15 20>25° °C=2θ±1°
Lattice Constants with esd's:

| | | |
|---|---|---|
| a = 10.803(2)Å | $\alpha$ = 85.59(2)° | V = 1407.9(6)$Å^3$ |
| b = 9.825(2)Å | $\beta$ = 96.23(2)° | Z = 1 |
| c = 14.212(4)Å | $\gamma$ = 109.99(2)° | $\lambda$ = 0.71073Å |

Molecular Weight: 1209.24 amu
Calculated Density: 1.427 $g/cm^3$
Linear Absorption Coefficient:[3a] 0.96 $mm^{-1}$

Tables I - V list the resultant parameters used to generate the molecular structures shown in Figure 1 and Figure 2.

Table I. Atomic Coordinates for Nonhydrogen Atoms in Crystalline $[Cr_5(NC_4H_4)_{10}(OC_4H_8)_4]$ [a]

| Atom Type [b] | Fractional Coordinates $10^4x$ | $10^4y$ | $10^4z$ | Equivalent Isotropic Thermal Parameter, B, $Å^2 \times 10$ [c] |
|---|---|---|---|---|
| $Cr_1$ | 0 [d] | 0 [d] | 0 [d] | 25(1) |
| $Cr_2$ | 636(1) | 2281(1) | 1500(1) | 24(1) |
| $Cr_3$ | -1179(1) | 841(1) | 3122(1) | 28(1) |
| $N_{1a}$ | -1155(3) | 935(3) | 715(2) | 25(1) |
| $C_{1a}$ | -2195(4) | 64(4) | 1231(3) | 31(1) |
| $C_{2a}$ | -3313(4) | 390(5) | 965(3) | 41(1) |
| $C_{3a}$ | -3014(4) | 1486(5) | 257(3) | 43(1) |
| $C_{4a}$ | -1728(4) | 1791(4) | 116(3) | 34(1) |
| $N_{1b}$ | 1566(3) | 1902(3) | 331(2) | 29(1) |
| $C_{1b}$ | 1753(4) | 3095(4) | -308(3) | 36(1) |
| $C_{2b}$ | 3035(5) | 3751(5) | -432(3) | 51(2) |
| $C_{3b}$ | 3736(4) | 2986(5) | 131(3) | 51(2) |
| $C_{4b}$ | 2823(4) | 1865(4) | 587(3) | 38(1) |
| $N_{1c}$ | -320(3) | 2997(3) | 2480(2) | 27(1) |
| $C_{1c}$ | 375(4) | 3732(4) | 3273(3) | 34(1) |
| $C_{2c}$ | 29(5) | 4919(4) | 3383(3) | 43(1) |
| $C_{3c}$ | -908(5) | 4967(4) | 2631(3) | 42(1) |
| $C_{4c}$ | -1105(4) | 3809(4) | 2101(3) | 32(1) |
| $N_{1d}$ | 443(3) | 350(3) | 2743(2) | 28(1) |
| $C_{1d}$ | 1600(4) | 715(4) | 3289(3) | 36(1) |
| $C_{2d}$ | 2321(4) | -133(5) | 3102(3) | 46(2) |
| $C_{3d}$ | 1567(5) | -1070(5) | 2403(3) | 46(2) |
| $C_{4d}$ | 422(4) | -763(4) | 2203(3) | 36(1) |

Table I. (continued)

| Atom Type [b] | Fractional Coordinates $10^4x$ | $10^4y$ | $10^4z$ | Equivalent Isotropic Thermal Parameter, B, $Å^2$ x 10 [c] |
|---|---|---|---|---|
| $N_{1e}$ | -1972(3) | -1122(3) | 3801(2) | 35(1) |
| $C_{1e}$ | -1344(5) | -2107(4) | 4069(3) | 41(1) |
| $C_{2e}$ | -2189(5) | -3307(4) | 4503(3) | 44(1) |
| $C_{3e}$ | -3361(5) | -3061(4) | 4531(3) | 47(1) |
| $C_{4e}$ | -3206(5) | -1731(4) | 4097(3) | 47(1) |
| $O_{1f}$ | 2351(3) | 3985(3) | 1883(2) | 32(1) |
| $C_{1f}$ | 3536(4) | 4018(4) | 2483(3) | 43(1) |
| $C_{2f}$ | 4470(6) | 5479(6) | 2336(5) | 76(2) |
| $C_{3f}$ | 3642(5) | 6408(5) | 2147(4) | 62(2) |
| $C_{4f}$ | 2396(4) | 5463(4) | 1635(3) | 40(1) |
| $O_{1g}$ | -2551(3) | 1543(3) | 3659(2) | 35(1) |
| $C_{1g}$ | -3763(4) | 1733(5) | 3232(3) | 44(1) |
| $C_{2g}$ | -4097(5) | 2625(6) | 3907(4) | 57(2) |
| $C_{3g}$ | -3524(5) | 2241(6) | 4845(3) | 57(2) |
| $C_{4g}$ | -2319(5) | 1977(6) | 4633(3) | 50(2) |

[a] The numbers in parentheses are the estimated standard deviations in the last significant digit.
[b] Atoms are labeled in agreement with Figure I.
[c] This is one-third of the trace of the orthogonalized $B_{ij}$ tensor.
[d] This is a symmetry-required value and is therefore listed without an estimated standard deviation.

Table II. Anisotropic Thermal Parameters for Nonhydrogen Atoms in Crystalline $[Cr_5(NC_4H_4)_{10}(OC_4H_8)_4]$ [a,b]

| Atom Type [c] | Anisotropic Thermal Parameter ($Å^2$ x 10) $B_{11}$ | $B_{22}$ | $B_{33}$ | $B_{12}$ | $B_{13}$ | $B_{23}$ |
|---|---|---|---|---|---|---|
| $Cr_1$ | 20(1) | 23(1) | 32(1) | 5(1) | 5(1) | -4(1) |
| $Cr_2$ | 23(1) | 22(1) | 27(1) | 7(1) | 3(1) | -2(1) |
| $Cr_3$ | 27(1) | 26(1) | 34(1) | 11(1) | 8(1) | 1(1) |
| $N_{1a}$ | 21(1) | 27(1) | 29(1) | 8(1) | 1(1) | -2(1) |
| $C_{1a}$ | 28(2) | 31(2) | 30(2) | 4(1) | 8(1) | -4(1) |
| $C_{2a}$ | 23(2) | 49(2) | 49(2) | 8(2) | 5(2) | -16(2) |
| $C_{3a}$ | 31(2) | 51(2) | 52(2) | 22(2) | -7(2) | -11(2) |
| $C_{4a}$ | 36(2) | 32(2) | 34(2) | 15(1) | -2(1) | -3(1) |
| $N_{1b}$ | 24(1) | 25(1) | 35(1) | 3(1) | 5(1) | -4(1) |
| $C_{1b}$ | 40(2) | 31(2) | 33(2) | 2(1) | 11(1) | -1(1) |
| $C_{2b}$ | 46(2) | 42(2) | 54(2) | -7(2) | 24(2) | -5(2) |
| $C_{3b}$ | 25(2) | 50(2) | 71(3) | -3(2) | 15(2) | -27(2) |
| $C_{4b}$ | 29(2) | 38(2) | 48(2) | 10(1) | 0(2) | -15(2) |
| $N_{1c}$ | 28(1) | 25(1) | 30(1) | 11(1) | 3(1) | -2(1) |
| $C_{1c}$ | 36(2) | 35(2) | 31(2) | 10(1) | 4(1) | -3(1) |
| $C_{2c}$ | 52(2) | 34(2) | 43(2) | 13(2) | 6(2) | -13(1) |
| $C_{3c}$ | 51(2) | 31(2) | 50(2) | 22(2) | 5(2) | -5(2) |
| $C_{4c}$ | 35(2) | 34(2) | 31(2) | 16(1) | 4(1) | 1(1) |
| $C_{1d}$ | 32(1) | 23(1) | 31(1) | 12(1) | 6(1) | 3(1) |
| $C_{1d}$ | 33(2) | 32(2) | 42(2) | 9(1) | 6(2) | -0(1) |
| $C_{2d}$ | 36(2) | 50(2) | 59(2) | 24(2) | 6(2) | 11(2) |
| $C_{3d}$ | 61(3) | 44(2) | 47(2) | 36(2) | 11(2) | 3(2) |
| $C_{4d}$ | 49(2) | 35(2) | 31(2) | 23(2) | 4(2) | 1(1) |
| $N_{1e}$ | 36(2) | 30(1) | 42(2) | 13(1) | 14(1) | 4(1) |
| $C_{1e}$ | 46(2) | 36(2) | 46(2) | 20(2) | 10(2) | 6(2) |
| $C_{2e}$ | 64(3) | 30(2) | 37(2) | 15(2) | 7(2) | 4(1) |
| $C_{3e}$ | 55(3) | 31(2) | 46(2) | -1(2) | 18(2) | -0(2) |

Table II. (continued)

| Atom Type [c] | Anisotropic Thermal Parameter ($Å^2$ x 10) | | | | | |
|---|---|---|---|---|---|---|
| | $B_{11}$ | $B_{22}$ | $B_{33}$ | $B_{12}$ | $B_{13}$ | $B_{23}$ |
| $C_{4e}$ | 39(2) | 38(2) | 62(2) | 9(2) | 17(2) | 4(2) |
| $O_{1f}$ | 29(1) | 25(1) | 40(1) | 6(1) | -1(1) | -2(1) |
| $C_{1f}$ | 34(2) | 44(2) | 45(2) | 9(2) | -8(2) | -6(2) |
| $C_{2f}$ | 45(3) | 67(3) | 95(4) | -3(2) | -15(3) | -6(3) |
| $C_{3f}$ | 59(3) | 34(2) | 78(3) | -2(2) | -6(3) | -9(2) |
| $C_{4f}$ | 45(2) | 23(1) | 48(2) | 7(1) | 6(2) | -1(1) |
| $O_{1g}$ | 34(1) | 41(1) | 37(1) | 19(1) | 7(1) | -1(1) |
| $C_{1g}$ | 31(2) | 56(2) | 50(2) | 20(2) | 4(2) | -5(2) |
| $C_{2g}$ | 47(3) | 65(3) | 72(3) | 35(2) | 2(2) | -12(2) |
| $C_{3g}$ | 60(3) | 75(3) | 50(2) | 36(2) | 16(2) | -8(2) |
| $C_{4g}$ | 45(2) | 77(3) | 35(2) | 27(2) | 8(2) | -5(2) |

[a] The numbers in parentheses are the estimated standard deviations in the last significant digit.

[b] The form of the anisotropic thermal parameter is given in reference 8 on page 6 of the structure report.

[c] Atoms are labeled in agreement with Figure 1.

Table III. Atomic Coordinates for Hydrogen Atoms in Crystalline $[Cr_5(NC_4H_4)_{10}(OC_4H_8)_4]$ [a]

| Atom Type [b] | Fractional Coordinates $10^4x$ | $10^4y$ | $10^4z$ |
|---|---|---|---|
| $H_{1a}$ | -2129 | -661 | 1707 |
| $H_{2a}$ | -4154 | -55 | 1219 |
| $H_{3a}$ | -3608 | 1937 | -69 |
| $H_{4a}$ | -1267 | 2508 | -339 |
| $H_{1b}$ | 1053 | 3405 | -617 |
| $H_{2b}$ | 3405 | 4593 | -834 |
| $H_{3b}$ | 4676 | 3202 | 189 |
| $H_{4b}$ | 3031 | 1158 | 1020 |
| $H_{1c}$ | 1013 | 3445 | 3687 |
| $H_{2c}$ | 364 | 5592 | 3881 |
| $H_{3c}$ | -1331 | 5685 | 2512 |
| $H_{4c}$ | -1704 | 3580 | 1540 |
| $H_{1d}$ | 1881 | 1460 | 3743 |
| $H_{2d}$ | 3177 | -88 | 3396 |
| $H_{3d}$ | 1807 | -1790 | 2120 |
| $H_{4d}$ | -291 | -1252 | 1752 |
| $H_{1e}$ | -446 | -1976 | 3968 |
| $H_{2e}$ | -1997 | -4161 | 4742 |
| $H_{3e}$ | -4139 | -3699 | 4803 |
| $H_{4e}$ | -3878 | -1286 | 4012 |
| $H_{1fa}$ | 3351 | 3836 | 3136 |
| $H_{1fb}$ | 3882 | 3308 | 2299 |
| $H_{2fa}$ | 5068 | 5771 | 2893 |
| $H_{2fb}$ | 4965 | 5524 | 1806 |
| $H_{3fa}$ | 3462 | 6711 | 2728 |
| $H_{3fb}$ | 4068 | 7245 | 1757 |

Table III. (continued)

| Atom Type [b] | Fractional Coordinates $10^4x$ | $10^4y$ | $10^4z$ |
|---|---|---|---|
| $H_{4fa}$ | 2417 | 5653 | 964 |
| $H_{4fb}$ | 1641 | 5625 | 1839 |
| $H_{1ga}$ | -3631 | 2231 | 2623 |
| $H_{1gb}$ | -4455 | 813 | 3162 |
| $H_{2ga}$ | -5037 | 2381 | 3901 |
| $H_{2gb}$ | -3704 | 3640 | 3750 |
| $H_{3ga}$ | -4129 | 1385 | 5124 |
| $H_{3gb}$ | -3307 | 3025 | 5266 |
| $H_{4ga}$ | -2173 | 1220 | 5050 |
| $H_{4gb}$ | -1565 | 2846 | 4703 |

a Hydrogen atoms were included in the structure factor calculations as idealized atoms (assuming $sp^2$- or $sp^3$-hybridization of the carbon atoms and a C-H bond length of 0.96 Å) "riding" on their respective carbon atoms. The isotropic thermal parameter of each hydrogen atom was fixed at 1.2 times the equivalent isotropic thermal parameter of the carbon atom to which it is covalently bonded.

b Hydrogen atoms are labeled with the same numerical and literal subscripts as their carbon atoms with an additional literal subscript (a or b) where necessary to distinguish between hydrogen atoms bonded to the same carbon.

TABLE IV. Bond Lengths Involving Nonhydrogen Atoms in Crystalline $[Cr_5(NC_4H_4)_{10}(OC_4H_8)_4]$ [a]

| Type[b] | Length, Å | Type[b] | Length, Å |
|---|---|---|---|
| $Cr_1 \cdots Cr_2$ | 3.066(1) | $O_{1f}-C_{1f}$ | 1.451(5) |
| $Cr_2 \cdots Cr_3$ | 3.121(1) | $O_{1f}-C_{4f}$ | 1.453(5) |
| | | $O_{1g}-C_{1g}$ | 1.448(6) |
| $Cr_1-N_{1a}$ | 2.153(3) | $O_{1g}-C_{4g}$ | 1.451(5) |
| $Cr_1-N_{1b}$ | 2.092(3) | | |
| $Cr_2-N_{1a}$ | 2.178(3) | $C_{1a}-C_{2a}$ | 1.360(6) |
| $Cr_2-N_{1b}$ | 2.149(3) | $C_{2a}-C_{3a}$ | 1.395(6) |
| $Cr_2-N_{1c}$ | 2.112(4) | $C_{3a}-C_{4a}$ | 1.351(6) |
| $Cr_3-N_{1c}$ | 2.172(3) | $C_{1b}-C_{2b}$ | 1.338(6) |
| $Cr_3-N_{1d}$ | 2.101(4) | $C_{2b}-C_{3b}$ | 1.393(7) |
| $Cr_3-N_{1e}$ | 2.037(3) | $C_{3b}-C_{4b}$ | 1.376(6) |
| | | $C_{1c}-C_{2c}$ | 1.365(7) |
| $Cr_2-O_{1f}$ | 2.082(2) | $C_{2c}-C_{3c}$ | 1.400(6) |
| $Cr_3-O_{1g}$ | 2.068(3) | $C_{3c}-C_{4c}$ | 1.356(6) |
| | | $C_{1d}-C_{2d}$ | 1.376(7) |
| $N_{1a}-C_{1a}$ | 1.399(4) | $C_{2d}-C_{3d}$ | 1.396(6) |
| $N_{1a}-C_{4a}$ | 1.397(5) | $C_{3d}-C_{4d}$ | 1.367(8) |
| $N_{1b}-C_{1b}$ | 1.398(5) | $C_{1e}-C_{2e}$ | 1.370(5) |
| $N_{1b}-C_{4b}$ | 1.379(6) | $C_{2e}-C_{3e}$ | 1.374(8) |
| $N_{1c}-C_{1c}$ | 1.388(4) | $C_{3e}-C_{4e}$ | 1.366(6) |

Table IV. (continued)

| Type [b] | Length, Å | Type [b] | Length, Å |
|---|---|---|---|
| $N_{1c}$-$C_{4c}$ | 1.394(6) | | |
| $N_{1d}$-$C_{1d}$ | 1.349(5) | $C_{1f}$-$C_{2f}$ | 1.460(6) |
| $N_{1d}$-$C_{4d}$ | 1.377(5) | $C_{2f}$-$C_{3f}$ | 1.474(9) |
| $N_{1e}$-$C_{1e}$ | 1.370(6) | $C_{3f}$-$C_{4f}$ | 1.496(6) |
| $N_{1e}$-$C_{4e}$ | 1.361(6) | $C_{1g}$-$C_{4g}$ | 1.496(8) |
| | | $C_{2g}$-$C_{3g}$ | 1.485(7) |
| | | $C_{3g}$-$C_{4g}$ | 1.476(9) |

[a] The numbers in parentheses are the estimated standard deviations in the last significant digit.
[b] Atoms are labeled in agreement with Figure 1.

Table V. Bond Angles Involving Nonhydrogen Atoms in Crystalline $[Cr_5(NC_4H_4)_{10}(OC_4H_8)_4]$ [a]

| Type[b] | Angle,deg | Type[b] | Angle,deg |
|---|---|---|---|
| $N_{1a}Cr_1N_{1b}$ | 84.8(1) | $Cr_1N_{1a}Cr_2$ | 90.2(1) |
| $N_{1a}Cr_1N_{1a'}$ [c] | 180.0(-)[d] | $Cr_1N_{1a}C_{1a}$ | 121.2(2) |
| $N_{1b}Cr_1N_{1a'}$ [c] | 95.2(1) | $Cr_2N_{1a}C_{1a}$ | 118.0(2) |
| $N_{1b}Cr_1N_{1b'}$ [c] | 180.0(-)[d] | $Cr_1N_{1a}C_{4a}$ | 113.4(2) |
| | | $Cr_2N_{1a}C_{4a}$ | 110.6(2) |
| $N_{1a}Cr_2N_{1b}$ | 82.9(1) | $C_{1a}N_{1a}C_{4a}$ | 103.5(3) |
| $N_{1a}Cr_2N_{1c}$ | 96.5(1) | $Cr_1N_{1b}Cr_2$ | 92.6(1) |
| $N_{1b}Cr_2N_{1c}$ | 168.9(1) | $Cr_1N_{1b}C_{1b}$ | 117.9(2) |
| $N_{1a}Cr_2O_{1f}$ | 162.4(1) | $Cr_2N_{1b}C_{1b}$ | 107.6(3) |
| $N_{1b}Cr_2O_{1f}$ | 89.5(1) | $Cr_1N_{1b}C_{4b}$ | 120.6(3) |
| $N_{1c}Cr_2O_{1f}$ | 87.9(1) | $Cr_2N_{1b}C_{4b}$ | 113.0(3) |
| | | $C_{1b}N_{1b}C_{4b}$ | 104.4(3) |
| $N_{1c}Cr_3N_{1d}$ | 88.1(1) | $Cr_2N_{1c}Cr_3$ | 93.5(1) |
| $N_{1c}Cr_3N_{1e}$ | 176.5(1) | $Cr_2N_{1c}C_{1c}$ | 121.4(3) |
| $N_{1d}Cr_3N_{1e}$ | 93.5(1) | $Cr_3N_{1c}C_{1c}$ | 100.8(2) |
| $N_{1c}Cr_3O_{1g}$ | 88.8(1) | $Cr_2N_{1c}C_{4c}$ | 116.1(2) |
| $N_{1d}Cr_3O_{1g}$ | 170.4(1) | $Cr_3N_{1c}C_{4c}$ | 121.5(2) |
| $N_{1e}Cr_3O_{1g}$ | 89.1(1) | $C_{1c}N_{1c}C_{4c}$ | 104.2(3) |
| | | $Cr_3N_{1d}C_{1d}$ | 121.3(3) |
| $N_{1a}C_{1a}C_{2a}$ | 110.6(3) | $Cr_3N_{1d}C_{4d}$ | 127.8(3) |
| $C_{1a}C_{2a}C_{3a}$ | 107.5(4) | $C_{1d}N_{1d}C_{4d}$ | 106.4(4) |
| $C_{2a}C_{3a}C_{4a}$ | 106.9(4) | $Cr_3N_{1e}C_{1e}$ | 126.3(3) |
| $C_{3a}C_{4a}N_{1a}$ | 111.5(3) | $Cr_3N_{1e}C_{4e}$ | 128.3(3) |
| $N_{1b}C_{1b}C_{2b}$ | 111.2(4) | $C_{1e}N_{1e}C_{4e}$ | 105.3(3) |

Table V. (continued)

| Type [b] | Angle, deg. | Type [b] | Angle, deg. |
|---|---|---|---|
| $C_{1b}C_{2b}C_{3b}$ | 107.4(4) | | |
| $C_{2b}C_{3b}C_{4b}$ | 107.0(4) | $Cr_2O_{1f}C_{1f}$ | 131.5(2) |
| $C_{3b}C_{4b}N_{1b}$ | 110.1(4) | $Cr_2O_{1f}C_{4f}$ | 118.9(2) |
| $N_{1c}C_{1c}C_{2c}$ | 110.9(4) | $C_{1f}O_{1f}C_{4f}$ | 109.1(3) |
| $C_{1c}C_{2c}C_{3c}$ | 106.8(4) | $Cr_3O_{1g}C_{1g}$ | 131.9(3) |
| $C_{2c}C_{3c}C_{4c}$ | 107.2(4) | $Cr_3O_{1g}C_{4g}$ | 118.6(3) |
| $C_{3c}C_{4c}N_{1c}$ | 110.9(3) | $C_{1g}O_{1g}C_{4g}$ | 109.5(4) |
| $N_{1d}C_{1d}C_{2d}$ | 110.3(4) | | |
| $C_{1d}C_{2d}C_{3d}$ | 106.7(4) | $O_{1f}C_{1f}C_{2f}$ | 105.0(4) |
| $C_{2d}C_{3d}C_{4d}$ | 106.6(5) | $C_{1f}C_{2f}C_{3f}$ | 104.9(4) |
| $C_{3d}C_{4d}N_{1d}$ | 109.9(3) | $C_{2f}C_{3f}C_{4f}$ | 104.4(4) |
| $N_{1e}C_{1e}C_{2e}$ | 110.0(4) | $C_{3f}C_{4f}O_{1f}$ | 105.4(4) |
| $C_{1e}C_{2e}C_{3e}$ | 107.2(4) | $O_{1g}C_{1g}C_{2g}$ | 104.8(4) |
| $C_{2e}C_{3e}C_{4e}$ | 106.7(4) | $C_{1g}C_{2g}C_{3g}$ | 104.2(5) |
| $C_{3e}C_{4e}N_{1e}$ | 110.8(5) | $C_{2g}C_{3g}C_{4g}$ | 104.2(4) |
| | | $C_{3g}C_{4g}O_{1g}$ | 106.1(4) |

a The numbers in parentheses are the estimated standard deviations in the last significant digit.

b Atoms are labeled in agreement with Figure 1.

c Atoms labeled with a prime (') are related to nonprimed atoms by the symmetry operation -x, -y, -z where the fractional coordinates (x,y,z) are given in Table 1.

d This is a symmetry-required value and is therefore listed without an estimated standard deviation.

Example V

A single crystal x-ray structure was obtained for $Cr(NC_4H_4)_4$, a portion of Product III and shown in Figure 3. A single crystal x-ray structure was obtained for $[Na]_2[Cr(NC_4H_4)_4]\cdot 2(OC_4H_8)$, Product III and shown in Figure 4. The description of the single-crystal sample and mounting used for the data collection are as follows:

Color: Red-orange
Shape: Rectangular parallelepiped
Dimensions: 0.30 x 0.35 x 0.65 mm
Crystal Mount: Crystal was glued to the inside of a thin-walled glass capillary and sealed under $N_2$.
Crystal Orientation: Crystal was oriented with its longest edge nearly parallel to the phi axis of the diffractometer.
Width at Half-height from ω Scans: 0.86°

The space group and cell data are as follows:

Crystal System: Monoclinic
Space Group and Number: C2/c - $C^6_{2h}$ (No. 19)
Number of Computer-Centered Reflections Used in the Least-Squares Refinement of the Cell Dimensions:
Dimensions: 15 20>25° °C = 2θ±1°
Lattice Constants with esd's:

| | | |
|---|---|---|
| a = 9.522(2)Å | α = 90.00° | V = 2697(1)Å$^3$ |
| b = 15.118(2)Å | β = 98.99(1)° | Z = 4 |
| c = 18.967(3)Å | γ = 90.00° | λ = 6.71073Å |

Molecular Weight: 506.52 amu
Calculated Density: 1.248 g/cm$^3$
Linear Absorption Coefficient: $^{3a}$ 0.47mm$^{-1}$

Tables VI-X list the resultant parameters used to generate the molecular structures shown in Figure 3 and Figure 4.

Table VI.

| Atomic Coordinates for Nonhydrogen Atoms in Crystalline $\{Na\}_2\{Cr(NC_4H_4)_4\}$ - 2 $OC_4H_8$ [a] | | | | |
|---|---|---|---|---|
| Atom Type [b] | Fractional Coordinates | | | Equivalent Isotropic Thermal Parameter B, $Å^2 \times 10$ [c] |
| | $10^4x$ | $10^4y$ | $10^4z$ | |
| | Anion | | | |
| Cr | 8[d] | 2982(1) | 2588[d] | 58(1) |
| $N_1$ | 1981(4) | 2924(2) | 3183(2) | 56(1) |
| $N_2$ | 8[d] | 4343(3) | 2588[d] | 52(1) |
| $N_3$ | 8[d] | 1612(3) | 2588[d] | 78(2) |
| $C_{11}$ | 3241(5) | 2958(3) | 3888(3) | 65(2) |
| $C_{12}$ | 4224(6) | 2768(3) | 3587(3) | 73(2) |
| $C_{13}$ | 3513(7) | 2638(4) | 4146(3) | 82(2) |
| $C_{14}$ | 2894(7) | 2734(4) | 3884(3) | 76(2) |
| $C_{21}$ | 987(5) | 4884(3) | 2926(3) | 68(1) |
| $C_{22}$ | 582(4) | 5753(3) | 2766(3) | 69(2) |
| $C_{31}$ | 398(5) | 1881(3) | 1996(4) | 94(2) |
| $C_{32}$ | 236(7) | 213(3) | 2189(5) | 133(6) |
| | Cation | | | |
| Na | 2381(2) | 6879(1) | 1783(1) | 69(1) |
| | Solvent of Crystallization | | | |
| $O_1$ | 2865(4) | 5188(2) | 838(2) | 83(1) |
| $C_{41}$ | 2759(11) | 5174(5) | 239(4) | 143(4) |
| $C_{42}$ | 2884(11) | 4319(5) | -79(4) | 148(4) |
| $C_{43}$ | 1893(18) | 3786(5) | 264(5) | 142(4) |
| $C_{44}$ | 1699(9) | 4231(4) | 982(4) | 128(3) |

[a] The numbers in parentheses are the estimated standard deviations in the last significant digit.
[b] Atoms are labeled in agreement with Figures 1 and 2.
[c] This is one-third of the trace of the orthogonalized $B_{ij}$ tensor.
[d] This is a symmetry-required value and is therefore listed without an estimated standard deviation.

Table VII.

| Anisotropic Thermal Parameters of Nonhydrogen Atoms in Crystalline $\{Na\}_2\{Cr(NC_4H_4)_4\}$ - 2 $OC_4H_8$ [a,b] | | | | | | |
|---|---|---|---|---|---|---|
| Atom Type [c] | Anisotropic Thermal Parameter (Å x 10) | | | | | |
| | $B_{11}$ | $B_{22}$ | $B_{33}$ | $B_{12}$ | $B_{13}$ | $B_{23}$ |
| | Anion | | | | | |
| Cr | 64(1) | 34(1) | 55(1) | 8[d] | 15(1) | 8[d] |
| $N_1$ | 69(2) | 44(2) | 56(2) | 6(1) | 12(1) | 6(1) |
| $N_2$ | 64(3) | 39(2) | 56(3) | 8[d] | 16(2) | 8[d] |
| $N_3$ | 65(3) | 38(2) | 187(4) | 8[d] | 14(3) | 8[d] |
| $C_{11}$ | 78(3) | 58(2) | 78(3) | -6(2) | 18(2) | 2(2) |
| $C_{12}$ | 78(3) | 62(3) | 84(3) | 4(2) | 7(2) | -8(2) |
| $C_{13}$ | 183(4) | 79(3) | 58(3) | 22(3) | -8(3) | 8(2) |
| $C_{14}$ | 86(3) | 86(3) | 58(3) | 16(3) | 16(2) | 5(2) |
| $C_{21}$ | 66(2) | 45(2) | 78(3) | -2(2) | 15(2) | -6(2) |

[a] The numbers in parentheses are the estimated standard deviations in the last significant digit.
[b] The form of the anisotropic thermal parameter is given in reference 8 on page 6 of the structure report.
[c] Atoms are labeled in agreement with Figures 1 and 2.
[d] This is a symmetry-required value and is therefore listed without an estimated standard deviation.

Table VII. (continued)

| Anisotropic Thermal Parameters of Nonhydrogen Atoms in Crystalline $\{Na\}_2\{Cr(NC_4H_4)_4\}$ - 2 $OC_4H_8$ [a,b] | | | | | | |
|---|---|---|---|---|---|---|
| Atom Type [c] | Anisotropic Thermal Parameter (Å x 10) | | | | | |
| | $B_{11}$ | $B_{22}$ | $B_{33}$ | $B_{12}$ | $B_{13}$ | $B_{23}$ |
| | Anion | | | | | |
| $C_{22}$ | 68(3) | 38(2) | 185(4) | -7(2) | 27(2) | -9(2) |
| $C_{31}$ | 65(3) | 61(3) | 152(5) | 6(2) | 9(3) | -36(3) |
| $C_{32}$ | 71(5) | 46(2) | 266(15) | 6(3) | -28(6) | -44(4) |
| | Cation | | | | | |
| Na | 78(1) | 57(1) | 81(1) | -2(1) | 15(1) | -15(1) |
| | Solvent of Crystallization | | | | | |
| $O_1$ | 188(2) | 65(2) | 82(2) | -18(2) | 38(2) | -16(2) |
| $C_{41}$ | 222(8) | 112(5) | 116(5) | -46(5) | 92(6) | -22(4) |
| $C_{42}$ | 192(8) | 168(8) | 187(5) | 12(6) | 78(5) | -32(5) |
| $C_{43}$ | 147(6) | 189(6) | 177(8) | -27(5) | 48(6) | -69(6) |
| $C_{44}$ | 177(6) | 77(4) | 124(5) | -21(4) | 76(5) | -14(3) |

[a] The numbers in parentheses are the estimated standard deviations in the last significant digit.

[b] The form of the anisotropic thermal parameter is given in reference 8 on page 6 of the structure report.

[c] Atoms are labeled in agreement with Figures 1 and 2.

Table VIII.

| Atomic Coordinates for Hydrogen Atoms in Crystalline $\{Na\}_2\{Cr(N_4H_4)_4\}$ - 2 $OC_4H_8$ [a] | | | |
|---|---|---|---|
| Atom Type [b] | Fractional Coordinates | | |
| | $10^4x$ | $10^4y$ | $10^4z$ |
| | Anion | | |
| $H_{11}$ | 3456 | 3881 | 2541 |
| $H_{12}$ | 5235 | 2748 | 3688 |
| $H_{13}$ | 3922 | 2488 | 4628 |
| $H_{14}$ | 1341 | 2679 | 4164 |
| $H_{21}$ | 1665 | 4687 | 3285 |
| $H_{22}$ | 1871 | 6262 | 2985 |
| $H_{31}$ | 786 | 1274 | 1565 |
| $H_{32}$ | 483 | -381 | 1937 |
| | Solvent of Crystallization | | |
| $H_{41a}$ | 2258 | 5576 | -188 |
| $H_{41b}$ | 3718 | 5388 | 385 |
| $H_{42a}$ | 3756 | 4891 | -1 |
| $H_{42b}$ | 2464 | 4348 | -583 |
| $H_{43a}$ | 995 | 3787 | -39 |
| $H_{43b}$ | 2326 | 3228 | 377 |
| $H_{44a}$ | 2295 | 3973 | 1384 |
| $H_{44b}$ | 723 | 4191 | 969 |

[a] Hydrogen atoms were included in the structure factor calculations as idealized atoms (assuming $sp^2$- or $sp^3$-hybridization of the carbon atoms and a C-H bond length of 0.96Å) "riding" on their respective carbon atoms. The isotropic thermal parameter of each hydrogen atom was fixed at 1.2 times the equivalent isotropic thermal parameter of the carbon atom to which it is covalently bonded.

[b] Hydrogen atoms are labeled with the same numerical subscripts as the carbon atoms to which they are covalently bonded with an additional literal subscript (a or b) where necessary to distinguish between hydrogens bonded to the same carbon.

Table IX.

| Anion Bond Lengths and Bond Angles Involving Nonhydrogen Atoms in Crystalline $\{Na\}_2\{Cr(NC_4H_4)_4\}$ - 2 $OC_4H_8$ [a] | | | |
|---|---|---|---|
| Type [b] | Length, Å | Type [b] | Length, Å |
| $Cr-N_1$ | 2.057(3) | $C_{11}-C_{12}$ | 1.355(7) |
| $Cr-N_2$ | 2.056(4) | $C_{12}-C_{13}$ | 1.361(9) |
| $Cr-N_3$ | 2.072(5) | $C_{13}-C_{14}$ | 1.374(9) |
| | | $C_{21}-C_{22}$ | 1.372(6) |
| $N_1-C_{11}$ | 1.369(7) | $C_{22}-C_{22'}$ [c] | 1.379(9) |
| $N_1-C_{14}$ | 1.344(6) | $C_{31}-C_{32}$ | 1.376(7) |
| $N_2-C_{21}$ | 1.360(5) | $C_{32}-C_{32'}$ [c] | 1.327(18) |
| $N_3-C_{31}$ | 1.344(7) | | |
| Type [b] | Angle, deg. | Type [b] | Angle, deg. |
| $N_1CrN_2$ | 92.5(1) | $N_1C_{11}C_{12}$ | 110.5(5) |
| $N_1CrN_3$ | 87.5(1) | $C_{11}C_{12}C_{13}$ | 107.3(5) |
| $N_1CrN_{1'}$ [c] | 175.1(2) | $C_{12}C_{13}C_{14}$ | 106.4(5) |
| $N_2CrN_3$ | 180.0(-)[d] | $N_1C_{14}C_{13}$ | 110.9(5) |
| | | $N_2C_{21}C_{22}$ | 110.2(4) |
| $CrN_1C_{11}$ | 127.5(3) | $C_{21}C_{22}C_{22'}$ [c] | 106.8(3) |
| $CrN_1C_{14}$ | 127.1(4) | $N_3C_{31}C_{32}$ | 109.1(6) |
| $C_{11}N_1C_{14}$ | 104.9(4) | $C_{31}C_{32}C_{32'}$ [c] | 107.5(5) |
| $CrN_2C_{21}$ | 127.0(2) | | |
| $C_{21}N_2C_{21'}$ [c] | 106.0(5) | | |
| $CrN_3C_{31}$ | 126.7(3) | | |
| $C_{31}N_3C_{31'}$ [c] | 106.7(6) | | |

[a] The numbers in parentheses are the estimated standard deviations in the last significant digit.
[b] Atoms are labeled in agreement with Figure 1.
[c] Atom labeled with a prime(') are related to nonprimed atoms by the symmetry operation -x, y, ½-z.

Table X.

| Bond Lengths and Angles Involving the Nonhydrogen Atoms of the Cation and Solvent of Crystallization in $\{Na\}_2\{Cr(NC_4H_4)_4\}$ - 2 $OC_4H_8$ [a] | | | |
|---|---|---|---|
| Type [b] | Length, Å | Type [b] | Length, Å |
| $Na-O_1$ | 2.313(4) | $O_1-C_{41}$ | 1.390(10) |
| | | $O_1-C_{44}$ | 1.382(7) |
| $Na\cdots N_{1''}$ [c] | 2.888(4) | | |
| $Na\cdots N_{3''}$ [c] | 2.830(4) | $C_{41}-C_{42}$ | 1.43(1) |
| | | $C_{42}-C_{43}$ | 1.42(1) |
| | | $C_{43}-C_{44}$ | 1.42(1) |
| Type [b] | Angle, deg. | Type [b] | Angle, deg. |
| $O_1NaN_{1''}$ [c] | 128.6(3) | $C_{41}O_1C_{44}$ | 107.9(5) |
| $O_1NaN_{3''}$ [c] | 121.8(3) | | |
| $N_{1''}NaN_{3''}$ [c] | 59.9(3) | $O_1C_{41}C_{42}$ | 109.0(7) |
| | | $C_{41}C_{42}C_{43}$ | 105.0(8) |
| $NaO_1C_{41}$ | 125.7(4) | $C_{42}C_{43}C_{44}$ | 107.0(7) |
| $NaO_1C_{44}$ | 121.8(4) | $O_1C_{44}C_{43}$ | 107.6(7) |

[a] The numbers in parentheses are the estimated standard deviations in the last significant digit.
[b] Atoms are labeled in agreement with Figure 2.
[c] Atoms labeled with a double prime(") are related to nonprimed atoms by the symmetry operation ½-x, ½+y, ½-z.

Example VI

Single crystal x-ray structures were obtained for $[Cr(NC_4H_4)_5(OC_4H_8)]$, shown in Figure 5, and $(Cr(NC_4H_4)_5(OC_4H_8))[Na]_2 \cdot 4(OC_4H_8)$, Product IV, and shown in Figure 6. The description of the single-crystal sample and mounting used for data collection are as follows:

Color: Purple
Shape: Rectangular parallelepiped
Dimensions: 0.50 x 0.55 x 0.63 mm
Crystal Mount: Crystal was glued to the inside of a thin-walled glass capillary and sealed under $N_2$.
Crystal Orientation: Crystal was oriented with its longest edge nearly parallel to the phi axis of the diffractometer.
Width at Half-height from ω Scans: 0.42°

The space group and cell data are as follows:

Crystal System: Monoclinic
Space Group and Number: $P2_1$-$C_2$(No. 4)
Number of Computer-Centered Reflections Used in the Least-Squares Refinement of the Cell Dimensions:
15 $2\theta>20°$ °C=20±1°
Lattice Constants with esd's:

| | | |
|---|---|---|
| a = 10.042(2)Å | α = 90.00° | V = 2162(1)$Å^3$ |
| b = 17.242(4)Å | β = 106.54(2)° | Z = 2 |
| c = 13.025(3)Å | γ = 90.00° | λ = 0.71073Å |

Molecular Weight = 788.93 amu
Calculated Density: 1.212 $g/cm^3$
Linear Absorption coefficient:[3a] 0.32 $mm^{-1}$

Tables XI - XV list the resultant parameters used to generate the molecular structures shown in Figure 5 and Figure 6.

Table XI. Atomic Coordinates for Nonhydrogen Atoms in Crystalline $[Cr(NC_4H_4)_5(OC_4H_8)][Na]_2 \cdot 4\ OC_4H_8$ [a]

| Atom Type [b] | Fractional Coordinates $10^4x$ | $10^4y$ | $10^4z$ | Equivalent Isotropic Thermal Parameter, B, $Å^2 \times 10$ [c] |
|---|---|---|---|---|
| | | Anion | | |
| Cr | 198(1) | 1477 | 2531(1) | 32(1) |
| $N_{1a}$ | 1694(5) | 2026(3) | 2028(4) | 40(2) |
| $C_{1a}$ | 1749(7) | 2782(4) | 1742(6) | 48(2) |
| $C_{2a}$ | 2929(8) | 2926(5) | 1420(7) | 66(3) |
| $C_{3a}$ | 3661(7) | 2236(5) | 1554(6) | 62(3) |
| $C_{4a}$ | 2899(6) | 1695(5) | 1913(5) | 52(2) |
| $N_{1b}$ | 1651(5) | 1087(3) | 3885(4) | 40(2) |
| $C_{1b}$ | 1463(8) | 560(4) | 4575(5) | 48(2) |
| $C_{2b}$ | 2572(9) | 518(6) | 5423(8) | 82(4) |
| $C_{3b}$ | 3554(8) | 1064(6) | 5275(6) | 70(3) |
| $C_{4b}$ | 2952(6) | 1382(5) | 4340(5) | 48(2) |
| $N_{1c}$ | -1326(5) | 1888(3) | 1250(4) | 38(2) |
| $C_{1c}$ | -1200(8) | 2172(4) | 266(6) | 51(2) |
| $C_{2c}$ | -2458(8) | 2270(5) | -476(6) | 58(3) |
| $C_{3c}$ | -3435(8) | 2038(6) | 56(7) | 75(3) |
| $C_{4c}$ | -2710(7) | 1826(5) | 1091(6) | 56(3) |
| $N_{1d}$ | -32(5) | 2455(4) | 3445(5) | 43(2) |
| $C_{1d}$ | 504(7) | 2562(5) | 4505(6) | 49(2) |
| $C_{2d}$ | 107(9) | 3278(5) | 4774(8) | 72(3) |
| $C_{3d}$ | -698(8) | 3629(5) | 3832(6) | 59(3) |
| $C_{4d}$ | -769(7) | 3108(4) | 3055(6) | 52(2) |
| $N_{1e}$ | 315(5) | 505(4) | 1690(4) | 40(2) |
| $C_{1e}$ | -574(8) | 277(5) | 704(6) | 55(3) |
| $C_{2e}$ | -197(10) | -432(5) | 403(7) | 67(3) |
| $C_{3e}$ | 990(10) | -662(6) | 1256(8) | 79(4) |
| $C_{4e}$ | 1265(8) | -92(4) | 2016(7) | 51(3) |

**Table XI. (continued)**

| Atom Type [b] | Fractional Coordinates $10^4x$ | $10^4y$ | $10^4z$ | Equivalent Isotropic Thermal Parameter, B, $Å^2$ x 10 [c] |
|---|---|---|---|---|
| $O_{1f}$ | -1356(4) | 926(3) | 3083(4) | 43(1) |
| $C_{1f}$ | -2047(7) | 1244(5) | 3800(6) | 57(3) |
| $C_{2f}$ | -3263(10) | 713(6) | 3706(9) | 98(5) |
| $C_{3f}$ | -2833(11) | -21(6) | 3402(8) | 93(4) |
| $C_{4f}$ | -1903(8) | 171(5) | 2724(7) | 64(3) |
| | | Cation 1 | | |
| $Na_1$ | 2254(3) | 3336(2) | 3737(3) | 75(1) |
| | | Cation 2 | | |
| $Na_2$ | 1430(3) | 974(2) | 126(2) | 62(1) |
| | | Solvent Molecules of Crystallization | | |
| $O_{1g}$ | 4576(6) | 3329(4) | 4706(5) | 83(2) |
| $C_{1g}$ | 5748(9) | 3100(10) | 4433(9) | 125(6) |
| $C_{2g}$ | 6723(12) | 2831(11) | 5281(9) | 145(7) |
| $C_{3g}$ | 6503(15) | 3272(11) | 6146(11) | 204(8) |
| $C_{4g}$ | 5037(14) | 3498(11) | 5737(10) | 170(8) |
| $O_{1h}$ | 2342(7) | 4602(4) | 3279(6) | 97(3) |
| $C_{1h}$ | 1316(11) | 5151(7) | 2894(10) | 112(5) |
| $C_{2h}$ | 2017(16) | 5830(9) | 2541(11) | 153(7) |
| $C_{3h}$ | 3180(12) | 5561(10) | 2425(10) | 131(6) |
| $C_{4h}$ | 3551(13) | 4848(7) | 3070(11) | 115(6) |
| $O_{1i}$ | 1391(7) | 1752(4) | -1377(4) | 80(2) |
| $C_{1i}$ | 2235(19) | 1594(11) | -1998(13) | 160(8) |
| $C_{2i}$ | 2716(17) | 2287(14) | -2337(15) | 165(10) |
| $C_{3i}$ | 1991(28) | 2906(1[illegible] | -1934(14) | 204(12) |
| $C_{4i}$ | 1010(16) | 2533(7) | -1523(9) | 128(6) |

Table XI. (continued)

| Atom Type [b] | Fractional Coordinates $10^4x$ | $10^4y$ | $10^4z$ | Equivalent Isotropic Thermal Parameter, B, $Å^2$ x 10 [c] |
|---|---|---|---|---|
| $O_{1j}$ | 3037(5) | 155(4) | -264(5) | 72(2) |
| $C_{1j}$ | 4389(10) | 48(7) | 427(9) | 113(5) |
| $C_{2j}$ | 4998(16) | -571(10) | -23(16) | 174(8) |
| $C_{3j}$ | 4001(11) | -840(8) | -1006(10) | 127(6) |
| $C_{4j}$ | 2728(11) | -493(7) | -974(8) | 92(4) |

[a] The numbers in parentheses are the estimated standard deviations in the last significant digit.
[b] Atoms are labeled in agreement with Figures 1 and 2.
[c] This is one-third of the trace of the orthogonalized $B_{ij}$ tensor.

Table XII. Anisotropic Thermal Parameters Nonhydrogen Atoms in crystalline $[Cr(NC_4H_4)_5(OC_4H_8)][Cr]_2$ - 4 $OC_4H_8$ [a,b]

| Atom Type [c] | Anisotropic Thermal Parameter ($Å^2$ x 10) | | | | | |
|---|---|---|---|---|---|---|
| | $B_{11}$ | $B_{22}$ | $B_{33}$ | $B_{12}$ | $B_{13}$ | $B_{23}$ |
| | | | Anion | | | |
| Cr | 29(1) | 31(1) | 38(1) | 1(1) | 12(1) | 1(1) |
| $N_{1a}$ | 33(2) | 44(3) | 44(3) | -1(2) | 11(2) | 5(2) |
| $C_{1a}$ | 48(4) | 37(3) | 59(4) | -0(3) | 15(3) | 3(3) |
| $C_{2a}$ | 55(4) | 61(5) | 90(5) | -19(4) | 34(4) | 13(4) |
| $C_{3a}$ | 37(3) | 82(6) | 76(5) | -9(3) | 33(3) | 2(4) |
| $C_{4a}$ | 40(3) | 64(5) | 52(4) | 4(3) | 16(3) | -5(3) |
| $N_{1b}$ | 36(2) | 44(3) | 36(3) | 7(2) | 5(2) | 12(2) |
| $C_{1b}$ | 52(4) | 51(4) | 40(3) | -1(3) | 9(3) | 10(3) |
| $C_{2b}$ | 73(5) | 85(6) | 83(6) | 2(5) | 13(4) | 44(5) |
| $C_{3b}$ | 51(4) | 88(6) | 54(4) | 0(4) | -13(3) | 12(4) |
| $C_{4b}$ | 41(3) | 55(4) | 45(3) | 0(3) | 5(2) | 4(4) |
| $N_{1c}$ | 33(2) | 41(3) | 39(3) | 4(2) | 9(2) | 1(2) |
| $C_{1c}$ | 52(4) | 51(4) | 51(4) | 6(3) | 16(3) | 5(3) |
| $C_{2c}$ | 64(5) | 62(5) | 37(4) | -1(4) | -4(3) | 4(4) |
| $C_{3c}$ | 32(3) | 92(6) | 89(6) | 4(4) | -3(4) | 29(5) |
| $C_{4c}$ | 42(3) | 78(5) | 48(4) | -1(3) | 9(3) | 14(4) |
| $N_{1d}$ | 31(2) | 44(3) | 56(3) | 4(2) | 13(2) | -1(3) |
| $C_{1d}$ | 44(3) | 60(5) | 39(4) | -5(3) | 8(3) | -11(3) |
| $C_{2d}$ | 63(4) | 70(6) | 84(6) | -11(4) | 20(4) | -47(5) |
| $C_{3d}$ | 69(4) | 43(4) | 73(5) | 9(3) | 32(4) | -14(4) |
| $C_{4d}$ | 42(3) | 53(4) | 63(4) | 8(3) | 17(3) | 3(4) |
| $N_{1e}$ | 47(3) | 36(3) | 39(3) | -3(2) | 17(2) | -7(2) |
| $C_{1e}$ | 59(4) | 49(4) | 53(4) | -15(3) | 11(3) | -1(4) |
| $C_{2e}$ | 92(5) | 48(4) | 69(5) | -20(4) | 36(4) | -26(4) |
| $C_{3e}$ | 91(6) | 45(5) | 106(7) | 4(4) | 37(5) | -13(5) |
| $C_{4e}$ | 62(4) | 23(3) | 69(5) | 7(3) | 20(4) | -7(3) |

Table XII. (continued)

| Atom Type [c] | Anisotropic Thermal Parameter ($Å^2$ x 10) | | | | | |
|---|---|---|---|---|---|---|
| | $B_{11}$ | $B_{22}$ | $B_{33}$ | $B_{12}$ | $B_{13}$ | $B_{23}$ |
| $O_{1f}$ | 40(2) | 42(2) | 51(2) | -4(2) | 20(2) | 2(2) |
| $C_{1f}$ | 61(4) | 64(5) | 60(4) | -2(3) | 39(3) | 4(4) |
| $C_{2f}$ | 81(6) | 95(7) | 144(8) | -24(5) | 74(6) | 1(6) |
| $C_{3f}$ | 109(7) | 80(6) | 117(7) | -26(5) | 75(6) | -3(6) |
| $C_{4f}$ | 61(4) | 53(4) | 85(5) | -27(4) | 30(4) | -16(4) |
| | | | Cation 1 | | | |
| $Na_1$ | 57(2) | 71(2) | 95(2) | -13(1) | 21(2) | -2(2) |
| | | | Cation 2 | | | |
| $Na_2$ | 68(2) | 69(2) | 56(2) | -2(1) | 30(1) | -3(2) |
| | | | Solvent Molecules of Crystallization | | | |
| $O_{1g}$ | 58(3) | 95(4) | 92(4) | -8(3) | 15(3) | -2(4) |
| $C_{1g}$ | 54(5) | 215(14) | 108(8) | 0(7) | 29(5) | -7(9) |
| $C_{2g}$ | 96(7) | 226(15) | 121(9) | 52(9) | 43(7) | 51(10) |
| $C_{3g}$ | 129(10) | 277(19) | 148(11) | 52(12) | -56(9) | -134(13) |
| $C_{4g}$ | 134(10) | 250(18) | 128(10) | 44(11) | 39(9) | -89(11) |
| $O_{1h}$ | 71(4) | 68(4) | 152(6) | -8(3) | 32(4) | -3(4) |
| $C_{1h}$ | 92(7) | 95(8) | 144(9) | -2(6) | 28(7) | -3(7) |
| $C_{2h}$ | 212(14) | 108(9) | 140(10) | 36(10) | 50(10) | 66(9) |
| $C_{3h}$ | 99(8) | 175(14) | 101(8) | -6(9) | -2(6) | 32(9) |
| $C_{4h}$ | 99(8) | 79(7) | 168(11) | -13(6) | 38(8) | 29(8) |
| $O_{1i}$ | 98(4) | 82(4) | 73(3) | 8(3) | 47(3) | 13(3) |
| $C_{1i}$ | 230(15) | 128(11) | 168(12) | 8(11) | 131(12) | 74(10) |
| $C_{2i}$ | 112(10) | 222(21) | 156(15) | 1(12) | 28(10) | 23(16) |
| $C_{3i}$ | 370(26) | 124(12) | 135(12) | -93(15) | 99(15) | 34(10) |
| $C_{4i}$ | 223(13) | 81(7) | 106(8) | 32(8) | 91(9) | 31(6) |

Table XII. (continued)

| Atom Type [c] | Anisotropic Thermal Parameter (Å$^2$ x 10) $B_{11}$ | $B_{22}$ | $B_{33}$ | $B_{12}$ | $B_{13}$ | $B_{23}$ |
|---|---|---|---|---|---|---|
| $O_{1j}$ | 59(3) | 64(3) | 94(4) | 5(3) | 22(3) | -21(3) |
| $C_{1j}$ | 88(7) | 101(8) | 133(9) | 19(6) | 2(6) | -58(7) |
| $C_{2j}$ | 94(8) | 190(14) | 205(13) | 73(10) | -11(9) | -90(13) |
| $C_{3j}$ | 83(7) | 130(10) | 160(10) | 16(7) | 20(7) | -86(9) |
| $C_{4j}$ | 82(6) | 104(8) | 92(7) | -7(6) | 29(5) | -41(6) |

[a] The numbers in parentheses are the estimated standard deviations in the last significant digit.
[b] The form of the anisotropic thermal parameter is given in reference 8 on page 6 of the structure report.
[c] Atoms are labeled in agreement with Figures 1 and 2.

Table XIII. Atomic Coordinates for Hydrogen Atoms in Crystalline $[Cr(NC_4H_4)_5(OC_4H_8)][Na]_2$ - 4 $OC_4H_8$ [a]

| Atom Type [b] | Fractional Coordinates $10^4x$ | $10^4y$ | $10^4z$ |
|---|---|---|---|
| | Anion | | |
| $H_{1a}$ | 1061 | 3165 | 1756 |
| $H_{2a}$ | 3182 | 3406 | 1151 |
| $H_{3a}$ | 4547 | 2153 | 1428 |
| $H_{4a}$ | 3162 | 1162 | 2059 |
| $H_{1b}$ | 637 | 254 | 4479 |
| $H_{2b}$ | 2692 | 174 | 6022 |
| $H_{3b}$ | 4453 | 1179 | 5753 |
| $H_{4b}$ | 3373 | 1775 | 4016 |
| $H_{1c}$ | -326 | 2281 | 132 |
| $H_{2c}$ | -2637 | 2453 | -1199 |
| $H_{3c}$ | -4426 | 2031 | -243 |
| $H_{4c}$ | -3137 | 1655 | 1623 |
| $H_{1d}$ | 1070 | 2197 | 4997 |
| $H_{2d}$ | 349 | 3499 | 5480 |
| $H_{3d}$ | -1115 | 4135 | 3762 |
| $H_{4d}$ | -1278 | 3184 | 2317 |
| $H_{1e}$ | -1346 | 578 | 293 |
| $H_{2e}$ | -630 | -712 | -243 |
| $H_{3e}$ | 1503 | -1135 | 1285 |
| $H_{4e}$ | 1999 | -107 | 2676 |
| $H_{1fa}$ | -1447 | 1250 | 4520 |
| $H_{1fb}$ | -2359 | 1762 | 3588 |
| $H_{2fa}$ | -4069 | 899 | 3170 |
| $H_{2fb}$ | -3468 | 674 | 4380 |
| $H_{3fa}$ | -2341 | -312 | 4022 |
| $H_{3fb}$ | -3620 | -314 | 2996 |

Table XIII. (continued)

| Atom Type [b] | Fractional Coordinates $10^4x$ | $10^4y$ | $10^4z$ |
|---|---|---|---|
| $H_{4fa}$ | -2417 | 184 | 1980 |
| $H_{4fb}$ | -1165 | -201 | 2831 |
| | Solvent of Crystallization | | |
| $H_{1ga}$ | 6103 | 3536 | 4135 |
| $H_{1gb}$ | 5503 | 2694 | 3909 |
| $H_{2ga}$ | 6629 | 2283 | 5371 |
| $H_{2gb}$ | 7629 | 2940 | 5209 |
| $H_{3ga}$ | 6644 | 2947 | 6766 |
| $H_{3gb}$ | 7102 | 3717 | 6322 |
| $H_{4ga}$ | 4960 | 4045 | 5839 |
| $H_{4gb}$ | 4493 | 3223 | 6118 |
| $H_{1ha}$ | 596 | 4950 | 2301 |
| $H_{1hb}$ | 921 | 5310 | 3451 |
| $H_{2ha}$ | 2205 | 6231 | 3073 |
| $H_{2hb}$ | 1449 | 6034 | 1874 |
| $H_{3ha}$ | 3066 | 5447 | 1684 |
| $H_{3hb}$ | 3908 | 5936 | 2669 |
| $H_{4ha}$ | 4260 | 4953 | 3725 |
| $H_{4hb}$ | 3874 | 4459 | 2671 |
| $H_{1ia}$ | 3007 | 1289 | -1594 |
| $H_{1ib}$ | 1721 | 1306 | -2615 |
| $H_{2ia}$ | 3703 | 2328 | -2031 |
| $H_{2ib}$ | 2496 | 2303 | -3103 |
| $H_{3ia}$ | 1541 | 3249 | -2509 |
| $H_{3ib}$ | 2638 | 3195 | -1381 |
| $H_{4ia}$ | 101 | 2580 | -2020 |
| $H_{4ib}$ | 1010 | 2761 | -851 |

Table XIII. (continued)

| Atom Type [b] | Fractional Coordinates $10^4x$ | $10^4y$ | $10^4z$ |
|---|---|---|---|
| $H_{1ja}$ | 4929 | 513 | 470 |
| $H_{1jb}$ | 4341 | -91 | 1129 |
| $H_{2ja}$ | 5823 | -388 | -178 |
| $H_{2jb}$ | 5232 | -992 | 479 |
| $H_{3ja}$ | 3930 | -1396 | -1018 |
| $H_{3jb}$ | 4261 | -668 | -1623 |
| $H_{4ja}$ | 2185 | -862 | -715 |
| $H_{4jb}$ | 2215 | -324 | -1678 |

[a] Hydrogen atoms were included in the structure factor calculations as idealized atoms (assuming $sp^2$- or $sp^3$-hybridization of the carbon atoms and a C-H bond length of 0.96Å ) "riding" on their respective carbon atoms. The isotropic thermal parameter of each hydrogen atom was fixed at 1.2 times the equivalent isotropic thermal parameter of the carbon atom to which it is covalently bonded.

[b] Hydrogen atoms are labeled with the same numerical and literal subscripts as their carbon atoms with an additional literal subscript (a, or b ) where necessary to distinguish between hydrogen atoms bonded to the same carbon.

Table XIV. Bond Lengths Involving Nonhydrogen Atoms in Crystalline $[Cr(NC_4H_4)_5(OC_4H_8)][Na]_2 \cdot 4\ OC_4H_8$ [a]

| Type [b] | Length, Å | Type [b] | Length, Å |
|---|---|---|---|
| $Cr-N_{1a}$ | 2.035(6) | $Na_1-O_{1g}$ | 2.314(6) |
| $Cr-N_{1b}$ | 2.056(5) | $Na_1-O_{1h}$ | 2.271(8) |
| $Cr-N_{1c}$ | 2.044(5) | | |
| $Cr-N_{1d}$ | 2.114(6) | $Na_2-O_{1i}$ | 2.365(7) |
| $Cr-N_{1e}$ | 2.024(6) | $Na_2-O_{1j}$ | 2.307(7) |
| $Cr-O_{1f}$ | 2.120(5) | $C_{1g}-C_{2g}$ | 1.33(2) |
| | | $C_{2g}-C_{3g}$ | 1.43(2) |
| $N_{1a}-C_{1a}$ | 1.36(1) | $C_{3g}-C_{4g}$ | 1.47(2) |
| $N_{1a}-C_{4a}$ | 1.38(1) | $C_{1h}-C_{2h}$ | 1.51(2) |
| $N_{1b}-C_{1b}$ | 1.33(1) | $C_{2h}-C_{3h}$ | 1.30(2) |
| $N_{1b}-C_{4b}$ | 1.37(1) | $C_{3h}-C_{4h}$ | 1.48(2) |
| $N_{1c}-C_{1c}$ | 1.41(1) | $C_{1i}-C_{2i}$ | 1.41(3) |
| $N_{1c}-C_{4c}$ | 1.35(1) | $C_{2i}-C_{3i}$ | 1.47(3) |
| $N_{1d}-C_{1d}$ | 1.34(1) | $C_{3i}-C_{4i}$ | 1.40(3) |
| $N_{1d}-C_{4d}$ | 1.36(1) | $C_{1j}-C_{2j}$ | 1.44(2) |
| $N_{1e}-C_{1e}$ | 1.40(1) | $C_{2j}-C_{3j}$ | 1.46(2) |
| $N_{1e}-C_{4e}$ | 1.39(1) | $C_{3j}-C_{4j}$ | 1.42(2) |
| $O_{1f}-C_{1f}$ | 1.42(1) | $O_{1g}-C_{1g}$ | 1.38(1) |
| $O_{1f}-C_{4f}$ | 1.44(1) | $O_{1g}-C_{4g}$ | 1.32(1) |
| | | $O_{1h}-C_{1h}$ | 1.38(1) |

Table XIV. (continued)

| Type [b] | Length, Å | Type [b] | Length, Å |
|---|---|---|---|
| $C_{1a}$-$C_{2a}$ | 1.39(1) | $O_{1h}$-$C_{4h}$ | 1.39(2) |
| $C_{2a}$-$C_{3a}$ | 1.38(1) | $O_{1i}$-$C_{1i}$ | 1.36(2) |
| $C_{3a}$-$C_{4a}$ | 1.37(1) | $O_{1i}$-$C_{4i}$ | 1.40(1) |
| $C_{1b}$-$C_{2b}$ | 1.33(1) | $O_{1j}$-$C_{1j}$ | 1.41(1) |
| $C_{2b}$-$C_{3b}$ | 1.42(1) | $O_{1j}$-$C_{4j}$ | 1.43(1) |
| $C_{3b}$-$C_{4b}$ | 1.31(1) | | |
| $C_{1c}$-$C_{2c}$ | 1.37(1) | $Na_1$-$C_{1a}$ | 2.678(8) |
| $C_{2c}$-$C_{3c}$ | 1.41(1) | $Na_1$-$N_{1d}$ | 2.688(7) |
| $C_{3c}$-$C_{4c}$ | 1.39(1) | $Na_1$-$C_{1d}$ | 2.621(9) |
| $C_{1d}$-$C_{2d}$ | 1.37(1) | | |
| $C_{2d}$-$C_{3d}$ | 1.40(1) | | |
| $C_{3d}$-$C_{4d}$ | 1.34(1) | $Na_2$-$C_{4a}$ | 2.681(7) |
| $C_{1e}$-$C_{2e}$ | 1.37(1) | $Na_2$-$C_{1e}$ | 2.630(9) |
| $C_{2e}$-$C_{3e}$ | 1.43(1) | | |
| $C_{3e}$-$C_{4e}$ | 1.37(1) | | |
| $C_{1f}$-$C_{2f}$ | 1.50(1) | | |
| $C_{2f}$-$C_{3f}$ | 1.43(2) | | |
| $C_{3f}$-$C_{4f}$ | 1.49(2) | | |

[a] The numbers in parentheses are the estimated standard deviations in the last significant digit.

[b] Atoms are labeled in agreement with Figure 1.

Table XV. Bond Angles Involving Nonhydrogen Atoms in Crystalline $[Cr(NC_4H_4)_5(OC_4H_8)][Na]_2$-4 $OC_4H_8$ [a]

| Type [b] | Angle, deg | Type [b] | Angle, deg |
|---|---|---|---|
| $N_{1a}CrN_{1b}$ | 91.2(2) | $O_{1g}Na_1O_{1h}$ | 92.3(3) |
| $N_{1a}CrN_{1c}$ | 91.4(2) | $O_{1g}Na_1C_{1a}$ | 114.3(3) |
| $N_{1a}CrN_{1d}$ | 91.1(2) | $O_{1g}Na_1N_{1d}$ | 139.6(3) |
| $N_{1a}CrN_{1e}$ | 92.8(2) | $O_{1g}Na_1C_{1d}$ | 118.0(3) |
| $N_{1a}CrO_{1f}$ | 178.7(2) | $O_{1h}Na_1C_{1a}$ | 95.6(3) |
| $N_{1b}CrN_{1c}$ | 176.2(2) | $O_{1h}Na_1N_{1d}$ | 127.0(2) |
| $N_{1b}CrN_{1d}$ | 86.7(2) | $O_{1h}Na_1C_{1d}$ | 132.1(3) |
| $N_{1b}CrN_{1e}$ | 93.3(2) | $C_{1a}Na_1N_{1d}$ | 75.1(2) |
| $N_{1b}CrO_{1f}$ | 88.5(2) | $C_{1a}Na_1C_{1d}$ | 103.1(3) |
| $N_{1c}CrN_{1d}$ | 90.4(2) | $N_{1d}Na_1C_{1d}$ | 29.3(2) |
| $N_{1c}CrN_{1e}$ | 89.4(2) | | |
| $N_{1c}CrO_{1f}$ | 88.8(2) | $O_{1i}Na_2O_{1j}$ | 90.7(3) |
| $N_{1d}CrN_{1e}$ | 176.1(2) | $O_{1i}Na_2C_{4a}$ | 109.3(3) |
| $N_{1d}CrO_{1f}$ | 87.6(2) | $O_{1i}Na_2C_{1e}$ | 131.5(2) |
| $N_{1e}CrO_{1f}$ | 88.5(2) | $O_{1j}Na_2C_{4a}$ | 103.2(2) |
| | | $O_{1j}Na_2C_{1e}$ | 115.1(3) |
| $CrN_{1a}C_{1a}$ | 128.7(5) | $C_{4a}Na_2C_{1e}$ | 103.9(3) |
| $CrN_{1a}C_{4a}$ | 126.3(5) | | |
| $CrN_{1b}C_{1b}$ | 127.0(4) | $Na_1O_{1g}C_{1g}$ | 131.4(6) |
| $CrN_{1b}C_{4b}$ | 127.3(5) | $Na_1O_{1g}C_{4g}$ | 124.0(8) |
| $CrN_{1c}C_{1c}$ | 128.5(5) | $Na_1O_{1h}C_{1h}$ | 132.2(7) |

Table XV. (continued)

| Type [b] | Angle,deg | Type [b] | Angle,deg |
|---|---|---|---|
| $CrN_{1c}C_{4c}$ | 126.7(5) | $Na_1O_{1h}C_{4h}$ | 116.6(6) |
| $CrN_{1d}C_{1d}$ | 127.7(5) | $Na_2O_{1i}C_{1i}$ | 120.9(8) |
| $CrN_{1d}C_{4d}$ | 125.7(5) | $Na_2O_{1i}C_{4i}$ | 126.8(7) |
| $CrN_{1e}C_{1e}$ | 127.7(5) | $Na_2O_{1j}C_{1j}$ | 123.1(6) |
| $CrN_{1e}C_{4e}$ | 126.2(4) | $Na_2O_{1j}C_{4j}$ | 125.8(5) |
| | | | |
| $CrO_{1f}C_{1f}$ | 126.4(4) | $C_{1g}O_{1g}C_{4g}$ | 104.3(8) |
| $CrO_{1f}C_{4f}$ | 123.1(5) | $C_{1h}O_{1h}C_{4h}$ | 108.9(9) |
| | | $C_{1i}O_{1i}C_{4i}$ | 107.8(11) |
| $C_{1a}N_{1a}C_{4a}$ | 105.0(6) | $C_{1j}O_{1j}C_{4j}$ | 107.7(7) |
| $C_{1b}N_{1b}C_{4b}$ | 105.2(5) | | |
| $C_{1c}N_{1c}C_{4c}$ | 104.0(5) | $O_{1g}C_{1g}C_{2g}$ | 111(1) |
| $C_{1d}N_{1d}C_{4d}$ | 106.6(6) | $C_{1g}C_{2g}C_{3g}$ | 103(1) |
| $C_{1e}N_{1e}C_{4e}$ | 106.0(6) | $C_{2g}C_{3g}C_{4g}$ | 103(1) |
| | | $C_{3g}C_{4g}O_{1g}$ | 110(1) |
| $C_{1f}O_{1f}C_{4f}$ | 110.5(6) | $O_{1h}C_{1h}C_{2h}$ | 106(1) |
| | | $C_{1h}C_{2h}C_{3h}$ | 106(1) |
| $N_{1a}C_{1a}C_{2a}$ | 111.1(7) | $C_{2h}C_{3h}C_{4h}$ | 109(1) |
| $C_{1a}C_{2a}C_{3a}$ | 106.1(8) | $C_{3h}C_{4h}O_{1h}$ | 106(1) |
| $C_{2a}C_{3a}C_{4a}$ | 107.5(7) | $O_{1i}C_{1i}C_{2i}$ | 110(2) |
| $C_{3a}C_{4a}N_{1a}$ | 110.3(7) | $C_{1i}C_{2i}C_{3i}$ | 105(2) |

**Table XV. (continued)**

| Type [b] | Angle, deg | Type [b] | Angle, deg |
|---|---|---|---|
| $N_{1b}C_{1b}C_{2b}$ | 110.6(7) | $C_{2i}C_{3i}C_{4i}$ | 106(2) |
| $C_{1b}C_{2b}C_{3b}$ | 107.6(8) | $C_{3i}C_{4i}O_{1i}$ | 107(1) |
| $C_{2b}C_{3b}C_{4b}$ | 104.4(7) | $O_{1j}C_{1j}C_{2j}$ | 106(1) |
| $C_{3b}C_{4b}N_{1b}$ | 112.2(7) | $C_{1j}C_{2j}C_{3j}$ | 109(1) |
| $N_{1c}C_{1c}C_{2c}$ | 112.4(7) | $C_{2j}C_{3j}C_{4j}$ | 104(1) |
| $C_{1c}C_{2c}C_{3c}$ | 104.5(7) | $C_{3j}C_{4j}O_{1j}$ | 108(1) |
| $C_{2c}C_{3c}C_{4c}$ | 107.8(7) | | |
| $C_{3c}C_{4c}N_{1c}$ | 111.2(7) | $Na_1C_{1a}N_{1a}$ | 95.0(4) |
| $N_{1d}C_{1d}C_{2d}$ | 109.0(7) | $Na_1C_{1a}C_{2a}$ | 106.7(5) |
| $C_{1d}C_{2d}C_{3d}$ | 107.6(8) | $Na_1N_{1d}Cr$ | 107.7(3) |
| $C_{2d}C_{3d}C_{4d}$ | 105.4(8) | $Na_1N_{1d}C_{1d}$ | 72.6(4) |
| $C_{3d}C_{4d}N_{1d}$ | 111.5(7) | $Na_1N_{1d}C_{4d}$ | 86.4(4) |
| $N_{1e}C_{1e}C_{2e}$ | 111.0(7) | $Na_1C_{1d}N_{1d}$ | 78.1(4) |
| $C_{1e}C_{2e}C_{3e}$ | 105.2(7) | $Na_1C_{1d}C_{2d}$ | 85.1(6) |
| $C_{2e}C_{3e}C_{4e}$ | 108.4(8) | | |
| $C_{3e}C_{4e}N_{1e}$ | 109.5(7) | $Na_2C_{4a}N_{1a}$ | 98.2(3) |
| | | $Na_2C_{4a}C_{3a}$ | 104.0(5) |
| $O_{1f}C_{1f}C_{2f}$ | 104.4(7) | $Na_2C_{1e}N_{1e}$ | 78.1(4) |
| $C_{1f}C_{2f}C_{3f}$ | 105.0(9) | $Na_2C_{1e}C_{2e}$ | 91.7(6) |
| $C_{2f}C_{3f}C_{4f}$ | 104.9(9) | | |
| $C_{3f}C_{4f}O_{1f}$ | 104.7(7) | | |

Table XV. (continued)

[a] The numbers in parentheses are the estimated standard deviations in the last significant digit.
[b] Atoms are labeled in agreement with Figure 1.

Example VII

The product obtained from the reaction of sodium 2,5-dimethylpyrrolide and $CrCl_2$ used in the preparation of an active catalyst was a light blue solid, Product VI. 2,5-Dimethylpyrrole (5.0ml/49.1mmole) was mixed with excess sodium (40% dispersion in mineral spirits) in tetrahydrofuran (125ml) at ambient temperature. The mixture was refluxed 12 hours under nitrogen then filtered to remove excess sodium. The sodium 2,5-dimethylpyrrolide was used in-situ and combined with chromous chloride (3.03g/24.7mmole) at ambient temperature. The reaction mixture was refluxed under nitrogen for 48 hours. The gray-green solution was filtered (medium porosity frit) at ambient temperature and stripped of solvent under vacuum, then pumped dry under vacuum for 12 hours resulting in a gray/green solid. This grey/green solid was then washed with pentane resulting in a light blue solid, Product VI, which was collected by filtration. Product VI was used in the preparation of an active catalyst without further purification.

Example VIII

Preparation of Catalysts

All polymerization runs were carried out in a two liter reactor under slurry (particle form) conditions. The diluent was isobutane and the reactor temperature was 90°C, Reactor pressure held at 550 psig during the polymerization, with ethylene being fed on demand.

The actual charging of the reactor was accomplished by the following method. After purging the reactor at 100°C with a stream of nitrogen for at least 15 minutes, the reactor temperature was lowered to 90°C and a preweighed amount of supported chromium pyrrolide catalyst was charged against a slight countercurrent of nitrogen. One liter of isobutane was then charged to the reactor and finally the reactor pressurized with ethylene.

The ethylene consumed was determined using a precalibrated ethylene flow meter. Samples of the liquid product mixture were taken after 30 minute run time without depressurizing the reactor. This was done by filling to 200-300 psig a steel sampling cylinder adapted to the reactor with a dip tube fitted with a fritted tip extending into the bottom of the reactor vessel. Samples taken this way were analyzed by gas chromatography and gas chromatography-mass spectrometry. Selectivities were normalized to 100%. Solid products were obtained by venting the reactor to atmosphere, separating by decantation of the liquids from the solid material. The solids were then dried at 100°C in a vacuum oven and weighed, The yield of solid product was obtained by weighing the combined solid and catalyst residues and subtracting from this the preweighed catalyst charge. The yield of volatile products was obtained by subtracting the yield of solid products from the grams of ethylene consumed as recorded by the flow meter.

Activity typically ranged from 300-1500 g product/g catalyst/hour calculated for 30 minute run time, as shown in Table XVI. The product obtained typically was represented by 97-99.5% by weight liquids and 0.5-3% by weight polymer (wax). The liquid fraction was typically 85% hexenes, 11% decenes, 2% tetradecences, based on the total weight of the liquid fraction. The balance of the liquid product mixture was a trace level distribution of olefins typically totalling about 1-2% by weight of the product mixture, see Table XVII.

Active catalysts were prepared from the chromium pyrrolide complexes as follows. All toluene and/or pentane rinses used about 15 to about 30mls of liquid.

Run 1: 0.158g of Product V (prepared in THF solvent), which was heated to 80°C for 4 hours under nitrogen flush to remove residual THF, was slurried with 15ml toluene at ambient temperature. 9.0ml of a 1M TEA in hexanes solution was added to the solution and stirred for 24 hours. The formation of a brown solution and the complete dissolution of Product V resulted immediately upon TEA addition. $AlPO_4$(P/Al mole ratio = 0.4) (2.00g) was added to the solution and stirred for an additional 24 hours. The supported catalyst was filtered from the solution as a brown solid, rinsed twice with toluene, and then twice with pentane. 0.3143g of the catalyst was charged directly to the reactor for polymerization. 1.0ml of a 0.5% TEA in heptanes solution was charged to the reactor after the catalyst charge but before the isobutane (reactor solvent) charge to purge feedstock poisons.

Run 2: 0.081g of Product V (prepared in THF solvent), which was heated to 80°C for 4 hours under nitrogen flush

to remove residual THF, was slurried with 15ml diethylbenzene at ambient temperature. 2.0ml of a 1M TEA in hexanes solution was added to the solution and stirred for 24 hours. The formation of a brown solution and the complete dissolution of Product V resulted immediately upon TEA addition. $AlPO_4$(P/Al mole ratio = 0.4) (1.50g) was added to the solution and stirred for an additional 1 hour. The supported catalyst was filtered from the solution as a brown solid, rinsed twice with dimethylbenzene, and then twice with pentane. 0.4333g of the catalyst was charged directly to the reactor for polymerization. 3.0ml of a 0.5% TEA in heptanes solution was charged to the reactor after the catalyst charge but before the isobutane (reactor solvent) charge to purge feedstock poisons.

Run 3: 0.093g of Product V (prepared in DME solvent) was slurried with 15ml toluene at ambient temperature. 5.0ml of a 1M TEA in hexanes solution was added to the solution and stirred for 24 hours. The formation of a brown solution and the complete dissolution of Product V resulted immediately upon TEA addition. $AlPO_4$(P/Al mole ratio = 0.4) (1.0g) was added to the solution and stirred for an additional 24 hours. The supported catalyst was filtered from the solution as a brown solid, rinsed twice with toluene, and then twice with pentane. 0.1564g of the catalyst was charged directly to the reactor for polymerization. 3.0m1 of a 0.5% TEA in heptanes solution was charged to the reactor after the catalyst charge but before the isobutane (reactor solvent) charge to purge feedstock poisons.

Run 4: 0.080g of Product I (prepared in THF solvent) was slurried with 15ml toluene at ambient temperature. 6.0ml of a 1M TEA in hexanes solution was added and the solution stirred for 16 hours. The formation of a brown solution and the complete dissolution of Product I resulted immediately upon TEA addition. $AlPO_4$(P/Al mole ratio = 0.4) (1.50g) was added to the solution and stirred for an additional 16 hours. The supported catalyst was filtered from the solution as a brown solid, rinsed twice with toluene, and then twice pentane. 1.1988g of the catalyst was charged directly to the reactor for polymerization.

Run 5: 0.079g of Product II (prepared in THF solvent) was slurried with 15ml toluene at ambient temperature. 2.0ml of a 1.9H TEA in toluene solution was added to the solution and stirred for 8 hours. The formation of a brown solution and the complete dissolution of Product II resulted immediately upon TEA addition. $AlPO_4$(P/Al mole ratio = 0.4) (0.50g) was added to the solution and stirred for an additional 16 hours. The supported catalyst was filtered from the solution as a brown solid, rinsed twice with toluene, and then twice with pentane. 0.4829g of the catalyst was charged directly to the reactor for polymerization.

Run 6: 0.071g of Product V (prepared in THF solvent), which was heated to 80°C for 4 hours under nitrogen flush to remove residual THF, was slurried with 15ml toluene at ambient temperature. 2.0ml of a 1M TEA in hexanes solution was added to the solution and stirred for 1 hour. The formation of a brown solution and the complete dissolution of Product V resulted immediately upon TEA addition. $SiO_2$(2.52g) was added to the solution and stirred for an additional 2 minutes. The supported catalyst was filtered from the solution as a brown solid, rinsed twice with toluene, and then twice with pentane. All of the catalyst was charged directly to the reactor for polymerization.

Run 7: 0.103g of Product II (prepared in THF solvent) was slurried with 15ml toluene at ambient temperature. 1.0ml of a 1.9M TEA in toluene solution was added to the solution and stirred for 10 minutes. The formation of a brown solution and the complete dissolution of Product II resulted immediately upon TEA addition. $Al_2O_3$ (2.27g) was added to the solution and stirred for an additional 2 minutes. The supported catalyst was filtered from the solution as a brown solid, rinsed twice with toluene, and then twice with pentane. 1.2926g of the catalyst was charged directly to the reactor for polymerization.

Run 8: 0.120g of Product I (prepared in THF solvent) was slurried with 15ml toluene at ambient temperature. 2.0ml of a 1M TEA in hexanes solution was added to the solution and stirred for 2 days. The formation of a brown solution and the complete dissolution of Product I resulted immediately upon TEA addition. $SiO_2$(1.0g) was added to the solution and stirred for an additional 3 weeks. The supported catalyst was filtered from the solution as a brown solid, rinsed twice with toluene, and then twice with pentane. All of the catalyst was charged directly to the reactor for polymerization.

Run 9: 0.106g of Product III (prepared in THF solvent) was slurried with 15ml toluene at ambient temperature. 2.5ml of a 1.9M TEA in toluene solution was added to the solution and stirred for 2 hours. The formation of a brown solution and the complete dissolution of Product III resulted immediately upon TEA addition. $AlPO_4$(P/Al mole ratio = 0.4) (0.65g) was added to the solution and stirred for an additional 2 hours. The supported catalyst was filtered from the solution as a brown solid, rinsed twice with toluene, and then twice with pentane. All of the catalyst was charged directly to the reactor for polymerization. 1.5ml of a 1.0% TEA in pentane solution was charged to the reactor after the catalyst charge but before the isobutane (reactor solvent) charge to purge feedstock poisons.

Run 10: 0.030g of Product V (prepared in THF solvent) which was heated to 80°C for 4 hours under nitrogen flush to remove residual THF was slurried with 15ml toluene at ambient temperature. 3.0ml of a 1M TEA in hexanes solution was added to the solution and stirred for 16 hours. The formation of a brown solution and the complete dissolution of Product V resulted immediately upon TEA addition. $AlPO_4$(P/Al = .9) (2.0g) was added to the solution and stirred for an additional 16 hours. The supported catalyst was filtered from the solution as a brown solid, rinsed twice with toluene, and then twice with pentane. 0.322 g of catalyst was charged directly to the reactor for polymerization.

Run 11: 0.067g of Product V (prepared in THF solvent) was slurried with 15ml pentane at ambient temperature. 4.0ml of a 1M TEA in hexanes solution was added to the solution and stirred for 24 hours. The formation of a brown

solution and the complete dissolution of Product V resulted immediately upon TEA addition. $AlPO_4$(P/Al mole ratio = 0.4) (1.0g) was added to the solution and stirred for an additional 24 hours. The supported catalyst was filtered from the solution as a brown solid, rinsed twice with pentane. All of the catalyst was charged directly to the reactor for polymerization. 3.0ml of a 0.5% TEA in heptanes solution was charged to the reactor after the catalyst charge but before the isobutane (reactor solvent) charge to purge feedstock poisons.

Run 12: 0.073g of Product V (prepared in THF solvent), which was heated to 80°C for 4 hours under nitrogen flush to remove residual THF, was slurried with 15ml toluene at ambient temperature. 6.0ml of a 1M TEA in hexanes solution was added and the solution stirred for 24 hours. The formation of a brown solution and the complete dissolution of Product V resulted immediately upon TEA addition. $P/SiO_2$ (7.0g) was added to the solution and stirred for an additional 24 hours which nearly decolorized it. The supported catalyst was filtered from the solution as a brown solid, rinsed twice with toluene, and then twice with pentane. 2.85g of catalyst was charged directly to the reactor for polymerization.

Run 13: 0.125g of Product II was slurried with 15ml diethylbenzene at ambient temperature. 9.0ml of a 1M TEA in hexanes solution was added to the solution and stirred for 8 hours. The formation of a brown solution and the complete dissolution of Product II resulted immediately upon TEA addition. $F/Al_2O_3$ (2.0g) was added to the solution and stirred for an additional 12 hours. The supported catalyst was filtered from the solution as a brown solid, rinsed twice with toluene, and then twice with pentane. 0.5477g of catalyst was charged directly to the reactor for polymerization.

Run 14: 0.125g of Product VI was slurried with 15ml toluene at ambient temperature. 1.5ml of a 1M TEA in hexanes solution was added and the solution stirred for 10 minutes. The formation of a red/brown solution and the complete dissolution of Product VI resulted immediately upon TEA addition. $SiO_2$ (2.0g) was added to the solution and stirred for an additional 1 minute which nearly decolorized it. The supported silica catalyst was filtered from the solution as a red/brown solid, rinsed twice with toluene, and then twice with pentane. All of the catalyst was charged directly to the reactor for polymerization.

Run 15: 0.30g of Product V (prepared in DME solvent) was dissolved with 15ml of dimethoxyethane forming a green solution. This solution was then mixed with 0.6g of $AlPO_4$(P/Al mole ratio = 0.4) (2.00g) and the mixture was stirred 1 hour. The green supported material was filtered from the solution, rinsed with dimethoxyethane and dried with a nitrogen purge at 90°C. This material was then stirred with 15ml of toluene and 3ml of triethylaluminum (Aldrich 1.0M, hexanes) for an additional 3 hours. The brown supported catalyst was collected by filtration, rinsed with pentane, and dried under vacuum. 0.4609g of the catalyst was charged directly to the reactor for polymerization. 3.0ml of a 0.5% TEA in heptanes solution was charged to the reactor after the catalyst charge but before the isobutane (reactor solvent) charge to purge feedstock poisons.

Run 16: 0.058g of Product V (prepared in THF solvent) which was heated to 80°C for 4 hours under nitrogen flush to remove residual THF was slurried with 15ml benzene at ambient temperature. 4.0ml of a 1M TEA in hexanes solution was added and the solution stirred for 2 hours. The formation of a brown solution and the complete dissolution of Product V resulted immediately upon TEA addition. $AlPO_4$(P/Al mole ratio = 0.4) (1.0g) was added to the solution and stirred for 1 hour. The supported catalyst was filtered from the solution as a brown solid, rinsed twice with benzene, and then twice with pentane. All of the catalyst was charged directly to the reactor for polymerization. 3.0ml of a 0.5% TEA in heptanes solution was charged to the reactor after the catalyst charge but before the isobutane (reactor solvent) charge to purge feedstock poisons.

Run 17: 0.1610g of Product I was charged directly to the reactor at 90°C. The reactor was charged with 1 liter isobutane and pressurized to 550 psig with ethylene. No ethylene consumption was detected, therefore 50 psig of dihydrogen was charged to the reactor which did not initiate ethylene consumption. Ethylene consumption was initiated after 2.0ml of 1M TEA in hexanes solution was charged.

Run 18: 0.3528g of Product VI was charged directly to the reactor at 90°C. The reactor was charged with 1 liter isobutane and pressurized to 550 psig with ethylene. No ethylene consumption was detected, therefore 2.0ml of a 1M TEA in hexanes solution was charged which did initiate ethylene consumption.

Run 19: 0.3482g of Product VI was charged directly to the reactor at 90°C. The reactor was also charged with 2.0ml of a 1M TEA in hexanes solution prior to a 1 liter isobutane charge. The reactor was then pressurized to 550 psig with ethylene. No ethylene consumption was detected, therefore 30 psi of dihydrogen was charged to the reactor which initiated ethylene consumption.

Run 20: 0.202g of Product V (prepared in dimethoxyethane (DME) solvent), 6.0ml of a 1.9H TEA in toluene solution, and 2.0g of $AlPO_4$(P/Al mole ratio = 0.4) were mixed with 15ml toluene at ambient temperature. The formation of a brown solution and the complete dissolution of Product V resulted immediately upon mixing. The brown solution was stirred for 48 hours. The supported catalyst was filtered from the solution as a brown solid, rinsed twice with toluene, and then twice with pentane. 0.0671g of the catalyst was charged directly to the reactor for polymerization. 1.0 ml of a 0.5% TEA in heptanes solution was charged to the reactor after the catalyst charge but before the isobutane (reactor solvent) charge to purge feedstock poisons.

The data in Table XVI show that the inventive chromium compounds can be used either supported (Runs 1-16,

20) or unsupported (Runs 17-19) to polymerize and/or trimerize olefins. Furthermore, conditions can be varied to increase the amount of trimer product (Runs 1-5 and 9) or to have a higher yield of solid, or polymer, product (Runs 6, 7, and 13). Runs 1, 3, and 20 demonstrate that high activities are attainable.

Table XVI

| Run[a] | Catalyst | Support[b] | Products | Activity[c] |
|---|---|---|---|---|
| 1 | (V)/TEA/Toluene | $AlPO_4$ | 98.4% liquids, 1.6% solids | 1030 |
| 2 | (V)/TEA/Diethylbenzene | $AlPO_4$ | 99.4% liquids, 0.6% solids | 730 |
| 3 | (V)/TEA/Toluene | $AlPO_4$ | 99.4% liquids, 0.6% solids | 1450[d] |
| 4 | (I)/TEA/Toluene | $AlPO_4$ | 98.6% liquids, 1.4% solids | 360 |
| 5 | (II)/TEA/Toluene | $AlPO_4$ | 98.2% liquids, 1.8% solids | 580 |
| 6 | (V)/TEA/Toluene | $SiO_2$ | 89.0% liquids, 11.0% solids | 80 |
| 7 | (II)/TEA/Toluene | $Al_2O_3$ | 55.8% liquids, 44.2% solids | 50 |
| 8 | (I)/TEA/Toluene | $SiO_2$ | 93.3% liquids, 6.7% solids | 400 |
| 9 | (III)/TEA/Toluene | $AlPO_4$ | 99.8% liquids, 0.2% solids | 100 |
| 10 | (V)/TEA/Toluene | $AlPO_4$(.9) | 96.8% liquids, 3.2% solids | 930 |
| 11 | (V)/TEA/Pentane | $AlPO_4$ | (trace) liquids, (trace) solids | unreactive |
| 12 | (V)/TEA/Toluene | $P/SiO_2$ | 98.1% liquids, 1.9% solids | 90 |
| 13 | (II)/TEA/Diethylbenzene | $F/Al_2O_3$ | 88.0% liquids, 12.0% solids | 300 |
| 14 | (VI)/TEA/Toluene | $SiO_2$ | 94.3% liquids, 5.7% solids | 40 |
| 15 | (V)/DME | $AlPO_4$ | 98.0% liquids, 2.0% solids | 550 |

Table XVI Cont.

| Run[a] | Catalyst | Support[b] | Products | Activity[c] |
|---|---|---|---|---|
| 16 | (V)/TEA/Benzene | $AlPO_4$ | 99.1% liquids, 0.9% solids | 500 |
| 17 | (I)/TEA | unsupported | 98.3% liquids, 1.7% solids | 340 |
| 18 | (VI)/TEA | unsupported | 99.4% liquids, 0.6% solids | 180 |
| 19 | (VI)/TEA | unsupported | 98.1% liquids, 1.9% solids | 230 |
| 20 | (V)/TEA | $AlPO_4$ | 99.5% liquids, 0.5% solids | 2760 |

[a] All runs were made at 90°C, isobutane solvent, 550 psi total pressure.

[b] P/Al mole ratio = 0.4; except Run 10 whereby P/Al mole ratio = 0.9

[c] Grams product/grams chromium/hour based on 30 min. run times.

[d] Believed to be lower than actual due to experimental error; actual value believed to be near 2000.

Table XVII

| Run | C4 | 1-hexene | C6 | C8 | C10 | C12 | C14 | C16-C28 |
|---|---|---|---|---|---|---|---|---|
| 1 | 0.05 | 81.92 | 7.76 | 0.49 | 9.12 | 0.09 | 0.52 | .05 |
| 2 | 0.10 | 78.80 | 7.49 | 0.58 | 11.36 | 0.10 | 1.01 | .56 |
| 3 | 0.06 | 82.19 | 7.68 | 0.45 | 8.85 | 0.08 | 0.58 | .11 |
| 5 | 0.10 | 83.40 | 7.08 | 0.62 | 8.08 | 0.05 | 0.42 | .25 |
| 6 | 0.55 | 78.70 | 5.52 | 1.84 | 11.24 | 0.42 | 1.26 | .47 |
| 16 | 0.06 | 72.85 | 13.61 | 0.31 | 12.06 | 0.09 | 0.93 | .09 |
| 19 | 6.03 | 71.66 | 6.09 | 3.61 | 9.42 | 1.17 | 1.41 | .61 |

Example IX

Single crystal x-ray structures were obtained for $Cr(NC_4H_4)_3Cl(O_2C_2H_4(CH_3)_2)_3Na$, shown in Figures 7 and 8, and $Cr(NC_4H_4)_3Cl(O_2C_2H_4(CH_3)_2)$, and shown in Figure 9. These crystals were obtained in accordance with the procedure given in Example III. However, X-ray quality crystals were obtained after the dark green, filtered solution was maintained at ambient temperature and pressure under an inert atmosphere, nitrogen, for a time of about 2 days. Analysis calculated for $C_{24}H_{42}N_3O_6CrNacl$: C, 49-78; H, 7.31; N, 7.26% by weight. Found: C, 49.80; H 7.39; N, 7.18% by weight.

The description of the single-crystal sample and mounting used for data collection are as follows:

Color: Green-black
Shape: Rectangular parallelpiped
Dimensions: 0.44 x 0.62 x 0.62 mm
Crystal Mount: Crystal was glued to the inside of a thin-walled glass capillary under $N_2$.
Crystal Orientation: Crystal was oriented with one of its longer edges nearly parallel to the phi axis of the diffractometer.
Width at half-height from ω scans: 0.38°

The space group and cell data are as follows:

Crystal System: Monoclinic
Space Group and Number:[2] $P2_1/c$ - $C^5_{2h}$ (No. 14)
Number of Computer-Centered Reflections Used in the Least-Squares Refinement of the Cell Dimensions:
15 2θ>25° °C = 20±1
Lattice Constants with esd's:

| | | |
|---|---|---|
| a = 8.135(2)Å | α = 90.00° | V = 3027(1)$Å^3$ |
| b = 22.337(5)Å | β = 91.67(2)° | Z = 4 |
| c = 16.667(4)Å | γ = 90.00° | λ = 0.71073Å |

Molecular Weight: 579.05 amu
Calculated Density: 1.271 $g/cm^3$
Linear Absorption coefficient:[3a] 0.51$mm^{-1}$

Tables XVIII - XXII list the resultant parameters used to generate the molecular structures shown in Figure 7 and Figure 8.

Table XVIII Atomic Coordinates for Nonhydrogen Atoms in Crystalline $[Cr(NC_4H_4)_3(Cl)(O_2C_2H_4(CH_3)_2)_3Na]$ [a]

| Atom Type [b] | Fractional Coordinates $10^4x$ | $10^4y$ | $10^4z$ | Equivalent Isotropic Thermal Parameter, B, $Å^2$ x 10 [c] |
|---|---|---|---|---|
| Cr | -1030(1) | 559(1) | 3005(1) | 30(1) |
| Cl | 135(1) | -26(1) | 1981(1) | 41(1) |
| Na | -2167(2) | -1011(1) | 1832(1) | 46(1) |
| $N_1$ | -3062(4) | 65(2) | 2907(2) | 35(1) |
| $C_{11}$ | -4107(5) | 63(2) | 2251(3) | 40(1) |
| $C_{12}$ | -5189(5) | -409(2) | 2291(3) | 51(1) |
| C | -4810(5) | -713(2) | 2998(3) | 51(1) |
| $C_{14}$ | -3512(5) | -414(2) | 3361(3) | 46(1) |
| $N_2$ | -1817(4) | 1027(2) | 3950(2) | 37(1) |
| $C_{21}$ | -1188(6) | 1558(2) | 4234(3) | 47(1) |
| $C_{22}$ | -2205(7) | 1790(2) | 4799(3) | 60(2) |
| $C_{23}$ | -3499(7) | 1398(2) | 4874(3) | 60(2) |
| $C_{24}$ | -3248(5) | 934(2) | 4349(2) | 43(1) |
| $N_3$ | -1892(4) | 1185(2) | 2260(2) | 35(1) |
| $C_{31}$ | -3100(5) | 1588(2) | 2434(3) | 41(1) |
| $C_{32}$ | -3573(6) | 1901(2) | 1757(3) | 53(1) |
| $C_{33}$ | -2631(6) | 1686(2) | 1130(3) | 51(1) |
| $C_{34}$ | -1620(6) | 1249(2) | 1453(3) | 46(1) |
| $O_{1a}$ | 1317(3) | 971(1) | 3154(2) | 40(1) |
| $O_{2a}$ | 153(3) | -12(1) | 3878(2) | 40(1) |
| $C_{1a}$ | 2459(5) | 631(2) | 3651(3) | 53(1) |

Table XVIII (continued)

| Atom Type [b] | Fractional Coordinates $10^4x$ | $10^4y$ | $10^4z$ | Equivalent Isotropic Thermal Parameter, B, $Å^2 \times 10$ [c] |
|---|---|---|---|---|
| $C_{2a}$ | 1443(6) | 329(2) | 4268(3) | 53(1) |
| $C_{3a}$ | 2156(6) | 1247(2) | 2495(3) | 58(2) |
| $C_{4a}$ | 653(6) | -625(2) | 3733(3) | 49(1) |
| $O_{1b}$ | -2558(4) | -783(2) | 398(2) | 62(1) |
| $O_{2b}$ | -3877(5) | -1772(2) | 1111(2) | 76(1) |
| $C_{1b}$ | -3618(9) | -1166(3) | -25(4) | 89(2) |
| $C_{2b}$ | -3627(9) | -1765(3) | 302(4) | 83(2) |
| $C_{3b}$ | -2410(8) | -207(3) | 61(4) | 79(2) |
| $C_{4b}$ | -4149(9) | -2328(3) | 1440(5) | 106(3) |
| $O_{1c}$ | -1334(4) | -1823(2) | 2911(2) | 65(1) |
| $O_{2c}$ | 235(5) | -1589(2) | 1529(3) | 87(2) |
| $C_{1c}$ | 71(7) | -2144(3) | 2724(4) | 83(2) |
| $C_{2c}$ | 951(8) | -1913(4) | 2067(4) | 107(3) |
| $C_{3c}$ | -2090(8) | -2017(3) | 3614(4) | 83(2) |
| $C_{4c}$ | 1224(8) | -1393(3) | 900(4) | 88(2) |

[a] The numbers in parentheses are the estimated standard deviations in the last significant digit.
[b] Atoms are labeled in agreement with Figure 1.
[c] This is one-third of the trace of the orthogonalized $B_{ij}$ tensor.

Table XIX Anisotropic Thermal Parameters for Nonhydrogen Atoms in Crystalline $[Cr(NC_4H_4)_3(Cl)(O_2C_2H_4(CH_3)_2)_3Na]$ [a,b]

| Atom Type [c] | Anisotropic Thermal Parameter ($Å^2$ x 10) $B_{11}$ | $B_{22}$ | $B_{33}$ | $B_{12}$ | $B_{13}$ | $B_{23}$ |
|---|---|---|---|---|---|---|
| Cr | 28(1) | 31(1) | 30(1) | 2(1) | -0(1) | -2(1) |
| Cl | 39(1) | 43(1) | 41(1) | 2(1) | 5(1) | -9(1) |
| Na | 47(1) | 48(1) | 44(1) | 0(1) | 3(1) | -4(1) |
| $N_1$ | 31(1) | 39(2) | 35(2) | 0(1) | 2(1) | -3(1) |
| $C_{11}$ | 31(2) | 47(2) | 41(2) | 0(2) | -1(2) | -7(2) |
| $C_{12}$ | 33(2) | 59(3) | 61(3) | -3(2) | -4(2) | -16(2) |
| $C_{13}$ | 35(2) | 39(2) | 79(3) | -6(2) | 8(2) | 3(2) |
| $C_{14}$ | 39(2) | 45(2) | 54(2) | 1(2) | 2(2) | 10(2) |
| $N_2$ | 36(2) | 38(2) | 36(2) | 7(1) | -3(1) | -8(1) |
| $C_{21}$ | 55(2) | 38(2) | 47(2) | 9(2) | -6(2) | -5(2) |
| $C_{22}$ | 88(3) | 46(3) | 44(2) | 32(2) | -9(2) | -12(2) |
| $C_{23}$ | 65(3) | 74(3) | 42(2) | 32(2) | 7(2) | 0(2) |
| $C_{24}$ | 37(2) | 55(2) | 37(2) | 14(2) | -0(2) | 1(2) |
| $N_3$ | 38(2) | 35(2) | 32(2) | 3(1) | 0(1) | -0(1) |
| $C_{31}$ | 35(2) | 43(2) | 43(2) | 6(2) | -3(2) | 1(2) |
| $C_{32}$ | 52(2) | 47(2) | 58(3) | 8(2) | -11(2) | 6(2) |
| $C_{33}$ | 62(3) | 51(3) | 39(2) | -2(2) | -8(2) | 12(2) |
| $C_{34}$ | 52(2) | 45(2) | 40(2) | -1(2) | 2(2) | 2(2) |
| $O_{1a}$ | 32(1) | 40(1) | 50(2) | -1(1) | -3(1) | -5(1) |
| $O_{2a}$ | 40(1) | 38(1) | 41(1) | 6(1) | -7(1) | -1(1) |
| $C_{1a}$ | 33(2) | 50(3) | 73(3) | 4(2) | -13(2) | -10(2) |
| $C_{2a}$ | 53(2) | 55(3) | 51(2) | 10(2) | -24(2) | -10(2) |
| $C_{3a}$ | 45(2) | 53(3) | 76(3) | -15(2) | 8(2) | -5(3) |

Table XIX (continued)

| Atom | Anisotropic Thermal Parameter (Å$^2$ x 10) | | | | | |
|---|---|---|---|---|---|---|
| Type [c] | $B_{11}$ | $B_{22}$ | $B_{33}$ | $B_{12}$ | $B_{13}$ | $B_{23}$ |
| $C_{4a}$ | 50(2) | 40(2) | 58(3) | 12(2) | -8(2) | -1(2) |
| $O_{1b}$ | 76(2) | 63(2) | 47(2) | -14(2) | -5(2) | 1(2) |
| $O_{2b}$ | 101(3) | 62(2) | 63(2) | -28(2) | -5(2) | -2(2) |
| $C_{1b}$ | 120(5) | 91(4) | 56(3) | -29(4) | -25(3) | -3(3) |
| $C_{2b}$ | 116(5) | 64(3) | 68(4) | -18(3) | -24(3) | -12(3) |
| $C_{3b}$ | 81(4) | 84(4) | 72(4) | -9(3) | -1(3) | 19(3) |
| b | 118(5) | 84(4) | 113(5) | -51(4) | -38(4) | 29(4) |
| $O_{1c}$ | 61(2) | 64(2) | 70(2) | 8(2) | 0(2) | 4(2) |
| $O_{2c}$ | 74(2) | 76(3) | 112(3) | 29(2) | 31(2) | 30(2) |
| $C_{1c}$ | 73(3) | 65(3) | 113(5) | 23(3) | 9(3) | 25(3) |
| $C_{2c}$ | 83(4) | 143(6) | 96(5) | 61(4) | 24(4) | 14(5) |
| $C_{3c}$ | 84(4) | 64(3) | 101(5) | -8(3) | 3(4) | 16(3) |
| $C_{4c}$ | 77(4) | 98(5) | 90(5) | 13(3) | 29(3) | -5(4) |

[a] The numbers in parentheses are the estimated standard deviations in the last significant digit.

[b] The form of the anisotropic thermal parameter is given in reference 8 on page 6 of the crystal structure analysis report.

[c] Atoms are labeled in agreement with Figure 1.

Table XX Atomic Coordinates for Hydrogen Atoms in Crystalline $[Cr(NC_4H_4)_3(Cl)(O_2C_2H_4(CH_3)_2)_3Na]$ [a]

| Atom Type [b] | Fractional Coordinates $10^4x$ | $10^4y$ | $10^4z$ |
|---|---|---|---|
| $H_{11}$ | -4089 | 350 | 1823 |
| $H_{12}$ | -6043 | -509 | 1905 |
| $H_{13}$ | -5349 | -1064 | 3195 |
| $H_{14}$ | -2993 | -526 | 3863 |
| $H_{21}$ | -188 | 1740 | 4063 |
| $H_{22}$ | -2044 | 2158 | 5089 |
| $H_{23}$ | -4404 | 1441 | 5226 |
| $H_{24}$ | -3967 | 597 | 4273 |
| $H_{31}$ | -3554 | 1644 | 2954 |
| $H_{32}$ | -4392 | 2210 | 1720 |
| $H_{33}$ | -2680 | 1817 | 581 |
| $H_{34}$ | -840 | 1021 | 1159 |
| $H_{1aa}$ | 3014 | 339 | 3336 |
| $H_{1ab}$ | 3254 | 892 | 3906 |
| $H_{2aa}$ | 967 | 626 | 4606 |
| $H_{2ab}$ | 2127 | 67 | 4588 |
| $H_{3aa}$ | 1391 | 1487 | 2185 |
| $H_{3ab}$ | 2589 | 938 | 2162 |
| $H_{3ac}$ | 3040 | 1495 | 2696 |
| $H_{4aa}$ | -256 | -834 | 3484 |
| $H_{4ab}$ | 926 | -806 | 4242 |
| $H_{4ac}$ | 1586 | -646 | 3395 |

Table XX (continued)

| Atom Type [b] | Fractional Coordinates $10^4x$ | $10^4y$ | $10^4z$ |
|---|---|---|---|
| $H_{1ba}$ | -4712 | -1006 | -11 |
| $H_{1bb}$ | -3277 | -1186 | -570 |
| $H_{2ba}$ | -2588 | -1951 | 204 |
| $H_{2bb}$ | -4492 | -1988 | 37 |
| $H_{3ba}$ | -1696 | 26 | 407 |
| $H_{3bb}$ | -3461 | -14 | 7 |
| $H_{3bc}$ | -1935 | -243 | -458 |
| $H_{4ba}$ | -4380 | -2289 | 2000 |
| $H_{4bb}$ | -3108 | -2524 | 1385 |
| $H_{4bc}$ | -4998 | -2561 | 1178 |
| $H_{1ca}$ | 795 | -2146 | 3189 |
| $H_{1cb}$ | -255 | -2547 | 2596 |
| $H_{2ca}$ | 1398 | -2252 | 1795 |
| $H_{2cb}$ | 1831 | -1676 | 2294 |
| $H_{3ca}$ | -3168 | -1848 | 3661 |
| $H_{3cb}$ | -1397 | -1884 | 4055 |
| $H_{3cc}$ | -2164 | -2446 | 3624 |
| $H_{4ca}$ | 456 | -1357 | 454 |
| $H_{4cb}$ | 2033 | -1690 | 780 |
| $H_{4cc}$ | 1756 | -1015 | 996 |

[a] The 6 methyl groups were refined as rigid rotors with $sp^3$-hybridized geometry and C-H bond lengths of 0.96Å. The initial orientation of each methyl group was determined from difference Fourier positions for the hydrogen atoms. The final orientation of each methyl group

Table XX . (continued)

was determined by three rotational parameters. The refined positions for the rigid rotor methyl groups gave O-C-H angles which ranged from 103° to 115°. The remaining hydrogen atoms were included in the structure factor calculations as idealized atoms (assuming $sp^2$- or $sp^3$-hybridization of the carbon atoms and a C-H bond length of 0.96Å) "riding" on their respective carbon atoms. The isotropic thermal parameter of each hydrogen was fixed at 1.2 times the equivalent isotropic thermal parameter of the carbon atom to which it is covalently bonded.

b Hydrogens are labeled with the same subscripts as their carbon atoms with an additional literal subscript (a , b or c) where necessary to distinguish between hydrogen atoms bonded to the same carbon.

Table XXI Bond Lengths Involving Nonhydrogen Atoms in Crystalline $[Cr(NC_4H_4)_3(Cl)(O_2C_2H_4(CH_3)_2)_3Na]$ [a]

| Type [b] | Length, Å | Type [b] | Length, Å |
|---|---|---|---|
| Cr-Cl | 2.369(1) | $Cr-N_1$ | 1.990(3) |
| | | $Cr-N_2$ | 2.010(3) |
| $Cr-O_{1a}$ | 2.128(3) | $Cr-N_3$ | 1.986(3) |
| $Cr-O_{2a}$ | 2.142(3) | | |
| $N_1-C_{11}$ | 1.365(5) | $C_{11}-C_{12}$ | 1.376(6) |
| $N_1-C_{14}$ | 1.366(6) | $C_{12}-C_{13}$ | 1.386(7) |
| $N_2-C_{21}$ | 1.370(6) | $C_{13}-C_{14}$ | 1.376(6) |
| $N_2-C_{24}$ | 1.374(5) | $C_{21}-C_{22}$ | 1.373(7) |
| $N_3-C_{31}$ | 1.370(5) | $C_{22}-C_{23}$ | 1.377(8) |
| $N_3-C_{34}$ | 1.376(6) | $C_{23}-C_{24}$ | 1.375(7) |
| | | $C_{31}-C_{32}$ | 1.373(7) |
| $O_{1a}-C_{1a}$ | 1.443(5) | $C_{32}-C_{33}$ | 1.399(7) |
| $O_{1a}-C_{3a}$ | 1.448(6) | $C_{33}-C_{34}$ | 1.375(7) |
| $O_{2a}-C_{2a}$ | 1.437(5) | | |
| $O_{2a}-C_{4a}$ | 1.450(5) | $C_{1a}-C_{2a}$ | 1.498(7) |
| $O_{1b}-C_{1b}$ | 1.391(8) | $C_{1b}-C_{2b}$ | 1.445(9) |
| $O_{1b}-C_{3b}$ | 1.410(7) | $C_{1c}-C_{2c}$ | 1.422(10) |
| $O_{2b}-C_{2b}$ | 1.370(7) | | |
| $O_{2b}-C_{4b}$ | 1.379(8) | Na···Cr | 4.108(2) |
| $O_{1c}-C_{1c}$ | 1.392(7) | | |
| $O_{1c}-C_{3c}$ | 1.408(8) | Na-Cl | 2.896(2) |
| $O_{2c}-C_{2c}$ | 1.278(9) | | |
| $O_{2c}-C_{4c}$ | 1.409(8) | $Na-N_1$ | 3.098(4) |

Table XXI (continued)

| Type [b] | Length, Å | Type [b] | Length, Å |
|---|---|---|---|
| Na-$C_{11}$ | 2.967(5) | Na-$O_{1b}$ | 2.454(4) |
| Na-$C_{12}$ | 2.924(5) | Na-$O_{2b}$ | 2.483(4) |
| Na-$C_{13}$ | 3.015(5) | Na-$O_{1c}$ | 2.629(5) |
| Na-$C_{14}$ | 3.104(5) | Na-$O_{2c}$ | 2.408(5) |
| Na-$C_{g1}$ [c] | 2.788(-) | | |

[a] The numbers in parentheses are the estimated standard deviations in the last significant digit.
[b] Atoms are labeled in agreement with Figure 1.
[c] The symbol $C_{g1}$ is used to designate the center of gravity for the five-membered ring containing $N_1$, $C_{11}$, $C_{12}$, $C_{13}$, and $C_{14}$; this value is therefore listed without an estimated standard deviation.

Table XXII Bond Angles Involving Nonhydrogen Atoms in Crystalline $[Cr(NC_4H_4)_3(Cl)(O_2C_2H_4(CH_3)_2)_3Na]$ [a]

| Type [b] | Angle, deg. | Type [b] | Angle, deg. |
|---|---|---|---|
| $ClCrN_1$ | 89.1(1) | $ClCrN_2$ | 173.9(1) |
| $N_1CrN_2$ | 94.0(1) | $ClCrN_3$ | 94.5(1) |
| $N_1CrN_3$ | 93.5(1) | $N_2CrN_3$ | 90.5(1) |
| $ClCrO_{1a}$ | 86.9(1) | $N_1CrO_{1a}$ | 171.9(1) |
| $N_2CrO_{1a}$ | 89.4(1) | $N_3CrO_{1a}$ | 93.8(1) |
| $ClCrO_{2a}$ | 88.9(1) | $N_1CrO_{2a}$ | 94.7(1) |
| $N_2CrO_{2a}$ | 85.7(1) | $N_3CrO_{2a}$ | 171.2(1) |
| $_{1a}CrO_{2a}$ | 78.2(1) | | |
| | | | |
| $CrN_1C_{11}$ | 124.4(3) | $C_{11}N_1C_{14}$ | 105.7(3) |
| $CrN_1C_{14}$ | 128.6(3) | $C_{21}N_2C_{24}$ | 106.1(4) |
| $CrN_2C_{21}$ | 126.6(3) | $C_{31}N_3C_{34}$ | 106.0(3) |
| $CrN_2C_{24}$ | 126.5(3) | | |
| $CrN_3C_{31}$ | 125.0(3) | $CrO_{1a}C_{1a}$ | 113.5(2) |
| $CrN_3C_{34}$ | 128.3(3) | $CrO_{1a}C_{3a}$ | 122.5(3) |
| | | $C_{1a}O_{1a}C_{3a}$ | 110.5(3) |
| N $C_{11}C_{12}$ | 110.3(4) | $CrO_{2a}C_{2a}$ | 107.4(2) |
| $C_{11}C_{12}C_{13}$ | 106.9(4) | $CrO_{2a}C_{4a}$ | 124.9(3) |
| $C_{12}C_{13}C_{14}$ | 106.5(4) | $C_{2a}O_{2a}C_{4a}$ | 111.8(3) |
| $N_1C_{14}C_{13}$ | 110.6(4) | $C_{1b}O_{1b}C_{3b}$ | 114.7(4) |
| $N_2C_{21}C_{22}$ | 109.7(4) | $C_{2b}O_{2b}C_{4b}$ | 115.6(5) |
| $C_{21}C_{22}C_{23}$ | 107.4(4) | $C_{1c}O_{1c}C_{3c}$ | 114.2(5) |
| $C_{22}C_{23}C_{24}$ | 107.1(4) | $C_{2c}O_{2c}C_{4c}$ | 116.0(5) |

Table XXII (continued)

| Type [b] | Angle, deg. | Type [b] | Angle, deg. |
|---|---|---|---|
| $N_2C_{24}C_{23}$ | 109.7(4) | | |
| $N_3C_{31}C_{32}$ | 110.3(4) | $O_{1a}C_{1a}C_{2a}$ | 105.8(3) |
| $C_{31}C_{32}C_{33}$ | 107.0(4) | $O_{2a}C_{2a}C_{1a}$ | 109.8(4) |
| $C_{32}C_{33}C_{34}$ | 106.6(4) | $O_{1b}C_{1b}C_{2b}$ | 112.8(5) |
| $N_3C_{34}C_{33}$ | 110.2(4) | $O_{2b}C_{2b}C_{1b}$ | 112.6(5) |
| | | $O_{1c}C_{1c}C_{2c}$ | 114.9(6) |
| $ClNaC_{g1}$ [c] | 83.6(-) | $O_{2c}C_{2c}C_{1c}$ | 121.1(6) |
| $ClNaO_{1b}$ | 89.5(1) | $C_{g1}NaO_{1b}$ [c] | 111.1(-) |
| $ClNaO_{2b}$ | 156.0(1) | $C_{g1}NaO_{2b}$ [c] | 110.2(-) |
| $ClNaO_{1c}$ | 108.2(1) | $C_{g1}NaO_{1c}$ [c] | 99.4(-) |
| $ClNaO_{2c}$ | 84.2(1) | $C_{g1}NaO_{2c}$ [c] | 155.9(-) |
| $ClNaN_1$ | 61.5(1) | $O_{1b}NaO_{2b}$ | 67.4(2) |
| $ClNaC_{11}$ | 73.3(2) | $O_{1b}NaO_{1c}$ | 146.4(2) |
| $ClNaC_{12}$ | 100.0(2) | $O_{1b}NaO_{2c}$ | 89.4(2) |
| $ClNaC_{13}$ | 104.4(2) | $O_{2b}NaO_{1c}$ | 89.3(2) |
| $ClNaC_{14}$ | 81.1(2) | $O_{2b}NaO_{2c}$ | 88.8(2) |
| | | $O_{1c}NaO_{2c}$ | 65.1(2) |
| $N_1NaC_{11}$ | 25.9(2) | | |
| $N_1NaC_{14}$ | 25.5(2) | $N_1NaC_{12}$ | 43.8(2) |
| $C_{11}NaC_{12}$ | 27.0(2) | $N_1NaC_{13}$ | 43.2(2) |
| $C_{12}NaC_{13}$ | 26.9(2) | $C_{11}NaC_{13}$ | 43.5(2) |

Table XXII (continued)

| Type [b] | Angle, deg. | Type [b] | Angle, deg. |
|---|---|---|---|
| $C_{13}NaC_{14}$ | 25.9(2) | $C_{12}NaC_{14}$ | 42.9(2) |
| | | $C_{11}NaC_{14}$ | 41.9(2) |

[a] The numbers in parentheses are the estimated standard deviations in the last significant digit.
[b] Atoms are labeled in agreement with Figure 1.
[c] The symbol $C_{g1}$ is used to designate the center of gravity for the five-membered ring containing $N_1$, $C_{11}$, $C_{12}$, $C_{13}$, and $C_{14}$; this value is therefore listed without an estimated standard deviation.

## Claims

### Claims for the following Contracting States : AT, BE, CH, LI, DE, DK, FR, GB, GR, IT, LU, NL, SE

1. A process for preparing chromium-compounds, characterized by refluxing a mixture comprising a chromium salt, a metal amide and an electron pair donor solvent.

2. The process of claim 1, wherein refluxing occurs during a period of time from 1 minute to 1 week.

3. The process of claim 1 or 2, wherein said chromium salt is selected from chromium adds, chromium halides, and mixtures thereof.

4. The process of claim 3, wherein said chromium halide is selected from chromous chloride, chromic chloride, and mixtures thereof.

5. The process of any of claims 1-4 wherein said metal amide is selected from alkali metal amide salts, alkaline earth metal amide salts, silyl amide salts, and mixtures thereof.

6. The process of any of claims 1-5, wherein said metal amide has from 1 to 20 carbon atoms.

7. The process of any of claims 1-6, wherein said metal amide is an unsaturated compound.

8. The process of any of claims 1-7, wherein said metal amide is an aromatic compound.

9. The process of claim 1, wherein said metal amide has at least one amine ligand being selected from pyrrole ligands, derivatives of pyrrole ligands, and mixtures thereof.

10. The process of any of claims 1-9, wherein said metal amide is soluble in said electron pair donor solvent.

11. The process of any of claims 1-10, wherein said electron pair donor solvent has from 2-20 carbon atoms.

12. The process of any of claims 1-11, wherein said electron pair donor solvent is an aliphatic compound.

13. The process of any of claims 1-12, wherein said electron pair donor solvent is an ether selected from tetrahydrofuran derivatives of tetrahydrofuran, dimethoxyethane, derivates of dimethoxyethane, and mixtures thereof.

**14.** The process of any of claims 1 to 13, wherein, upon completion of the reaction, the liquid is filtered to remove any reaction by-products.

**15.** The process of claim 14, wherein the thus-filtered liquid is treated to form a solid.

**16.** The process of claim 15, wherein the thus-filtered liquid is slowly evaporated.

**17.** The process of claim 15, wherein the thus-filtered liquid is treated with a non-polar solvent.

**18.** The process of any of claims 1, 2, and 14-17, wherein said reaction mixture comprises about 1 mole of chromous chloride, about 2 moles of sodium pyrrole and tetrahydrofuran.

**19.** The process of any of claims 1, 2, and 14-17, wherein said reaction mixture comprises about 1 mole of chromous chloride, an excess of sodium pyrrole and an excess of tetrahydrofuran.

**20.** The process of any of claims 1, 2, and 14-17, wherein said reaction mixture comprises about 1 mole of chromic chloride, about 3 moles of sodium pyrrole and an excess of dimethoxyethane.

**21.** The process of any of claims 1, 2, and 14-17, wherein said reacting mixture comprises about 1 mole of chromous chloride, an excess of dimethyl pyrrole and an excess of tetrahydrofuran.

**22.** The process of any of claims 1 to 21, further comprising reacting the obtained chromium compound with at least one activating compound selected from metal alkyls and Lewis acids to obtain a catalyst.

**23.** The process of any of claims 1-21, further comprising reacting the obtained chromium-compound with at least one activating compound selecting from metal alkyls and Lewis acids, then adding an inorganic oxide as supports and recovering a solid product catalyst.

**24.** The process of any of claims 1-21, further comprising mixing the chromium compound-containing reaction mixture with an inorganic oxide to form a slurry, adding at least one activating compound selected from metal alkyls and Lewis acids, and recovering a solid product catalyst.

**25.** The process of claim 23 or 24, wherein said activating compound is dissolved in an aromatic compound, preferably toluene.

**26.** The process of any of claims 22-25, wherein said activating compound is a trialkylaluminum compound preferably triethylaluminum.

**27.** The process of any of claims 22-26, wherein said chromium compound is a chromium pyrrolide.

**28.** The process of any of claims 23-27, wherein said inorganic oxide is aluminium phosphate.

**29.** Chromium compound of the general formula I

$$\{[Cr_m(C_4H_4N)_n]\ (OC_4H_8)_p\ [Na]_q\} \qquad (I)$$

wherein m, n, p and q have the following meaning:

| m | n | p | q |
|---|---|---|---|
| 5 | 10 | 4 | 0 |
| 1 | 4 | 0 | 0 |
| 1 | 4 | 2 | 2 |
| 1 | 5 | 1 | 0 |
| 1 | 5 | 5 | 2 |

**30.** The compound of claim 29, having the formula

$$Cr_5(C_4H_4N)_{10}(OC_4H_8)_4.$$

**31.** The compound of claim 29, having the formula

$$[Cr(C_4H_4N)_4].$$

**32.** The compound of claim 29, having the formula

$$[Cr(C_4H_4N)_4][Na]_2x2(OC_4H_8).$$

**33.** The compound of claim 29, having the formula

$$[Cr(C_4H_4N)_5(OC_4H_8)].$$

**34.** The compound of claim 29, having the formula

$$[Cr(C_4H_4N)_5(OC_4H_8)][Na]_2x4(OC_4H_8).$$

**35.** A composition comprising at least one of the chromium compounds (I) of claim 29.

**36.** The composition of claim 35, wherein said chromium compound is supported on an inorganic oxide.

**37.** The composition of claim 35 or 36, further comprising an activating compound selected from metal alkyls and Lewis acids.

**38.** The composition of claim 37, wherein said metal alkyl is trialkylaluminum, preferably triethylaluminum.

**39.** The use of the chromium compounds or of the chromium compound-containing compositions as defined in any of claims 29-38 for polymerizing and/or trimerizing olefins.

**40.** The use of claim 39, wherein said olefin has from 2-30 carbon atoms.

**41.** The use of claim 40, wherein said olefin is ethylene.

**42.** The use of any of claims 39-41, wherein said polymerization and/or trimerization is carried out in the presence of an activating compound selected from metal alkyls and Lewis acids.

**Claims for the following Contracting State : ES**

**1.** A process for preparing chromium-compounds, characterized by refluxing a mixture comprising a chromium salt, a metal amide and an electron pair donor solvent.

**2.** The process of claim 1, wherein refluxing occurs during a period of time from 1 minute to 1 week.

**3.** The process of claim 1 or 2, wherein said chromium salt is selected from chromium adds, chromium halides, and mixtures thereof.

**4.** The process of claim 3, wherein said chromium halide is selected from chromous chloride, chromic chloride, and mixtures thereof.

5. The process of any of claims 1-4 wherein said metal amide is selected from alkali metal amide salts, alkaline earth metal amide salts, silyl amide salts, and mixtures thereof.

6. The process of any of claims 1-5, wherein said metal amide has from 1 to 20 carbon atoms.

7. The process of any of claims 1-6, wherein said metal amide is an unsaturated compound

8. The process of any of claims 1-7, wherein said metal amide is an aromatic compound.

9. The process of claim 1, wherein said metal amide has at least one amine ligand being selected from pyrrole ligands, derivatives of pyrrole ligands, and mixtures thereof.

10. The process of any of claims 1-9, wherein said metal amide is soluble in said electron pair donor solvent.

11. The process of any of claims 1-10, wherein said electron pair donor solvent has from 2-20 carbon atoms.

12. The process of any of claims 1-11, wherein said electron pair donor solvent is an aliphatic compound.

13. The process of any of claims 1-12, wherein said electron pair donor solvent is an ether selected from tetrahydrofuran, derivatives of tetrahydrofuran, dimethoxyethane, derivatives of dimethoxyethane, and mixtures thereof.

14. The process of any of claims 1 to 13, wherein, upon completion of the reaction, the liquid is filtered to remove any reaction by-products.

15. The process of claim 14, wherein the thus-filtered liquid is treated to form a solid.

16. The process of claim 15, wherein the thus-filtered liquid is slowly evaporated.

17. The process of claim 15, wherein the thus-filtered is treated with a non-polar solvent.

18. The process of any of claims 1, 2, and 14-17, wherein said reaction mixture comprises about 1 mole of chromous chloride, about 2 moles of sodium pyrrole and tetrahydrofuran.

19. The process of any of claims 1,2, and 14-17, wherein said reaction mixture comprises about 1 mole of chromous chloride, an excess of sodium pyrrole and an excess of tetrahydrohran.

20. The process of any of claims 1,2, and 14-17, wherein said reaction mixture comprises about 1 mole of chromic chloride, about 3 moles of sodium pyrrole and an excess of dimethoxyethane.

21. The process of any of claims 1,2, and 14-17, wherein said reacting mixture comprises about 1 mole of chromous chloride, an excess of dimethyl pyrrole and an excess of tetrahydrofuran.

22. The process of any of claims 1 to 21, further comprising reacting the obtained chromium compound with at least one activating compound selected from metal alkyls and Lewis acids to obtain a catalyst.

23. The process of any of claims 1-21, further comprising reacting the obtained chromium-compound with at least one activating compound selecting from metal alkyls and Lewis acids, then adding an inorganic oxide as support, and recovering a solid product catalyst.

24. The process of any of claims 1-21, further comprising mixing the chromium compound-containing reaction mixture with an inorganic oxide to form a slurry, adding at least one activating compound selected from metal alkyls and Lewis acids, and recovering a solid product catalyst.

25. The process of claim 23 or 24, wherein said activating compound is dissolved in an aromatic compound, preferably toluene.

26. The process of any of claims 22-25, wherein said activating compound is a trialkylaluminum compound, preferably triethylaluminum.

**27.** The process of any of claims 22-26, wherein said chromium compound is a chromium pyrrolide.

**28.** The process of any of claims 23-27, wherein said inorganic oxide is aluminium phosphate.

**29.** Chromium compound of the general formula I

$$\{[Cr_m\ (C_4H_4N)_n]\ (OC_4H_8)_p\ [Na]_q\} \qquad (I)$$

wherein m, n, p and q have the following meaning:

| m | n | p | q |
|---|---|---|---|
| 5 | 10 | 4 | 0 |
| 1 | 4 | 0 | 0 |
| 1 | 4 | 2 | 2 |
| 1 | 5 | 1 | 0 |
| 1 | 5 | 5 | 2 |

**30.** The compound of claim 29, having the formula

$$Cr_5(C_4H_4N)_{10}(OC_4H_8)_4.$$

**31.** The compound of claim 29, having the formula

$$[Cr(C_4H_4N)_4].$$

**32.** The compound of claim 29, having the formula

$$[Cr(C_4H_4N)_4[Na]_2x2(OC_4H_8).$$

**33.** The compound of claim 29, having the formula

$$[Cr(C_4H_4N)_5(OC_4H_8)].$$

**34.** The compound of claim 29, having the formula

$$[Cr(C_4H_4N)_5(OC_4H_8)][Na]_2x4(OC_4H_8).$$

**35.** A composition comprising at least one of the chromium compounds (I) of claim 29.

**36.** The composition of claim 35, wherein said chromium compound is supported on an inorganic oxide.

**37.** The composition of claim 35 or 36, further comprising an activating compound selected from metal alkyls and Lewis acids.

**38.** The composition of claim 37, wherein said metal alkyl is trialkylaluminum preferably triethylaluminum.

**39.** The use of the chromium compounds or of the chromium compound-containing compositions as defined in any of claims 29-38 for polymerizing and/or trimerizing olefins.

**40.** The use of claim 39, wherein said olefin has from 2-30 carbon atoms.

**41.** The use of claim 40, wherein said olefin is ethylene.

**42.** The use of any of claims 39-41, wherein said polymerization and/or trimerization is carried out in the presence of an activating compound selected from metal alkyls and Lewis acids.

**43.** The process of any of claims 1 - 28, wherein $CrCl_2$ and sodium pyrrolide ($NaC_4H_4N$) in a molar ratio of 1:2 are reacted in refluxing tetrahydrofuran (THF) to obtain $Cr_5(C_4H_4N)_{10}(C_4H_8O)_4$.

**44.** The process of any of claims 1 - 28, wherein $CrCl_2$ and sodium pyrrolide are reacted in refluxing THF to obtain $[Cr(C_4H_4N)_4][Na]_2 \cdot 2\ C_4H_8O$ and $[Cr(C_4H_4N)_5(C_4H_8O)][Na]_2 \cdot 4\ C_4H_8O$, the sodium pyrrolide being used in a molar excess over $CrCl_2$.

**45.** The process of any of claims 1 - 28, wherein $CrCl_3$ and sodium pyrrolide in a molar ratio of 1 : 3 are reacted in refluxing dimethoxyethane (DME).

**46.** The process of any of claims 1 - 28, wherein $CrCl_2$ and 2,5-dimethylpyrrolide in a molar ratio of 1 : 1.99 are reacted in refluxing THF.

**47.** A process for preparing the composition of any of claims 35 - 38, wherein at least one of the chromium compounds of claim 29, obtainable according to the process of any of claims 1 - 28 and 43 - 46 is mixed with a metal alkyl such as trimethylaluminum.

**Patentansprüche**

**Patentansprüche für folgende Vertragsstaaten : AT, BE, CH, LI, DE, DK, FR, GB, GR, IT, LU, NL, SE**

**1.** Verfahren zur Herstellung von Chromverbindungen, gekennzeichnet durch Rückflußkochen eines Gemisches, das ein Chromsalz, ein Metallamid und ein Elektronenpaardonator-Lösungsmittel enthält.

**2.** Verfahren nach Anspruch 1, wobei das Rückflußkochen für eine Zeitspanne von 1 Minute bis 1 Woche erfolgt.

**3.** Verfahren nach Anspruch 1 oder 2, wobei das Chromsalz unter Chromsäuren, Chromhalogeniden und Gemischen davon ausgewählt wird.

**4.** Verfahren nach Anspruch 3, wobei das Chromhalogenid unter Chrom(II)-chlorid, Chrom(III)-chlorid und Gemischen davon ausgewählt wird.

**5.** Verfahren nach einem der Ansprüche 1 bis 4, wobei das Metallamid unter Alkalimetallamidsalzen, Erdalkalimetallamidsalzen, Silylamidsalzen und Gemischen davon ausgewählt wird.

**6.** Verfahren nach einem der Ansprüche 1 bis 5, wobei das Metallamid 1 bis 20 Kohlenstoffatome aufweist.

**7.** Verfahren nach einem der Ansprüche 1 bis 6, wobei es sich beim Metallamid um eine ungesättigte Verbindung handelt.

**8.** Verfahren nach einem der Ansprüche 1 bis 7, wobei es sich beim Metallamid um eine aromatische Verbindung handelt.

**9.** Verfahren nach Anspruch 1, wobei das Metallamid mindestens einen Aminliganden, der unter Pyrrolliganden, Derivaten von Pyrrolliganden und Gemischen davon ausgewählt ist, aufweist.

**10.** Verfahren nach einem der Ansprüche 1 bis 9, wobei das Metallamid im Elektronenpaardonator-Lösungsmittel löslich ist.

**11.** Verfahren nach einem der Ansprüche 1 bis 10, wobei das Elektronenpaardonator-Lösungsmittel 2 bis 20 Kohlenstoffatome aufweist.

**12.** Verfahren nach einem der Ansprüche 1 bis 11, wobei es sich beim Elektronenpaardonator-Lösungsmittel um eine aliphatische Verbindung handelt.

**13.** Verfahren nach einem der Ansprüche 1 bis 12, wobei es sich beim Elektronenpaardonator-Lösungsmittel um einen Ether, der unter Tetrahydrofuran, Derivaten von Tetrahydrofuran, Dimethoxyethan, Derivaten von Dimethoxyethan und Gemischen davon ausgewählt ist, handelt.

**14.** Verfahren nach einem der Ansprüche 1 bis 13, wobei nach Beendigung der Umsetzung die Flüssigkeit zur Entfernung von etwaigen Reaktionsnebenprodukten filtriert wird.

**15.** Verfahren nach Anspruch 14, wobei die auf diese Weise filtrierte Flüssigkeit zur Bildung eines Feststoffs behandelt wird.

**16.** Verfahren nach Anspruch 15, wobei die auf diese Weise filtrierte Flüssigkeit langsam eingedampft wird.

**17.** Verfahren nach Anspruch 15, wobei die auf diese Weise filtrierte Flüssigkeit mit einem nicht-polaren Lösungsmittel behandelt wird.

**18.** Verfahren nach einem der Ansprüche 1, 2 und 14-17, wobei das Reaktionsgemisch etwa 1 Mol Chrom(II)-chlorid, etwa 2 Mol Natriumpyrrol und Tetrahydrofuran enthält.

**19.** Verfahren nach einem der Ansprüche 1, 2 und 14-17, wobei das Reaktionsgemisch etwa 1 Mol Chrom(II)-chlorid, einen Überschuß an Natriumpyrrol und einen Überschuß an Tetrahydrofuran enthält.

**20.** Verfahren nach einem der Ansprüche 1, 2 und 14-17, wobei das Reaktionsgemisch etwa 1 Mol Chrom(III)-chlorid, etwa 3 Mol Natriumpyrrol und einen Überschuß an Dimethoxyethan enthält.

**21.** Verfahren nach einem der Ansprüche 1, 2 und 14-17, wobei das Reaktionsgemisch etwa 1 Mol Chrom(II)-chlorid, einen Überschuß an Dimethylpyrrol und einen Überschuß an Tetrahydrofuran enthält.

**22.** Verfahren nach einem der Ansprüche 1 bis 21, ferner umfassend die Umsetzung der erhaltenen Chromverbindung mit mindestens einer aktivierenden Verbindung, die unter Metallalkylen und Lewis-Säuren ausgewählt ist, unter Bildung eines Katalysators.

**23.** Verfahren nach Anspruch 1 bis 21, ferner umfassend die Umsetzung der erhaltenen Chromverbindung mit mindestens einer aktivierenden Verbindung, die unter Metallalkylen und Lewis-Säuren ausgewählt ist, die anschließende Zugabe eines anorganischen Oxids als Träger und die Gewinnung eines festen Katalysatorprodukts.

**24.** Verfahren nach einem der Ansprüche 1 bis 21, ferner umfassend das Vermischen des die Chromverbindung enthaltenden Reaktionsgemisches mit einem anorganischen Oxid unter Bildung einer Aufschlämmung, die Zugabe von mindestens einer aktivierenden Verbindung, die unter Metallalkylen und Lewis-Säuren ausgewählt ist, und die Gewinnung eines festen Katalysatorprodukts.

**25.** Verfahren nach Anspruch 23 oder 24, wobei die aktivierende Verbindung in einer aromatischen Verbindung, vorzugsweise Toluol, gelöst wird.

**26.** Verfahren nach einem der Ansprüche 22 bis 25, wobei es sich bei der aktivierenden Verbindung um eine Trialkylaluminiumverbindung und insbesondere um Triethylaluminium handelt.

**27.** Verfahren nach einem der Ansprüche 22 bis 26, wobei es sich bei der Chromverbindung um ein Chrompyrrolid handelt.

**28.** Verfahren nach einem der Ansprüche 23 bis 27, wobei es sich beim anorganischen Oxid um Aluminiumphosphat handelt.

**29.** Chromverbindung der allgemeinen Formel I

$$\{[Cr_m(C_4H_4N)_n]\ (OC_4H_8)_p[Na]_q\} \qquad (I)$$

worin m, n, p und q die folgenden Bedeutungen haben:

| m | n | p | q |
|---|---|---|---|
| 5 | 10 | 4 | 0 |
| 1 | 4 | 0 | 0 |
| 1 | 4 | 2 | 2 |
| 1 | 5 | 1 | 0 |
| 1 | 5 | 5 | 2 |

**30.** Verbindung nach Anspruch 29 der Formel

$$Cr_5(C_4H_4N)_{10}(OC_4H_8)_4.$$

**31.** Verbindung nach Anspruch 29 der Formel

$$[Cr(C_4H_4N)_4].$$

**32.** Verbindung nach Anspruch 29 der Formel

$$[Cr(C_4H_4N)_4]\ [Na]_2x2\ (OC_4H_8).$$

**33.** Verbindung nach Anspruch 29 der Formel

$$[Cr(C_4H_4N)_5(OC_4H_8)].$$

**34.** Verbindung nach Anspruch 29 der Formel

$$[Cr(C_4H_4N)_5(OC_4H_8)]\ [Na]_2x4\ (OC_4H_8).$$

**35.** Zusammensetzung, enthaltend mindestens eine der Chromverbindungen (I) nach Anspruch 29.

**36.** Zusammensetzung nach Anspruch 35, wobei die Chromverbindung auf ein anorganisches Oxid als Träger aufgebracht ist.

**37.** Zusammensetzung nach Anspruch 35 oder 36, ferner umfassend eine aktivierende Verbindung, die unter Metallalkylen und Lewis-Säuren ausgewählt ist.

**38.** Zusammensetzung nach Anspruch 37, wobei es sich beim Metallalkyl um Trialkylaluminium und vorzugsweise um Triethylaluminium handelt.

**39.** Verwendung der Chromverbindungen oder der die Chromverbindungen enthaltenden Zusammensetzungen nach einem der Ansprüche 29 bis 38 zur Polymerisation und/oder Trimerisation von Olefinen.

**40.** Verwendung nach Anspruch 39, wobei das Olefin 2 bis 30 Kohlenstoffatome aufweist.

**41.** Verwendung nach Anspruch 40, wobei es sich beim Olefin um Ethylen handelt.

**42.** Verwendung nach einem der Ansprüche 39 bis 41, wobei die Polymersisation und/oder die Trimerisation in Gegenwart einer aktivierenden Verbindung, die unter Metallalkylen und Lewis-Säuren ausgewählt ist, durchgeführt wird.

**Patentansprüche für folgenden Vertragsstaat : ES**

1. Verfahren zur Herstellung von Chromverbindungen, gekennzeichnet durch Rückflußkochen eines Gemisches, das ein Chromsalz, ein Metallamid und ein Elektronenpaardonator-Lösungsmittel enthält.

2. Verfahren nach Anspruch 1, wobei das Rückflußkochen für eine Zeitspanne von 1 Minute bis 1 Woche erfolgt.

3. Verfahren nach Anspruch 1 oder 2, wobei das Chromsalz unter Chromsäuren, Chromhalogeniden und Gemischen davon ausgewählt wird.

4. Verfahren nach Anspruch 3, wobei das Chromhalogenid unter Chrom(II)-chlorid, Chrom(III)-chlorid und Gemischen davon ausgewählt wird.

5. Verfahren nach einem der Ansprüche 1 bis 4, wobei das Metallamid unter Alkalimetallamidsalzen, Erdalkalimetallamidsalzen, Silylamidsalzen und Gemischen davon ausgewählt wird.

6. Verfahren nach einem der Ansprüche 1 bis 5, wobei das Metallamid 1 bis 20 Kohlenstoffatome aufweist.

7. Verfahren nach einem der Ansprüche 1 bis 6, wobei es sich beim Metallamid um eine ungesättigte Verbindung handelt.

8. Verfahren nach einem der Ansprüche 1 bis 7, wobei es sich beim Metallamid um eine aromatische Verbindung handelt.

9. Verfahren nach Anspruch 1, wobei das Metallamid mindestens einen Aminliganden, der unter Pyrrolliganden, Derivaten von Pyrrolliganden und Gemischen davon ausgewählt ist, aufweist.

10. Verfahren nach einem der Ansprüche 1 bis 9, wobei das Metallamid im Elektronenpaardonator-Lösungsmittel löslich ist.

11. Verfahren nach einem der Ansprüche 1 bis 10, wobei das Elektronenpaardonator-Lösungsmittel 2 bis 20 Kohlenstoffatome aufweist.

12. Verfahren nach einem der Ansprüche 1 bis 11, wobei es sich beim Elektronenpaardonator-Lösungsmittel um eine aliphatische Verbindung handelt.

13. Verfahren nach einem der Ansprüche 1 bis 12, wobei es sich beim Elektronenpaardonator-Lösungsmittel um einen Ether, der unter Tetrahydrofuran, Derivaten von Tetrahydrofuran, Dimethoxyethan, Derivaten von Dimethoxyethan und Gemischen davon ausgewählt ist, handelt.

14. Verfahren nach einem der Ansprüche 1 bis 13, wobei nach Beendigung der Umsetzung die Flüssigkeit zur Entfernung von etwaigen Reaktionsnebenprodukten filtriert wird.

15. Verfahren nach Anspruch 14, wobei die auf diese Weise filtrierte Flüssigkeit zur Bildung eines Feststoffs behandelt wird.

16. Verfahren nach Anspruch 15, wobei die auf diese Weise filtrierte Flüssigkeit langsam eingedampft wird.

17. Verfahren nach Anspruch 15, wobei die auf diese Weise filtrierte Flüssigkeit mit einem nicht-polaren Lösungsmittel behandelt wird.

18. Verfahren nach einem der Ansprüche 1, 2 und 14-17, wobei das Reaktionsgemisch etwa 1 Mol Chrom(II)-chlorid, etwa 2 Mol Natriumpyrrol und Tetrahydrofuran enthält.

**19.** Verfahren nach einem der Ansprüche 1, 2 und 14-17, wobei das Reaktionsgemisch etwa 1 Mol Chrom(II)-chlorid, einen Überschuß an Natriumpyrrol und einen Überschuß an Tetrahydrofuran enthält.

**20.** Verfahren nach einem der Ansprüche 1, 2 und 14-17, wobei das Reaktionsgemisch etwa 1 Mol Chrom(III)-chlorid, etwa 3 Mol Natriumpyrrol und einen Überschuß an Dimethoxyethan enthält.

**21.** Verfahren nach einem der Ansprüche 1, 2 und 14-17, wobei das Reaktionsgemisch etwa 1 Mol Chrom(II)-chlorid, einen Überschuß an Dimethylpyrrol und einen Überschuß an Tetrahydrofuran enthält.

**22.** Verfahren nach einem der Ansprüche 1 bis 21, ferner umfassend die Umsetzung der erhaltenen Chromverbindung mit mindestens einer aktivierenden Verbindung, die unter Metallalkylen und Lewis-Säuren ausgewählt ist, unter Bildung eines Katalysators.

**23.** Verfahren nach Anspruch 1 bis 21, ferner umfassend die Umsetzung der erhaltenen Chromverbindung mit mindestens einer aktivierenden Verbindung, die unter Metallalkylen und Lewis-Säuren ausgewählt ist, die anschließende Zugabe eines anorganischen Oxids als Träger und die Gewinnung eines festen Katalysatorprodukts.

**24.** Verfahren nach einem der Ansprüche 1 bis 21, ferner umfassend das Vermischen des die Chromverbindung enthaltenden Reaktionsgemisches mit einem anorganischen Oxid unter Bildung einer Aufschlämmung, die Zugabe von mindestens einer aktivierenden Verbindung, die unter Metallalkylen und Lewis-Säuren ausgewählt ist, und die Gewinnung eines festen Katalysatorprodukts.

**25.** Verfahren nach Anspruch 23 oder 24, wobei die aktivierende Verbindung in einer aromatischen Verbindung, vorzugsweise Toluol, gelöst wird.

**26.** Verfahren nach einem der Ansprüche 22 bis 25, wobei es sich bei der aktivierenden Verbindung um eine Trialkylaluminiumverbindung und insbesondere um Triethylaluminium handelt.

**27.** Verfahren nach einem der Ansprüche 22 bis 26, wobei es sich bei der Chromverbindung um ein Chrompyrrolid handelt.

**28.** Verfahren nach einem der Ansprüche 23 bis 27, wobei es sich beim anorganischen Oxid um Aluminiumphosphat handelt.

**29.** Chromverbindung der allgemeinen Formel I

$$\{[Cr_m(C_4H_4N)_n]\,(OC_4H_8)_p\,[Na]_q\} \qquad (I)$$

worin m, n, p und q die folgenden Bedeutungen haben:

| m | n | p | q |
|---|---|---|---|
| 5 | 10 | 4 | 0 |
| 1 | 4 | 0 | 0 |
| 1 | 4 | 2 | 2 |
| 1 | 5 | 1 | 0 |
| 1 | 5 | 5 | 2 |

**30.** Verbindung nach Anspruch 29 der Formel

$$Cr_5(C_4H_4N)_{10}(OC_4H_8)_4.$$

**31.** Verbindung nach Anspruch 29 der Formel

$$[Cr(C_4H_4N)_4].$$

**32.** Verbindung nach Anspruch 29 der Formel

$$[Cr(C_4H_4N)_4]\ [Na]_2x2\ (OC_4H_8).$$

**33.** Verbindung nach Anspruch 29 der Formel

$$[Cr(C_4H_4N)_5(OC_4H_8)].$$

**34.** Verbindung nach Anspruch 29 der Formel

$$[Cr(C_4H_4N)_5(OC_4H_8)][Na]_2x4(OC_4H_8).$$

**35.** Zusammensetzung, enthaltend mindestens eine der Chromverbindungen (I) nach Anspruch 29.

**36.** Zusammensetzung nach Anspruch 35, wobei die Chromverbindung auf ein anorganisches Oxid als Träger aufgebracht ist.

**37.** Zusammensetzung nach Anspruch 35 oder 36, ferner umfassend eine aktivierende Verbindung, die unter Metallalkylen und Lewis-Säuren ausgewählt ist.

**38.** Zusammensetzung nach Anspruch 37, wobei es sich beim Metallalkyl um Trialkylaluminium und vorzugsweise um Triethylaluminium handelt.

**39.** Verwendung der Chromverbindungen oder der die Chromverbindungen enthaltenden Zusammensetzungen nach einem der Ansprüche 29 bis 38 zur Polymerisation und/oder Trimerisation von Olefinen.

**40.** Verwendung nach Anspruch 39, wobei das Olefin 2 bis 30 Kohlenstoffatome aufweist.

**41.** Verwendung nach Anspruch 40, wobei es sich beim Olefin um Ethylen handelt.

**42.** Verwendung nach einem der Ansprüche 39 bis 41, wobei die Polymersisation und/oder die Trimerisation in Gegenwart einer aktivierenden Verbindung, die unter Metallalkylen und Lewis-Säuren ausgewählt ist, durchgeführt wird.

**43.** Verfahren nach einem der Ansprüche 1 bis 28, wobei $CrCl_2$ und Natriumpyrrolid ($NaC_4H_4N$) in einem Molverhältnis von 1:2 in unter Rückfluß siedendem Tetrahydrofuran (THF) unter Bildung von $Cr_5(C_4H_4N)_{10}(C_4H_8O)_4$ umgesetzt werden.

**44.** Verfahren nach einem der Ansprüche 1 bis 28, wobei $CrCl_2$ und Natriumpyrrolid in unter Rückfluß siedendem THF unter Bildung von $[Cr(C_4H_4N)_4][Na]_2 \cdot 2\ C_4H_8O$ und $[Cr(C_4H_4N)_5(C_4H_8O)][Na]_2 \cdot 4\ C_4H_8O$ umgesetzt werden, wobei das Natriumpyrrolid in molarem Überschuß gegenüber $CrCl_2$ eingesetzt wird.

**45.** Verfahren nach einem der Ansprüche 1 bis 28, wobei $CrCl_3$ und Natriumpyrrolid in einem Molverhältnis von 1:3 in unter Rückfluß siedendem Dimethoxyethan (DME) umgesetzt werden.

**46.** Verfahren nach einem der Ansprüche 1 bis 28, wobei $CrCl_2$ und 2,5-Dimethylpyrrolid in einem Molverhältnis von 1:2 in unter Rückfluß siedendem (THF) umgesetzt werden.

**47.** Verfahren zur Herstellung der Zusammensetzung nach einem der Ansprüche 35 bis 38, wobei mindestens eine der Chromverbindungen von Anspruch 29, die nach dem Verfahren gemäß einem der Ansprüche 1 bis 28 und 43 bis 46 erhältlich ist, mit einem Metallalkyl, wie Trimethylaluminium, vermischt wird.

**Revendications**

**Revendications pour les Etats contractants suivants : AT, BE, CH, LI, DE, DK, FR, GB, GR, IT, LU, NL, SE**

1. Un procédé pour préparer des composés de chrome caractérisé par le chauffage au reflux d'un mélange comprenant un sel de chrome, un amide de métal et un solvant donneur à paire d'électrons.

2. Le procédé selon la revendication 1, dans lequel le chauffage au reflux a lieu pendant une période de temps de 1 minute à 1 semaine.

3. Le procédé selon la revendication 1 ou 2, dans lequel ledit sel de chrome est choisi parmi les acides du chrome, les halogénures du chrome et leurs mélanges.

4. Le procédé selon la revendication 3, dans lequel ledit halogénure de chrome est choisi parmi le chlorure chromeux, le chlorure chromique et leurs mélanges.

5. Le procédé selon l'une quelconque des revendications 1 à 4, dans lequel ledit amide de métal est choisi parmi les sels d'amide de métaux alcalins, les sels d'amide de métaux alcalino-terreux, les sels d'amide de silyle et leurs mélanges.

6. Le procédé selon l'une quelconque des revendications 1 à 5, dans lequel ledit amide de métal renferme de 1 à 20 atomes de carbone.

7. Le procédé selon l'une quelconque des revendications 1 à 6, dans lequel ledit amide de métal est un composé insaturé.

8. Le procédé selon l'une quelconque des revendications 1 à 7, dans lequel ledit amide de métal est un composé aromatique.

9. Le procédé selon la revendication 1, dans lequel ledit amide de métal renferme au moins un ligand amine choisi à partir des ligands pyrrole, des dérivés de ligands pyrrole et leurs mélanges.

10. Le procédé selon l'une quelconque des revendications 1 à 9, dans lequel ledit amide de métal est soluble dans ledit solvant donneur à paire d'électrons.

11. Le procédé selon l'une quelconque des revendications 1 à 10, dans lequel ledit solvant donneur à paire d'électrons renferme de 2 à 20 atomes de carbone.

12. Le procédé selon l'une quelconque des revendications 1 à 11, dans lequel ledit solvant donneur à paire d'électrons est un composé aliphatique.

13. Le procédé selon l'une quelconque des revendications 1 à 12, dans lequel ledit solvant donneur à paire d'électrons est un éther choisi parmi le tétrahydrofuranne, les dérivés de tétrahydrofuranne, le diméthoxyéthane, les dérivés de diméthoxyéthane et leurs mélanges.

14. Le procédé selon l'une quelconque des revendications 1 à 13, dans lequel à la fin de la réaction, le liquide est filtré pour éliminer tous les sous-produits de réaction.

15. Le procédé selon la revendication 14, dans lequel le liquide ainsi filtré est traité pour former un solide.

16. Le procédé selon la revendication 15, dans lequel le liquide ainsi filtré est évaporé lentement.

17. Le procédé selon la revendication 15, dans lequel le liquide ainsi filtré est traité avec un solvant non polaire.

18. Le procédé selon l'une quelconque des revendications 1, 2 et 14 à 17, dans lequel ledit mélange de réaction comprend environ 1 mole de chlorure chromeux, environ 2 moles de pyrrole sodique et de tétrahydrofuranne.

**19.** Le procédé selon l'une quelconque des revendications 1, 2 et 14 à 17, dans lequel ledit mélange de réaction comprend environ 1 mole de chlorure chromeux, un excès de pyrrole sodique et un excès de tétrahydrofuranne.

**20.** Le procédé selon l'une quelconque des revendications 1, 2 et 14 à 17, dans lequel ledit mélange de réaction comprend environ 1 mole de chlorure chromique, environ 3 moles de pyrrole sodique et un excès de diméthoxyéthane.

**21.** Le procédé selon l'une quelconque des revendications 1, 2 et 14 à 17, dans lequel ledit mélange de réaction comprend environ 1 mole de chlorure chromeux, un excès de diméthylpyrrole et un excès de tétrahydrofuranne.

**22.** Le procédé selon l'une quelconque des revendications 1 à 21, comprenant, en outre la réaction du composé de chrome obtenu avec au moins un composé d'activation choisi parmi les alkyl-métaux et les acides de Lewis pour obtenir un catalyseur.

**23.** Le procédé selon l'une quelconque des revendications 1 à 21, comprenant, en outre la réaction du composé de chrome obtenu avec au moins un composé d'activation choisi parmi les alkyl-métaux et les acides de Lewis et l'addition ensuite d'un oxyde non organique comme support et la récupération d'un catalyseur solide.

**24.** Le procédé selon l'une quelconque des revendications 1 à 21, comprenant, en outre, le mélange du mélange de réaction contenant le composé de chrome avec un oxyde non organique pour former une suspension, l'addition d'au moins un composé d'activation choisi parmi les alkyl-métaux et les acides de Lewis et la récupération d'un catalyseur solide.

**25.** Le procédé selon la revendication 23 ou 24, dans lequel ledit composé d'activation est dissous dans un composé aromatique, de préférence, le toluène.

**26.** Le procédé selon l'une quelconque des revendications 22 à 25, dans lequel ledit composé d'activation est un composé de trialkylaluminium, de préférence le triéthylaluminium.

**27.** Le procédé selon l'une quelconque des revendications 22 à 26, dans lequel ledit composé de chrome est un pyrrylchrome.

**28.** Le procédé selon l'une quelconque des revendications 23 à 27, dans lequel ledit oxyde non organique est du phosphate d'aluminium.

**29.** Composé de chrome de formule générale (I) :

$$\{[Cr_m(C_4H_4N)_n](OC_4H_8)_p[Na]_q\} \quad (I)$$

dans laquelle m, n, p et q ont la significations suivante :

| m | n | p | q |
|---|---|---|---|
| 5 | 10 | 4 | 0 |
| 1 | 4 | 0 | 0 |
| 1 | 4 | 2 | 2 |
| 1 | 5 | 1 | 0 |
| 1 | 5 | 5 | 2 |

**30.** Le composé selon la revendication 29 répondant à la formule :

$$Cr_5(C_4H_4N)_{10}(OC_4H_8)_4$$

**31.** Le composé selon la revendication 29 répondant à la formule :

$$[Cr(C_4H_4N)_4]$$

**32.** Le composé selon la revendication 29 répondant à la formule :

$$[Cr(C_4H_4N)_4][Na]_2x2(OC_4H_8)$$

**33.** Le composé selon la revendication 29 répondant à la formule :

$$[Cr(C_4H_4N)_5(OC_4H_8)]$$

**34.** Le composé selon la revendication 29 répondant à la formule :

$$[Cr(C_4H_4N)_5(OC_4H_8)][Na]_2x4(OC_4H_8)$$

**35.** Une composition comprenant au moins un des composés de chrome (I) de la revendication 29.

**36.** La composition selon la revendication 35, dans laquelle ledit composé de chrome est fixé sur un oxyde non organique.

**37.** La composition selon la revendication 35 ou 36 comprenant, en outre, un composé d'activation choisi parmi les alkyl-métaux et les acides de Lewis.

**38.** La composition selon la revendication 37, dans laquelle ledit alkyl-métal est un trialkylaluminium, de préférence le triéthylaluminium.

**39.** L'utilisation des composés de chrome ou du composé de chrome renfermant des compositions comme définies dans l'une quelconque des revendications 29 à 38 pour la polymérisation et/ou la trimérisation d'oléfines.

**40.** L'utilisation selon la revendication 39, dans laquelle ladite oléfine renferme de 2 à 30 atomes de carbone.

**41.** L'utilisation selon la revendication 40, dans laquelle ladite oléfine est l'éthylène.

**42.** L'utilisation selon l'une quelconque des revendications 39 à 41, dans laquelle ladite polymérisation et/ou trimérisation est mise en oeuvre en présence d'un composé d'activation choisi parmi les alkyl-métaux et les acides de Lewis.

**Revendications pour l'Etat contractant suivant : ES**

**1.** Un procédé pour préparer des composés de chrome caractérisé par le chauffage au reflux d'un mélange comprenant un sel de chrome, un amide de métal et un solvant donneur à paire d'électrons.

**2.** Le procédé selon la revendication 1, dans lequel le chauffage au reflux a lieu pendant une période de temps de 1 minute à 1 semaine.

**3.** Le procédé selon la revendication 1 ou 2, dans lequel ledit sel de chrome est choisi parmi les acides du chrome, les halogénures du chrome et leurs mélanges.

**4.** Le procédé selon la revendication 3, dans lequel ledit halogénure de chrome est choisi parmi le chlorure chromeux, le chlorure chromique et leurs mélanges.

**5.** Le procédé selon l'une quelconque des revendications 1 à 4, dans lequel ledit amide de métal est choisi parmi les sels d'amide de métaux alcalins, les sels d'amide de métaux alcalino-terreux, les sels d'amide de silyle et leurs mélanges.

6. Le procédé selon l'une quelconque des revendications 1 à 5, dans lequel ledit amide de métal renferme de 1 à 20 atomes de carbone.

7. Le procédé selon l'une quelconque des revendications 1 à 6, dans lequel ledit amide de métal est un composé insaturé.

8. Le procédé selon l'une quelconque des revendications 1 à 7, dans lequel ledit amide de métal est un composé aromatique.

9. Le procédé selon la revendication 1, dans lequel ledit amide de métal renferme au moins un ligand amine choisi à partir des ligands pyrrole, des dérivés de ligands pyrrole et leurs mélanges.

10. Le procédé selon l'une quelconque des revendications 1 à 9, dans lequel ledit amide de métal est soluble dans ledit solvant donneur à paire d'électrons.

11. Le procédé selon l'une quelconque des revendications 1 à 10, dans lequel ledit solvant donneur à paire d'électrons renferme de 2 à 20 atomes de carbone.

12. Le procédé selon l'une quelconque des revendications 1 à 11, dans lequel ledit solvant donneur à paire d'électrons est un composé aliphatique.

13. Le procédé selon l'une quelconque des revendications 1 à 12, dans lequel ledit solvant donneur à paire d'électrons est un éther choisi parmi le tétrahydrofuranne, les dérivés de tétrahydrofuranne, le diméthoxyéthane, les dérivés de diméthoxyéthane et leurs mélanges.

14. Le procédé selon l'une quelconque des revendications 1 à 13, dans lequel à la fin de la réaction, le liquide est filtré pour éliminer tous les sous-produits de réaction.

15. Le procédé selon la revendication 14, dans lequel le liquide ainsi filtré est traité pour former un solide.

16. Le procédé selon la revendication 15, dans lequel le liquide ainsi filtré est évaporé lentement.

17. Le procédé selon la revendication 15, dans lequel le liquide ainsi filtré est traité avec un solvant non polaire.

18. Le procédé selon l'une quelconque des revendications 1, 2 et 14 à 17, dans lequel ledit mélange de réaction comprend environ 1 mole de chlorure chromeux, environ 2 moles de pyrrole sodique et de tétrahydrofuranne.

19. Le procédé selon l'une quelconque des revendications 1, 2 et 14 à 17, dans lequel ledit mélange de réaction comprend environ 1 mole de chlorure chromeux, un excès de pyrrole sodique et un excès de tétrahydrofuranne.

20. Le procédé selon l'une quelconque des revendications 1, 2 et 14 à 17, dans lequel ledit mélange de réaction comprend environ 1 mole de chlorure chromique, environ 3 moles de pyrrole sodique et un excès de diméthoxyéthane.

21. Le procédé selon l'une quelconque des revendications 1, 2 et 14 à 17, dans lequel ledit mélange de réaction comprend environ 1 mole de chlorure chromeux, un excès de diméthylpyrrole et un excès de tétrahydrofuranne.

22. Le procédé selon l'une quelconque des revendications 1 à 21, comprenant, en outre la réaction du composé de chrome obtenu avec au moins un composé d'activation choisi parmi les alkyl-métaux et les acides de Lewis pour obtenir un catalyseur.

23. Le procédé selon l'une quelconque des revendications 1 à 21, comprenant, en outre la réaction du composé de chrome obtenu avec au moins un composé d'activation choisi parmi les alkyl-métaux et les acides de Lewis et l'addition ensuite d'un oxyde non organique comme support et la récupération d'un catalyseur solide.

24. Le procédé selon l'une quelconque des revendications 1 à 21, comprenant, en outre, le mélange du mélange de réaction contenant le composé de chrome avec un oxyde non organique pour former une suspension, l'addition d'au moins un composé d'activation choisi parmi les alkyl-métaux et les acides de Lewis et la récupération d'un

catalyseur solide.

**25.** Le procédé selon la revendication 23 ou 24, dans lequel ledit composé d'activation est dissous dans un composé aromatique, de préférence, le toluène.

**26.** Le procédé selon l'une quelconque des revendications 22 à 25, dans lequel ledit composé d'activation est un composé de trialkylaluminium, de préférence le triéthylaluminium.

**27.** Le procédé selon l'une quelconque des revendications 22 à 26, dans lequel ledit composé de chrome est un pyrryl-chrome.

**28.** Le procédé selon l'une quelconque des revendications 23 à 27, dans lequel ledit oxyde non organique est du phosphate d'aluminium.

**29.** Composé de chrome de formule générale (I) :

$$\{[Cr_m(C_4H_4N)_n](OC_4H_8)_p[Na]_q\} \quad (I)$$

dans laquelle m, n, p et q ont la significations suivante :

| m | n | p | q |
|---|---|---|---|
| 5 | 10 | 4 | 0 |
| 1 | 4 | 0 | 0 |
| 1 | 4 | 2 | 2 |
| 1 | 5 | 1 | 0 |
| 1 | 5 | 5 | 2 |

**30.** Le composé selon la revendication 29 répondant à la formule :

$$Cr_5(C_4H_4N)_{10}(OC_4H_8)_4$$

**31.** Le composé selon la revendication 29 répondant à la formule :

$$[Cr(C_4H_4N)_4]$$

**32.** Le composé selon la revendication 29 répondant à la formule :

$$[Cr(C_4H_4N)_4][Na]_2x2(OC_4H_8)$$

**33.** Le composé selon la revendication 29 répondant à la formule :

$$[Cr(C_4H_4N)_5(OC_4H_8)]$$

**34.** Le composé selon la revendication 29 répondant à la formule :

$$[Cr(C_4H_4N)_5(OC_4H_8)][Na]_2x4(OC_4H_8)$$

**35.** Une composition comprenant au moins un des composés de chrome (I) de la revendication 29.

**36.** La composition selon la revendication 35, dans laquelle ledit composé de chrome est fixé sur un oxyde non organique.

**37.** La composition selon la revendication 35 ou 36 comprenant, en outre, un composé d'activation choisi parmi les alkyl-métaux et les acides de Lewis.

**38.** La composition selon la revendication 37, dans laquelle ledit alkyl-métal est un trialkylaluminium, de préférence le triéthylaluminium.

**39.** L'utilisation des composés de chrome ou du composé de chrome renfermant des compositions comme définies dans l'une quelconque des revendications 29 à 38 pour la polymérisation et/ou la trimérisation d'oléfines.

**40.** L'utilisation selon la revendication 39, dans laquelle ladite oléfine renferme de 2 à 30 atomes de carbone.

**41.** L'utilisation selon la revendication 40, dans laquelle ladite oléfine est l'éthylène.

**42.** L'utilisation selon l'une quelconque des revendications 39 à 41, dans laquelle ladite polymérisation et/ou trimérisation est mise en oeuvre en présence d'un composé d'activation choisi parmi les alkyl-métaux et les acides de Lewis.

**43.** Le procédé selon l'une quelconque des revendications 1 à 28, dans lequel on fait réagir dans du tétrahydrofuranne au reflux (THF) du $CrCl_2$ et du pyrryl-sodium ($NaC_4H_4N$) dans un rapport molaire de 1:2 pour obtenir $Cr_5(C_4H_4N)_{10}(C_4H_8O)_4$.

**44.** Le procédé selon l'une quelconque des revendications 1 à 28, dans lequel on fait réagir $CrCl_2$ et du pyrryl-sodium dans du THF au reflux pour obtenir $[Cr(C_4H_4N)_4]$ $[Na]_2.2C_4H_8O$ et $[Cr(C_4H_4N)_5(C_4H_8O)][Na]_2.4C_4H_8O$, le pyrryl-sodium étant utilisé selon un excès molaire par rapport à $CrCl_2$.

**45.** Le procédé selon l'une quelconque des revendications 1 à 28, dans lequel on fait réagir dans du diméthoxyéthane (DME) au reflux du $CrCl_3$ et du pyrryl-sodium dans un rapport molaire de 1:3.

**46.** Le procédé selon l'une quelconque des revendications 1 à 28, dans lequel on fait réagir dans du THF au reflux $CrCl_2$ et du 2,5-diméthylpyrryle dans un rapport molaire de 1:2.

**47.** Un procédé pour préparer la composition selon l'une quelconque des revendications 35 à 38, dans lequel au moins l'un des composés de chrome de la revendication 29, pouvant être obtenu selon le procédé de l'une quelconque des revendications 1 à 28 et 43 à 46 est mélangé avec un alkyl-métal comme le triméthylaluminium.

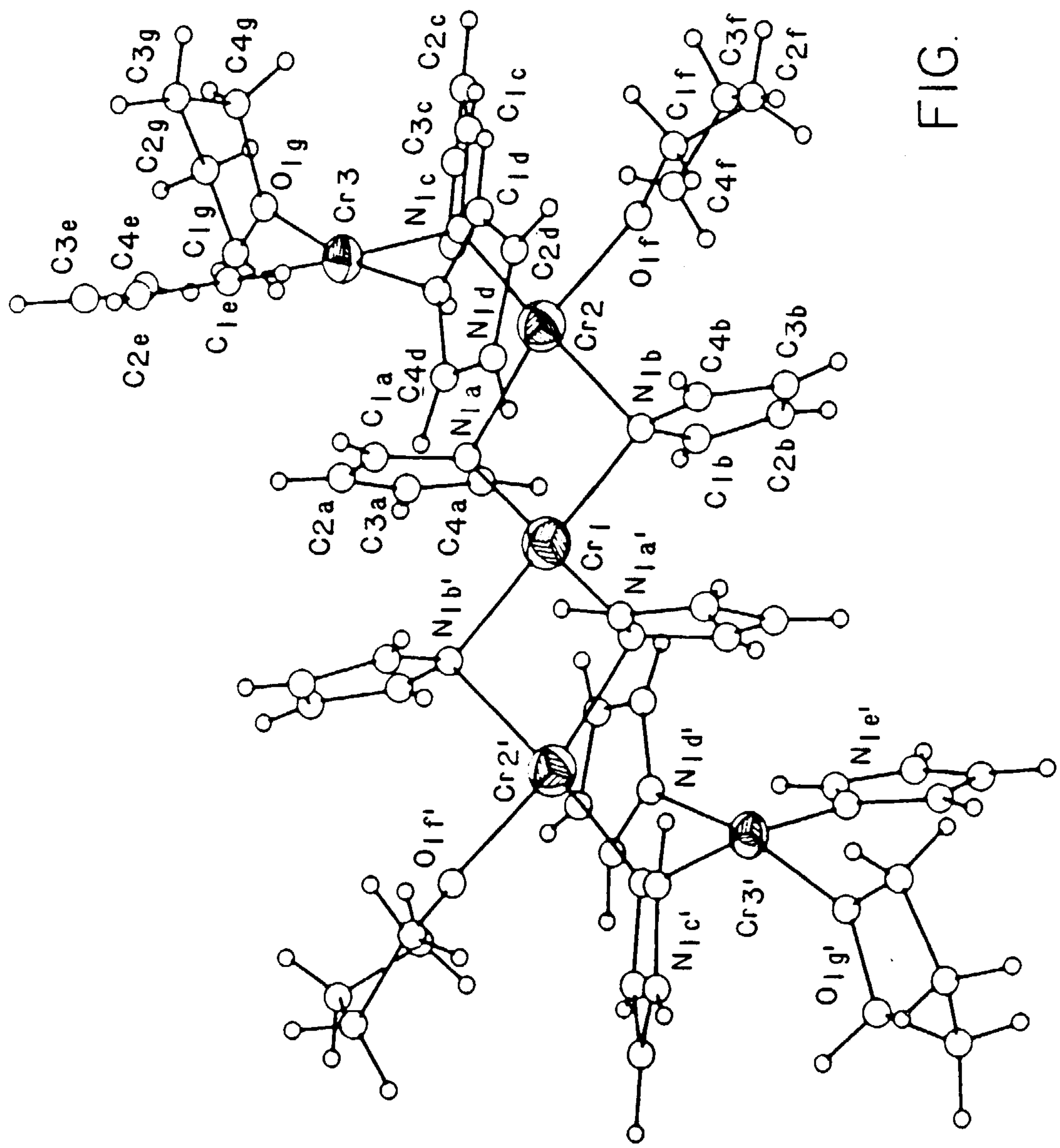

FIG. 1

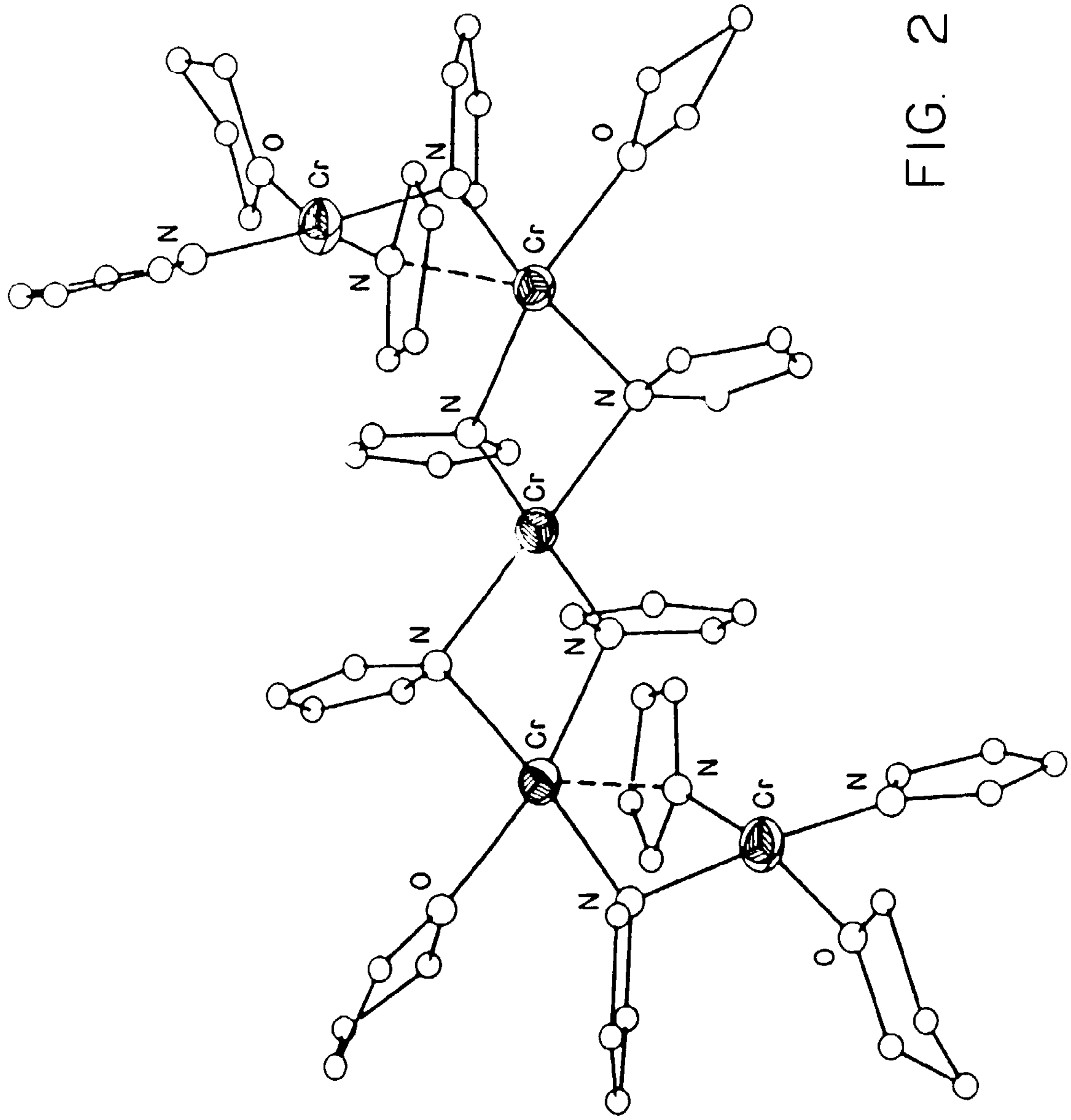

FIG. 2

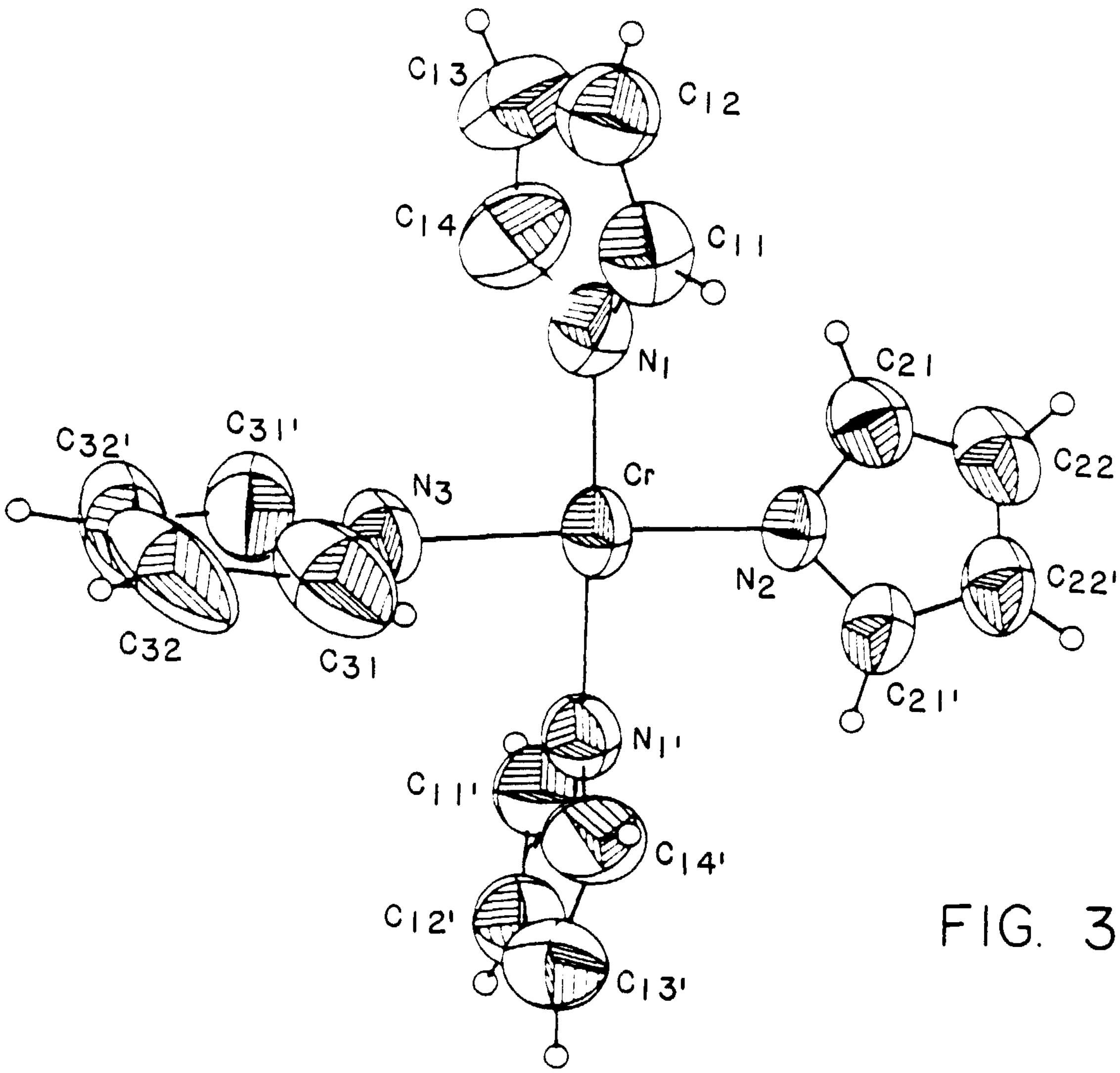

FIG. 3

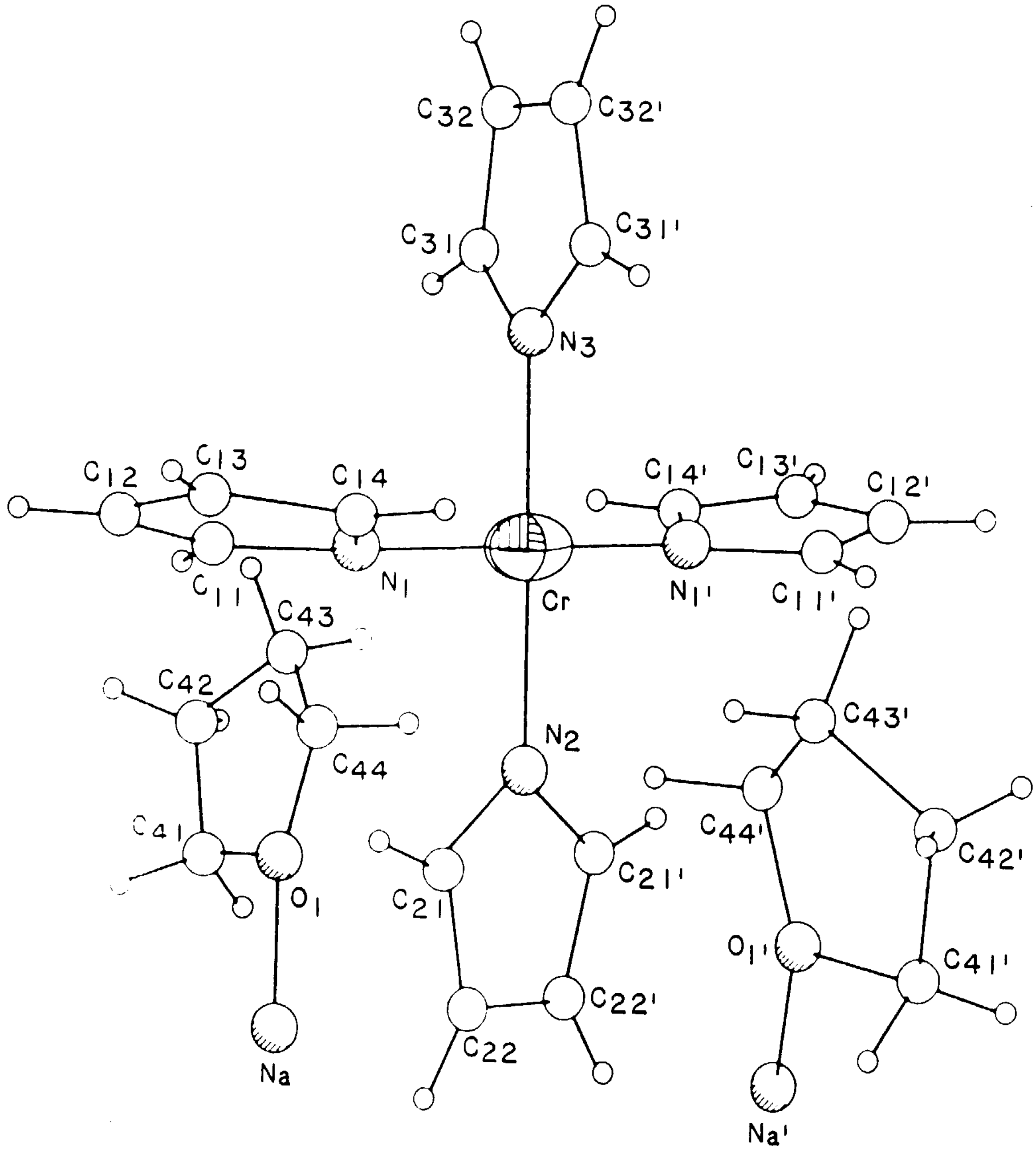

FIG. 4

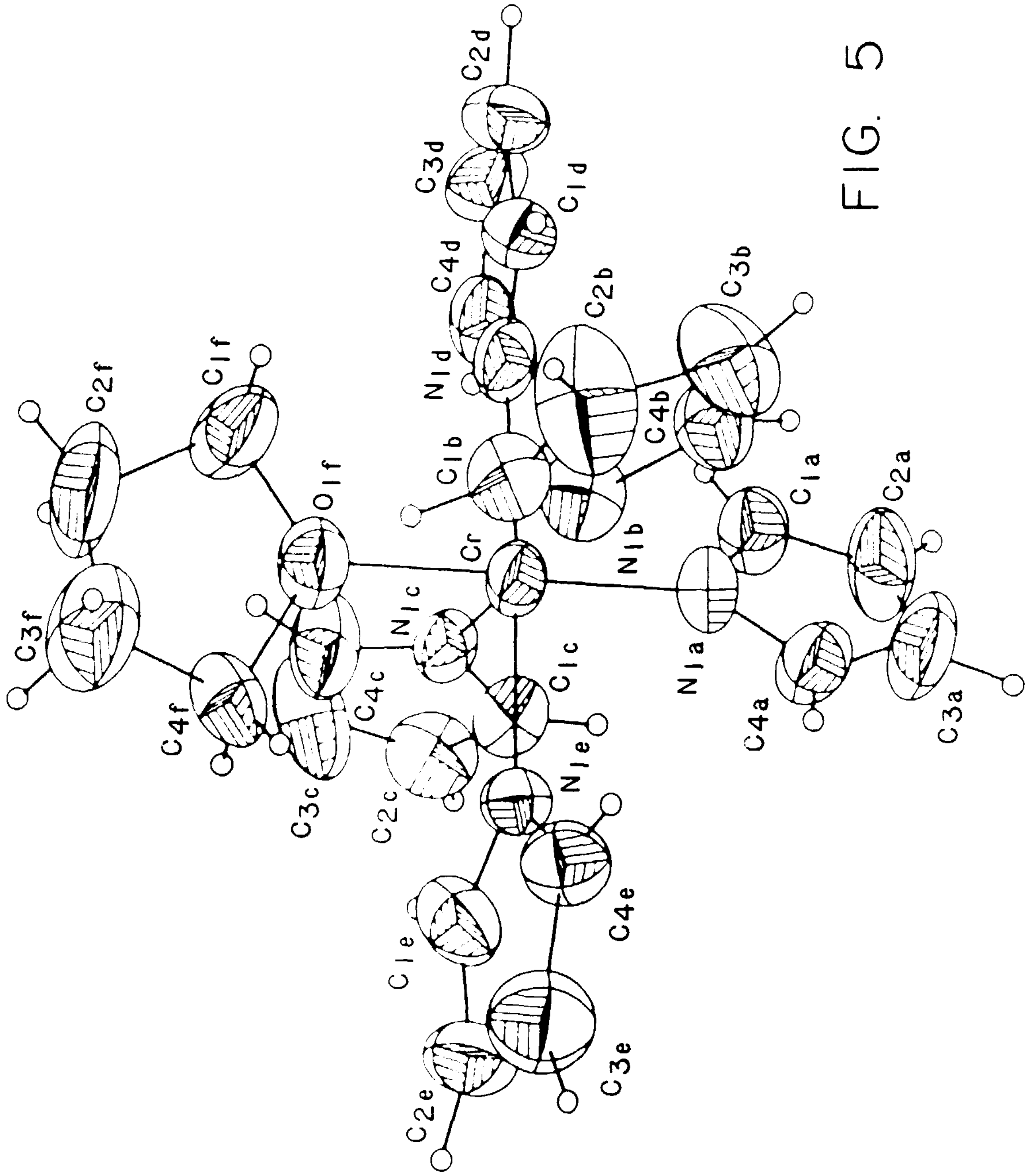

FIG. 5

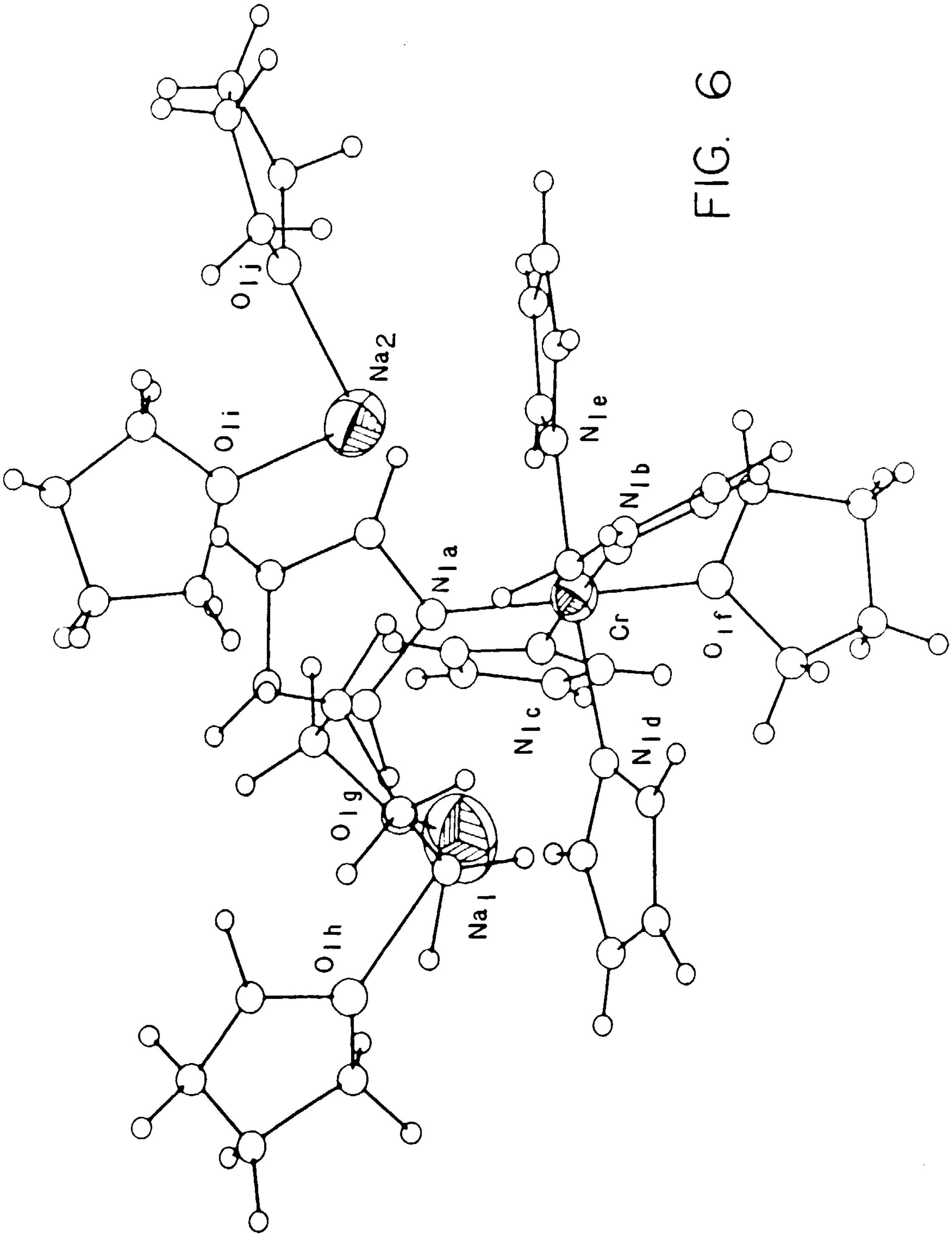

FIG. 6

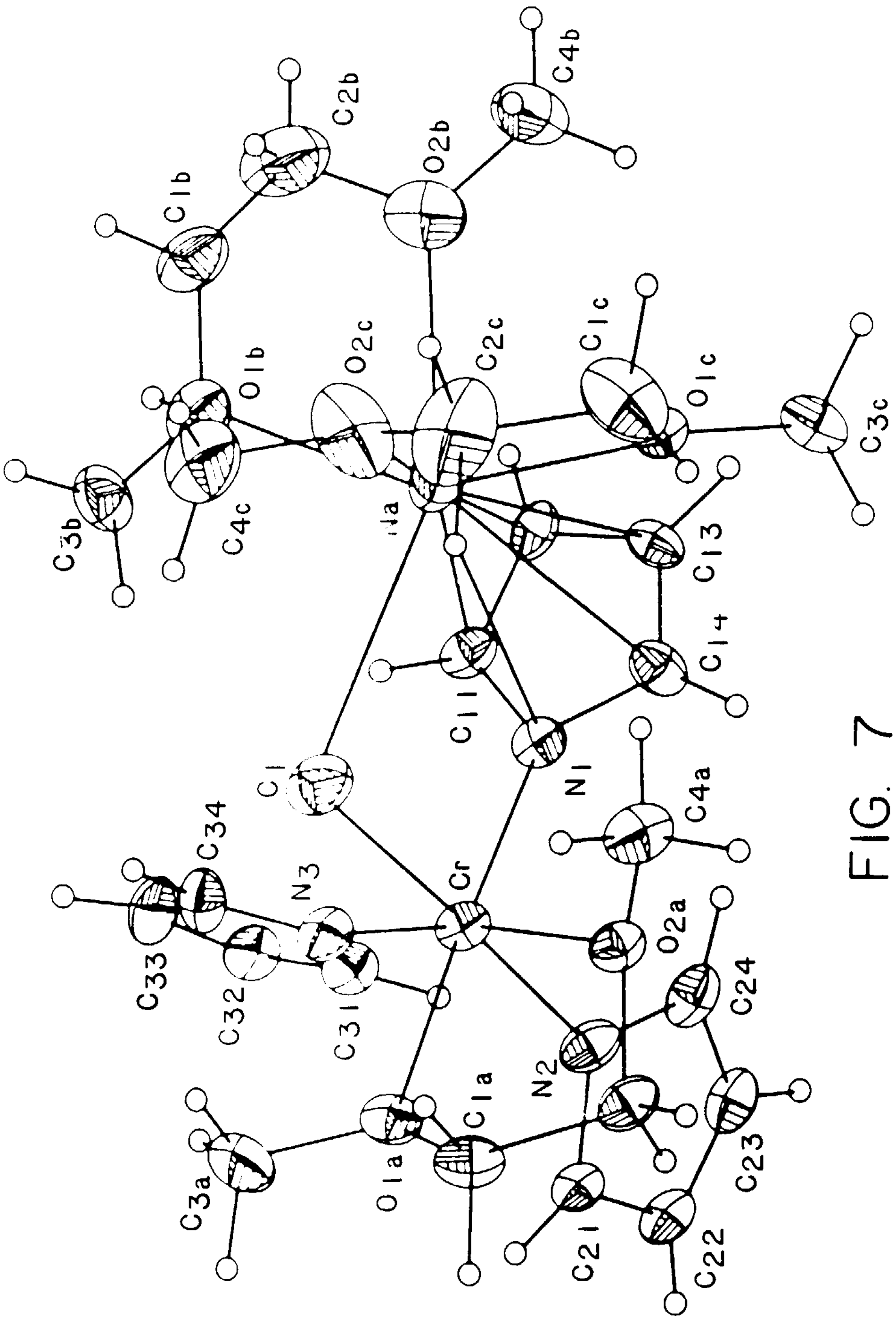

FIG. 7

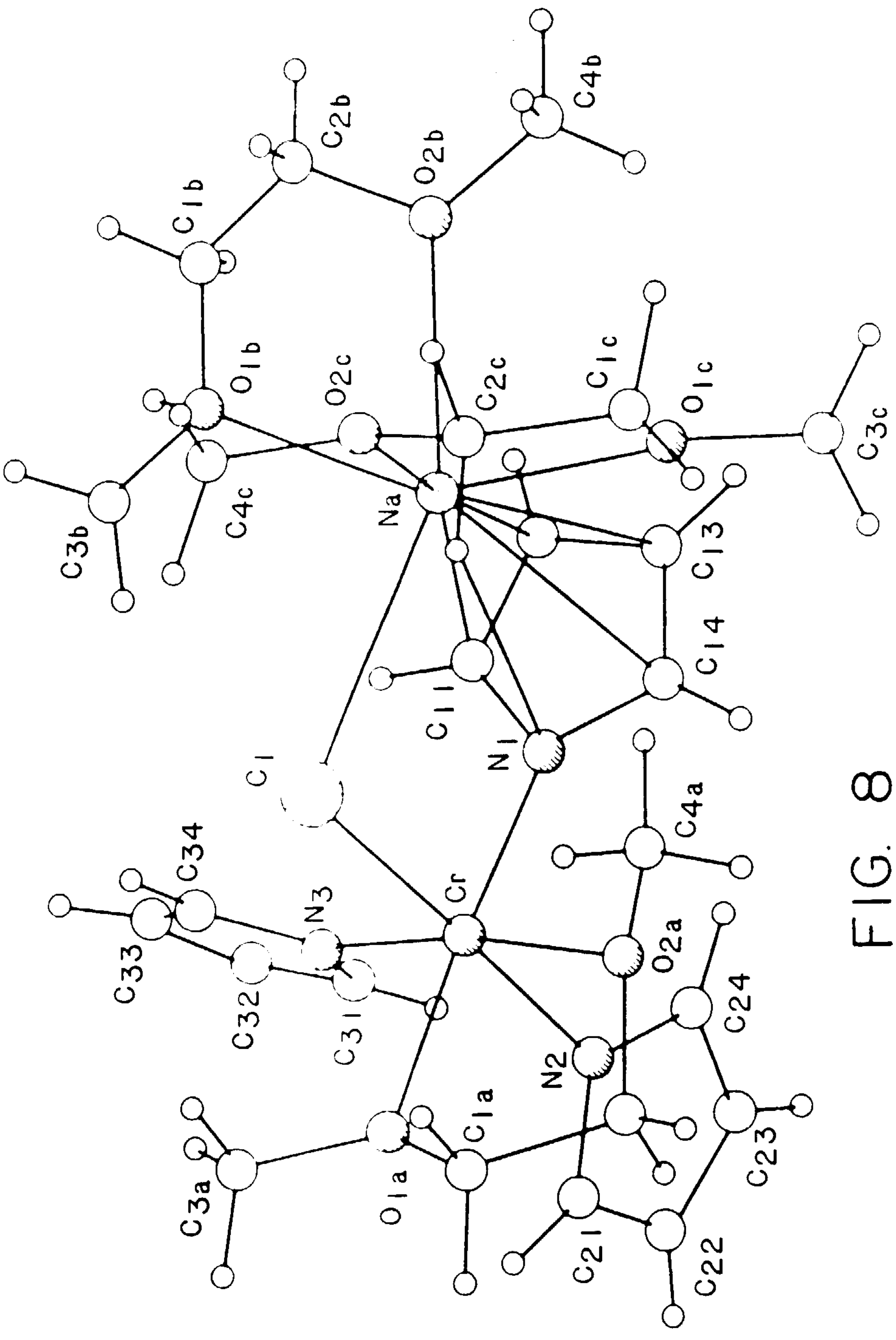

FIG. 8

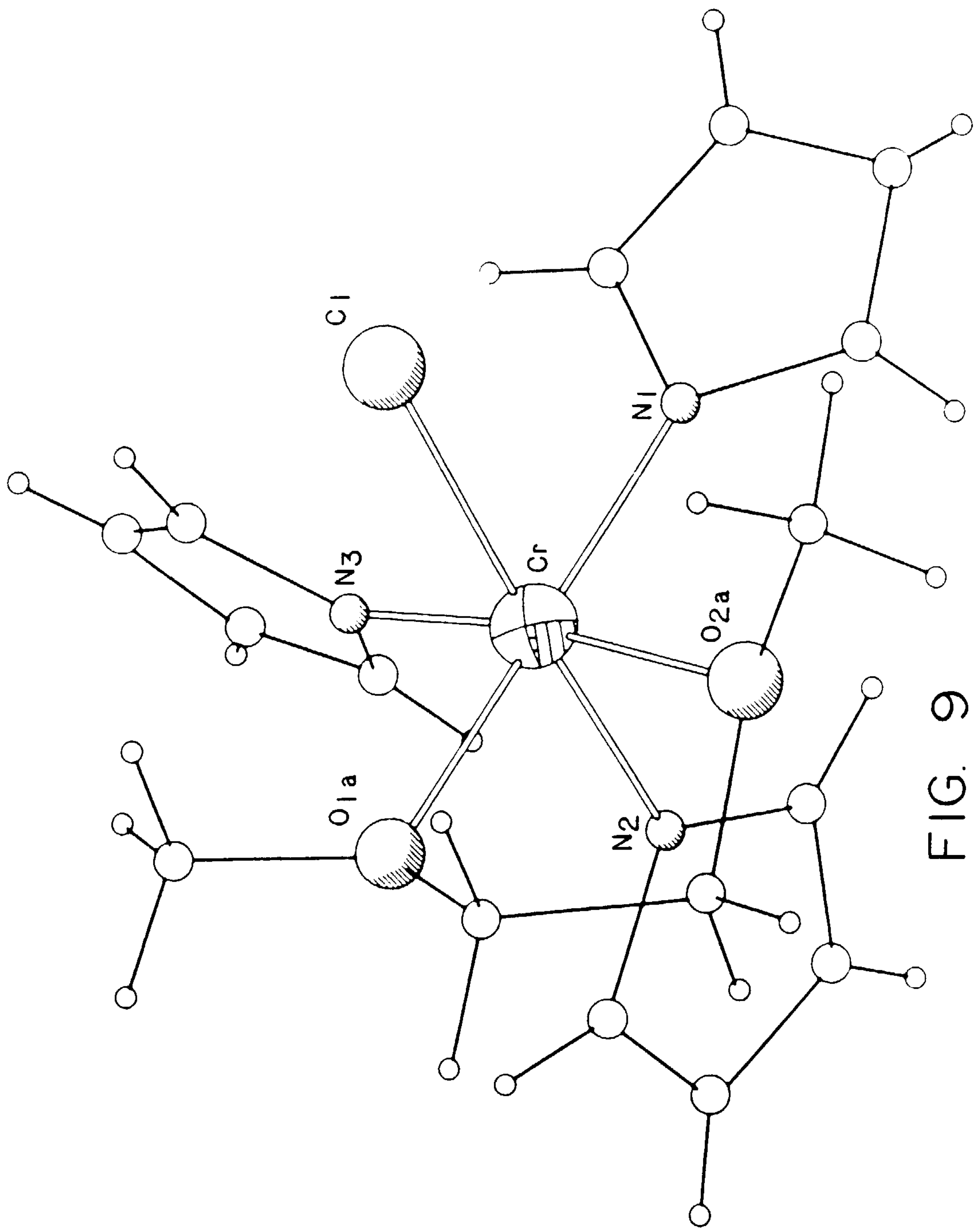

FIG. 9